# EUROPEAN PATENT SPECIFICATION

(11) **EP 1 063 264 B1**
(45) Date of publication and mention of the grant of the patent: **01.03.2006**
(21) Application number: 00113409.7
(22) Date of filing: 23.06.2000
(51) Int. Cl.: C09B 47/00, G03G 5/06, C09B 47/04, C07D 487/22, C07D 259/00, C07D 241/00, C07D 209/00

(54) **Reaction product, process of producing same, electrophotographic photoconductor using same, electrophotographic apparatus having the photoconductor, and process cartridge for electrophotographic apparatus**
Reaktionsprodukt, Verfahren zu dessen Herstellung, das Produkt enthaltender elektrophotographischer Photoleiter, elktrophotographisches Gerät worin dieser Photoleiter eingesetzt wird und Verfahrenskassette für dieses elektrophotographische Gerät
Produit de réaction, procédé pour sa fabrication, photoconducteur le contenant, appareil électrophotographique comprenant ce photoconducteur, et cartouche de traitement pour cet appareil électrophotographique

(30) Priority: 22.06.1999 JP 17521399; 22.06.1999 JP 17524099; 14.09.1999 JP 26063299; 14.09.1999 JP 26063399; 14.09.1999 JP 26063499; 14.03.2000 JP 2000070353
(43) Date of publication of application: 27.12.2000
(73) Proprietor: Ricoh Company, Ltd., Tokyo 143-8555 (JP)
(72) Inventor: Tadokoro, Kaoru, c/o RICOH COMPANY, Ltd., Tokyo 143-8555 (JP); Shoshi, Masayuki, c/o RICOH COMPANY, Ltd., Tokyo 143-8555 (JP); Namba, Michihiko, c/o RICOH COMPANY, Ltd., Tokyo 143-8555 (JP); Shimada, Tomoyuki, c/o RICOH COMPANY, Ltd., Tokyo 143-8555 (JP); Tanaka, Chiaki, c/o RICOH COMPANY, Ltd., Tokyo 143-8555 (JP)
(74) Representative: Barz, Peter

(56) References cited:
- WO-A-99/23096
- GB-A- 471 418
- JP-A- 2 039 160
- JP-A- 4 113 361
- JP-A- 4 283 581
- JP-A- 2000 144 005
- JIGAJINNI ET AL : "Structure-property relationships...." HIGH PERFORM. POLYM., vol. 5, no. 3, 1993, pages 239-257, XP002169052
- CHEMICAL ABSTRACTS, vol. 110, no. 18, 1 May 1989 (1989-05-01) Columbus, Ohio, US; abstract no. 156087j, ABD EL-GHAFFAR ET AL : "new class of metal pyridino-porphyrazine pigments" XP002169053 & PIGM. RESIN TECHNOL., vol. 17, no. 5, 1988, pages 4-6,
- PATENT ABSTRACTS OF JAPAN vol. 014, no. 544 (C-0784), 4 December 1990 (1990-12-04) & JP 02 232268 A (NIPPON SODA CO LTD), 14 September 1990 (1990-09-14)
- JAUNG J -Y ET AL: "Dicyanopyrazine studies. Part VI: Absorption spectra and aggregation behaviour of tetrapyrazinoporphyrazines with long alkyl groups" DYES AND PIGMENTS,GB,ELSEVIER APPLIED SCIENCE PUBLISHERS. BARKING, vol. 40, no. 1, January 1999 (1999-01), pages 73-81, XP004150458 ISSN: 0143-7208

## Description

This invention relates to a novel reaction product, to a process of producing such a reaction product, to an electrophotographic photoconductor using such reaction product, to an electrophotographic apparatus having such an electrophotographic photoconductor, and to a process cartridge for such an electrophotographic apparatus.

Conventionally, the photoconductive material for use in the electrophotographic process is roughly divided into two groups, that is, an inorganic photoconductive material and an organic photoconductive material. The above-mentioned electrophotographic process is one of the image forming processes, through which the surface of the photoconductor is charged uniformly in the dark to a predetermined polarity, for instance, by corona charge. The uniformly charged photoconductor is exposed to a light image to selectively dissipate the electric charge of the exposed area, so that a latent electrostatic image is formed on the photoconductor. The thus formed latent electrostatic image is developed into a visible image by use of a toner comprising a coloring agent such as a dye or pigment, and a polymeric material. Such an electrophotographic process is called "Carlson process".

The photoconductor employing the organic photoconductive material is advantageous over that employing the inorganic photoconductive material with respect to the degree of freedom in the wave range of the light to be employed, and the film-forming properties, flexibility, transparency, productivity, toxicity, and manufacturing cost of the photoconductor. In light of the above-mentioned advantages, most of the current photoconductors employ the organic photoconductive material. The photoconductor which is repeatedly operated by the above-mentioned electrophotographic process or the like is required to exhibit excellent electrostatic properties, more specifically, excellent photosensitivity, acceptance potential, retentivity of charge, potential stability, residual potential and spectral sensitivity.

In recent years, the development of data processing apparatus employing the above-mentioned electrophotographic process is remarkable. In particular, there is a remarkable improvement in the printing quality and the reliability of the digital printer which is capable of recording data by digital recording method, to be more specific, converting the data into digital signals and recording the data using a light. Such a digital recording system is applied not only to the printer, but also to the copying machine. Thus, the digital copying machine is actively developed. It is supposed that the demand for the digital copying machine will further increase in line with the addition of various data processing functions.

The photoconductor designed for the above-mentioned digital recording system is required to have special characteristics which are different from those required for the conventional analogue recording system. For instance, semiconductor laser (LD) or light emitting diode (LED) is widely employed as a light source for the digital recording system because of its compactness, cheapness and high reliability. The wave range of the currently used LD is within the near infrared region, while the wavelength of the currently used LED is 650 nm or more. Therefore, the electrophotographic photoconductors for use with the above-mentioned digital recording system are required to show sufficient sensitivity in the wavelength range from the visible region to the near infrared region. In light of the above-mentioned sensitivity, a squarylium dye (Japanese Laid-Open Patent Applications 49-105536 and 58-21416), a triphenylamine trisazo pigment (Japanese Laid-Open Patent Application 61-151659), and a phthalocyanine pigment (Japanese Laid-Open Patent Applications 48-34189 and 57-14874) are proposed as the photoconductive materials for use in the digital recording. In particular, the phthalocyanine pigment, that is, a titanyltetraazaporphyrin compound, can show absorption and photosensitivity in the relatively long wavelength range. In addition, a variety of phthalocyanine pigments can be obtained according to the kind of central metal or the type of crystalline form. Therefore, research and development of this type of phthalocyanine pigment has been actively conducted to obtain the improved photoconductive material for use with the digital recording. Examples of the conventional phthalocyanine pigments capable of showing good sensitivity include ε-type copper phthalocyanine, X-type metal-free phthalocyanine, τ-type metal-free phthalacyanine, vanadyl phthalocyanine, and titanyl phthalocyanine. To be more specific, titanylphthalocyanine pigments with high sensitivity are proposed in Japanese Laid-Open Patent Applications 64-17066, 3-128973 and 5-98182. Those titanylphthalocyanine pigments exhibit maximum absorption in the wavelength range of 700 to 860 nm, so that they can show remarkably high sensitivity with respect to the semiconductor laser beam. It is also known to be effective for the purpose of obtaining phthalocyanine pigments with specific crystal structures to treat the phthalocyanine pigments with an acid, an organic solvent or water (Japanese Laid-Open Patent Applications 145550). Japanese Laid-Open Patent Applications Nos. 8-6050, 8-283599 and 2-269776 disclose a method of treating titanyl phthalocyanines with trihaloacetic acid.

However, when each of the above-mentioned titanylphthalocyanine pigments is employed in the electrophotographic photoconductor, there still remain a lot of practical problems, for example, decline in charging performance due to fatigue, and an increase in temperature- and humidity-dependence of the charging characteristics although the sensitivity is sufficient [Y. Fujimaki, Proc. IS&T's 7th International Congress on Advances in Non-Impact Printing Technologies, 1,269 (1991); K. Daimon et al.; J. Imaging Sci. Technol., 40,249 (1996)] .

With regard to other tetraazaporphyrin derivatives, Japanese Patent Publication No. 2-39160, 3-27111, 4-283581 and 4-113361 disclose that pigments with asymmetric scheletons or mixed pigments such as a mixture of a phthalocyanine with a phthalocyanine-nitrogen or phthalocyanine-sulfur analogue is effective as the photoconductive material. When these pigments are used for an electrophotographic photoconductor, the sensitivity in the visible light and near infrared range, charging characteristics and resistance to repeated use are still unsatisfactory.

It is, therefore, an object of the present invention to solve the problems of the photoconductive material for the conventional electrophotographic photoconductors.

Another object of the present invention is to provide a novel reaction product useful as an organic photoconductive material for electrophotographic photoconductors.

It is a further object of the present invention to provide a process of producing such a reaction product, an electrophotographic photoconductor using such a reaction product, an electrophotographic machine using such a photoconductor and a process cartridge for such an electrophotographic machine.

The present inventors have made an earnest study with a view toward solving the above problems and have found that a reaction product obtained by reaction of a nitrile compound of the above formula (1) with a phthalonitrile derivative of the formula (2) and, if necessary, with a metal or a metal-containing compound, which reaction may be, if necessary, followed by a treatment with a solvent for changing a crystal structure of the product, shows excellent charge generating properties and excellent electrophotographic characteristics. The present invention is based on this finding.
In accordance with one aspect of the present invention, there is provided a product obtained by reacting a nitrile compound of the formula (1) with a phthalonitrile compound of the formula (2) or a 1,3-diiminoisoindolin compound of the formula (5) and, if necessary, with a metal or a metal-containing compound: 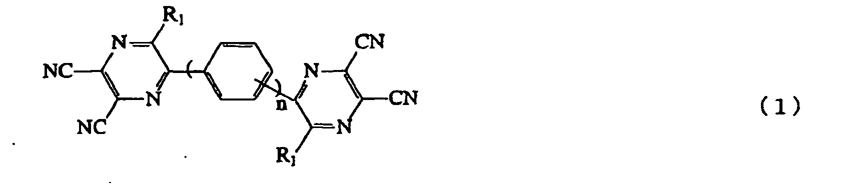 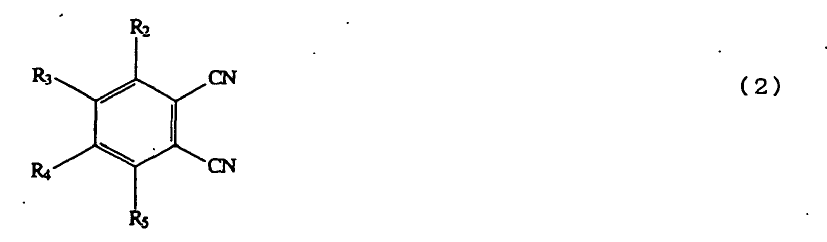 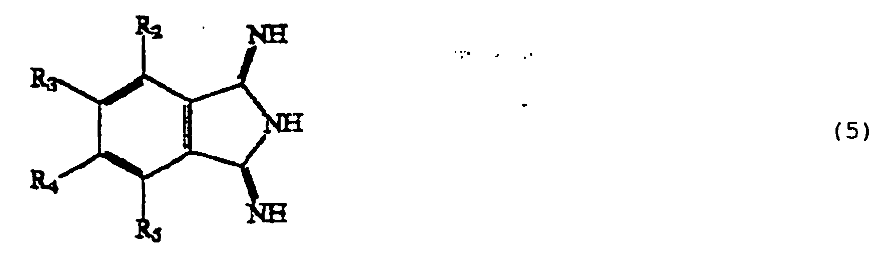
wherein R₁-R₅ stand, independently from each other, for a hydrogen atom, a halogen atom, a substituted or unsubstituted alkyl group, a substituted or unsubstituted aryl group, a heteroaryl group selected from thienyl group, benzothienyl group, furyl group, benzofuranyl group, carbazolyl group, pyridyl group, pyridinyl group, pyrrolidyl group and oxazolyl group, or any of the above heteroaryl groups having one or more substituents, a substituted or unsubstituted cycloalkyl group, a substituted or unsubstituted alkoxyl group, a substituted or unsubstituted aryloxy group, a nitro group or a cyano group and n is an integer of 1 or 2, with the proviso that two adjacent groups R₂-R₅ may together form a ring.

In another aspect, the present invention provides a process for the production of the above product, wherein the reaction is followed by a treatment with an organic solvent, an acid, water or a mixture thereof.

The present invention also provides an electrophotographic photoconductor comprising an electroconductive support and a photoconductive layer provided thereon and containing the above product.

The present invention further provides an electrophotographic machine comprising charging means, exposing means, developing means, transfer means, cleaning means, charge removing means and the above electrophotographic photoconductor.

The present invention further provides a process cartridge for an electrophotographic machine comprising charging means and the above electrophotographic photoconductor.

Other objects, features and advantages of the present invention will become apparent from the detailed description of the preferred embodiments of the invention which follows, when considered in the light of the accompanying drawings, in which:
Fig. 1 is an X-ray diffraction spectrum of a product obtained in Example 6;
Fig. 2 is an X-ray diffraction spectrum of a product obtained in Example 7;
Fig. 3 is an X-ray diffraction spectrum of a product obtained in Example 8;
Fig. 4 is an X-ray diffraction spectrum of a product obtained in Example 9;
Fig. 5 is an X-ray diffraction spectrum of a product obtained in Example 10;
Fig. 6 is an X-ray diffraction spectrum of a product obtained in Example 11;
Fig. 7 is an X-ray diffraction spectrum of a product obtained in Example 12;
Fig. 8 is an X-ray diffraction spectrum of a product obtained in Example 13;
Fig. 9 is an X-ray diffraction spectrum of a product obtained in Example 14;
Fig. 10 is an X-ray diffraction spectrum of a product obtained in Example 18;
Fig. 11 is an X-ray diffraction spectrum of a product obtained in Example 19;
Fig. 12 is an X-ray diffraction spectrum of a product obtained in Example 20;
Fig. 13 is an X-ray diffraction spectrum of a product obtained in Example 21;
Fig. 14 is an X-ray diffraction spectrum of a product obtained in Example 22;
Fig. 15 is an X-ray diffraction spectrum of a product obtained in Example 24;
Fig. 16 is an X-ray diffraction spectrum of a product obtained in Example 25;
Fig. 17 is an X-ray diffraction spectrum of a product obtained in Example 26;
Fig. 18 is an X-ray diffraction spectrum of a product obtained in Example 27;
Fig. 19 is an X-ray diffraction spectrum of a product obtained in Example 28;
Fig. 20 is an X-ray diffraction spectrum of a product obtained in Example 29;
Fig. 21 is an X-ray diffraction spectrum of a product obtained in Example 30;
Fig. 22 is an X-ray diffraction spectrum of a product obtained in Example 31;
Fig. 23 is an X-ray diffraction spectrum of a product obtained in Example 32;
Fig. 24 is an X-ray diffraction spectrum of a product obtained in Example 33;
Fig. 25 is an X-ray diffraction spectrum of a product obtained in Comparative Example;
Fig. 26 is a mass spectrum of a product obtained in Example 2;
Fig. 27 is an infrared absorption spectrum of a product obtained in Example 2;
Fig. 28 is an X-ray diffraction spectrum of a product obtained in Example 34;
Fig. 29 is an infrared absorption spectrum of a product obtained in Example 4;
Fig. 30 is an infrared absorption spectrum of a product obtained in Example 5;
Fig. 31 is a light absorption spectrum of a film prepared in Example 54 and containing a product obtained in Example 24;
Fig. 32 is a light absorption spectrum of a solution prepared in Example 54 and containing a product obtained in Example 24;
Fig. 33 is an infrared absorption spectrum of a product obtained in Example 1;
Fig. 34 is an infrared absorption spectrum of a product obtained in Example 3; and
Fig. 35 is an infrared absorption spectrum of a product obtained in Example 5.

The novel product according to the present invention is obtained by reacting a nitrile compound of the formula (1) with a phthalonitrile compound of the formula (2) and, if necessary, with a metal or a metal-containing compound: 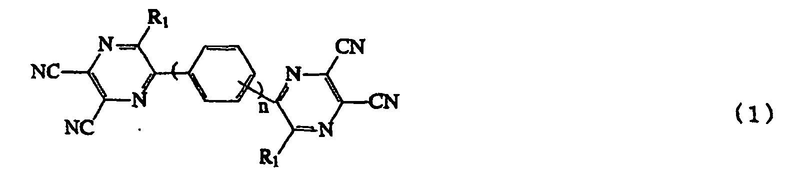 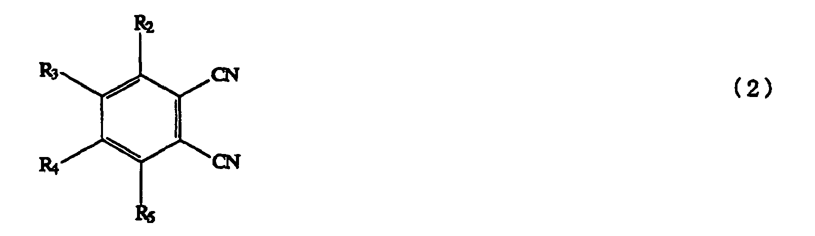
wherein R₁-R₅ stand, independently from each other, for:
a hydrogen atom;

a halogen atom, such as iodine, bromine, chlorine or fluorine;
a substituted or unsubstituted alkyl group, such as methyl group, ethyl group, n-propyl group, iso-propyl group, trifluoromethyl group, 2-cyanoethyl group, benzyl group, 4-chlorobenzyl group or 4-methylbenzyl group;
a substituted or unsubstituted alkoxyl group, such as methoxy group, ethoxy group, n-propoxy group, iso-propoxy group, n-butoxy group, iso-butoxy group, sec-butoxy group, tert-butoxy group, 2-hydroxyethoxy group, 2-cyanoethoxy group, benzyloxy group, 4-methylbenzyloxygroup or trifluoromethoxy group;
a substituted or unsubstituted aryl group, such as phenyl group, naphthyl group, biphenylyl group, terphenylyl group, pyrenyl group, fluorenyl group, 9,9-dimethyl-2-fluorenyl group, azulenyl group, anthryl group, triphenylenyl group, chrysenyl group, fluorenylidenephenyl group, 5H-dibenzo[a,d]cycloheptenylidenephenyl group, or any of the above aryl groups having one or more substituents such as the same alkyl group, alkoxyl group and/or halogen atom as previously mentioned;
a heteroaryl group selected from thienyl group, benzothienyl group, furyl group, benzofuranyl group, carbazolyl group, pyridyl group, pyridinyl group, pyrrolidyl group and oxazolyl group, or any of the above heteroaryl groups having one or more substituents such as the same alkyl group, alkoxyl group and/or halogen atom as previously mentioned;
a substituted or unsubstituted cycloalkyl group such as cyclopentyl group or cyclohexyl group,
a nitro group or
a cyano group,
and n is an integer of 1 or 2. One of R₂-R₅ may form, together with its adjacent one of R₂-R₅, a ring.

The metal or metal-containing compound to be reacted may be, for example, Mg, Li, Na or a compound having a metal such as Na, Li, Cu, Ti, Mg, Co, Mn, Pb, V, Fe, Zn, Ge, Sn, Ni, Al, Ga, Mo or In. A metal halide such as TiCl₄, CoCl₂, CuCl₂, CuCl, InCl₃, InBr₃, AlCl₃, GaCl₂ or VCl₃, or a metal alkoxide such as Ti(OBu)₄ or Mg(OEt)₂ may be suitably used as the metal-containing compound.

Shown in Tables 1 and 2 are examples of combinations of raw materials, i.e. the nitrile compounds (1), phthalonitrile compounds (2) and metals or metal-containing compounds, for the production of products according to the present invention.

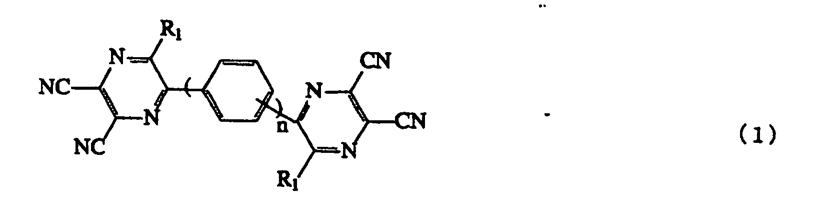

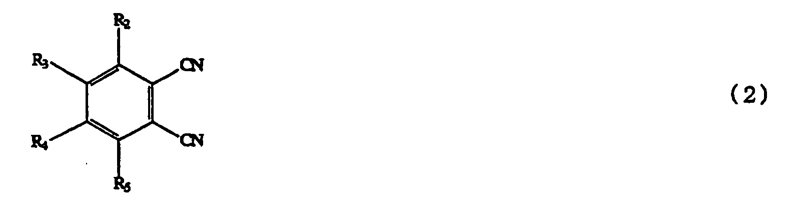

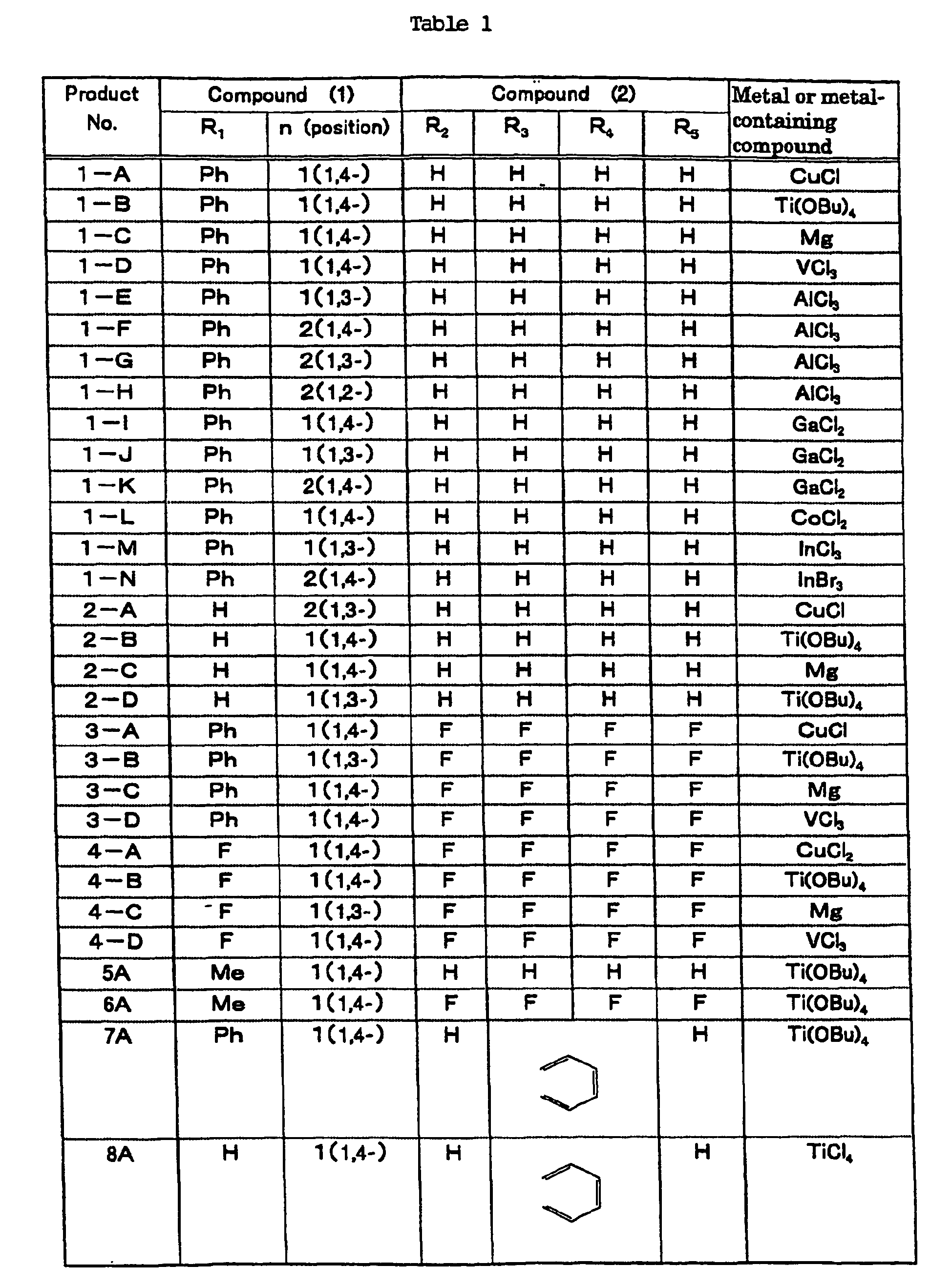

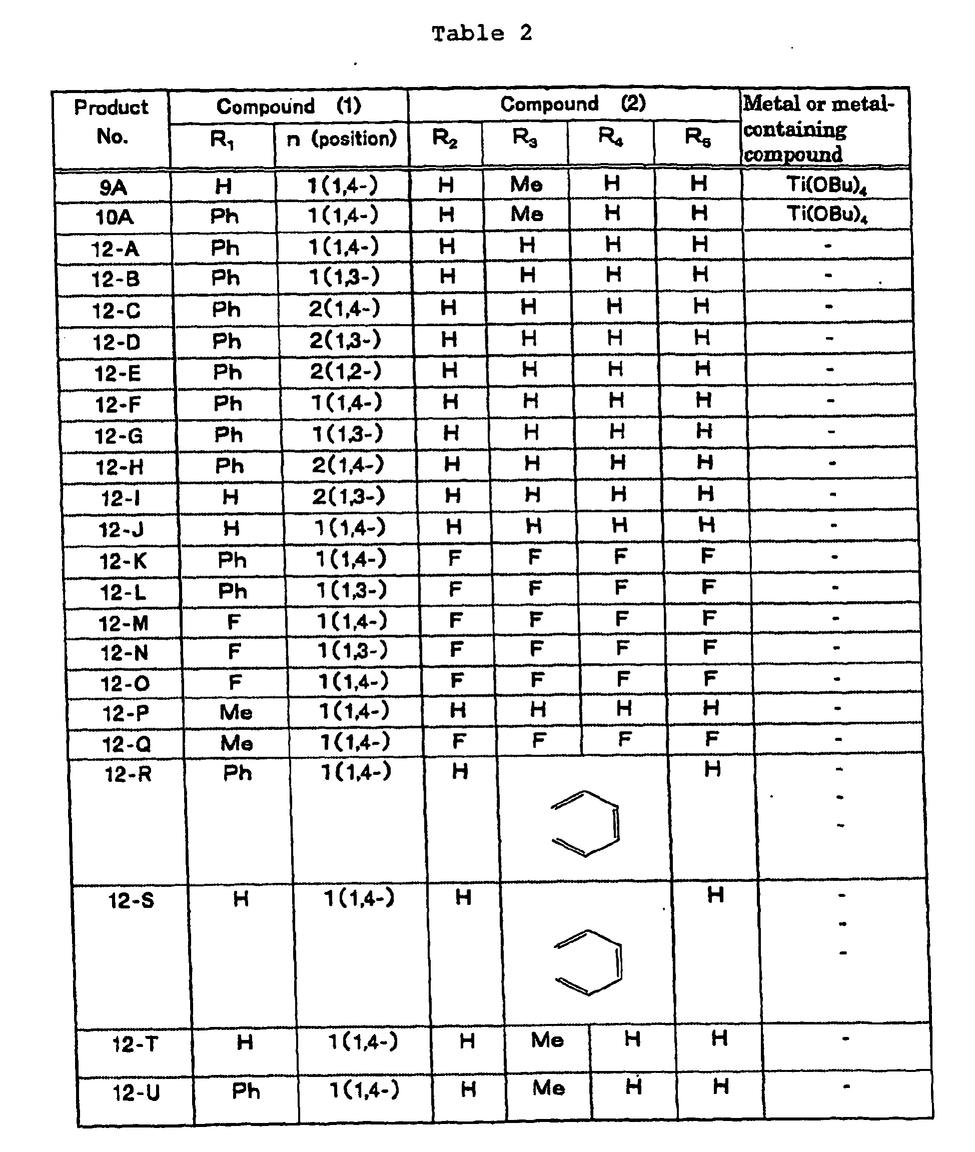

The reaction products may be obtained by reacting a nitrile compound of the formula (1) with a phthalonitrile compound of the formula (2) and, if necessary, with a metal or a metal-containing compound at a temperature of generally from room temperature to 300°C with or without using a solvent. To prevent the formation of by-products and to facilitate the reaction, the use of a reaction temperature from 40°C to 200°C is preferred. The solvent may be, for example, a halogenated hydrocarbon such as α - chloronaphthalene, dichlorobenzene or trichlorobenzene, an alcohol such as pentanol or octanol, an amine such as N,N-dimethylformamide or N-methylpyrrolidone, or an aromatic hydrocarbon such as benzene, toluene or nitrobenzene.

In place of the phthalonitrile compound of the formula (2), a 1,3-diiminoisoindolin of the following formula (5) may be used, if desired: 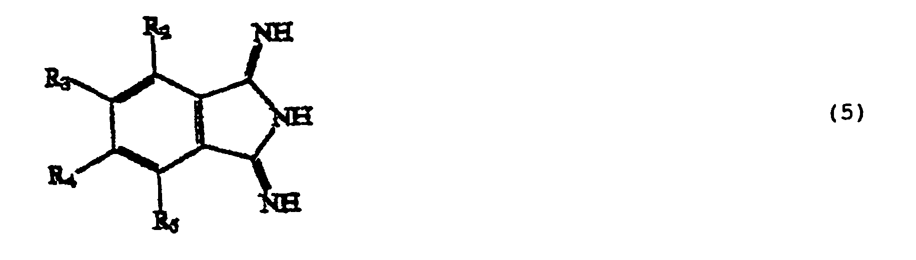
wherein R₁-R₅ has the same meaning as defined above with reference to the phthalonitrile compound of the formula (2). The phthalonitrile compound (2) and the 1,3-diiminoisoindolin (5) may be obtained by a method disclosed in literature "Phthalocyanine, Chemistry and Function" by Shirai and Kobayashi. Some of them are also commercially available.

The nitrile compound (1) may be prepared according to the following reaction: 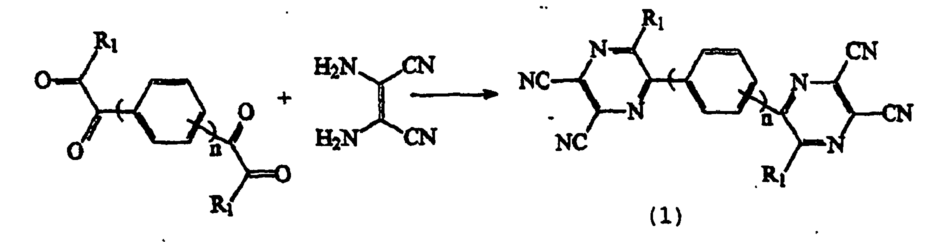
wherein R₁ and n are as defined above.

The above reaction is performed at a temperature of generally from room temperature to 300°C with or without using a solvent. To facilitate the reaction, the use of a reaction temperature from 100°C to 180°C is preferred. The solvent may be, for example, an alcohol such as ethanol, butanol, pentanol or octanol, an aromatic hydrocarbon such as benzene, toluene or nitrobenzene, a halogenated hydrocarbon such as α-chloronaphthalene, dichlorobenzene or trichlorobenzene, an acidic solvent such as acetic acid, or an amine such as N,N-dimethylformamide or 1-methyl-2-pyrrolidinone. For reasons of improving reaction yield and solubility of reaction raw materials, it is preferred that the reaction be performed in the presence of an acid catalyst such as acetic acid, sulfuric acid or hydrochloric acid.

The reaction of the nitrile compound (1) with the naphthonitrile compound (2) or 1,3-diiminoisoindolin (5) may be carried out in the presence of an amine catalyst such as urea, formamide or 1,8-diazabicyclo[5,4,0]-undecene (DBU). For reasons of improving electrophotographic characteristics such as increase of residual potential and prevention of chargeability, it is preferred that the molar ratio of the nitrile compound (1) to the phthalonitrile compound (2) or 1,3-diiminoisoindolin (5) be in the range of 0.001:999.999 to 99.999:0.001, more preferably in the range of 0.1:99.9 to 99.9 to 0.1.

The product obtained by the reaction of the nitrile compound (1) with the phthalonitrile compound (2) or 1,3-diiminoisoindolin (5) and, optionally, with the metal or metal-containing compound may be further treated with an organic solvent, an acid, water or a mixture (solution, emulsion or dispersion) thereof to change the crystal structure of the product. The treated product obtained as a result of this treatment has various different crystal structures or is amorphous. Appearance of sharp diffraction peaks and different diffraction peaks in X-ray diffraction patterns indicates the presence of various different crystal structures in the treated product. When no diffraction peaks are present, then the treated product is regarded as being amorphous.

As the method of converting crystal structure to obtain a treated product having different crystal structures, there may be mentioned, for example, an acid treatment, a solvent treatment, a mechanical treatment, a heat treatment and a combination of two or more of these treatments.

In the acid treatment, a product to be treated (hereinafter referred to as sample) is dissolved or dispersed in an acid such as sulfuric acid, trichloroacetic acid, trifluoroacetic acid or a mixture of such an acid with an organic solvent at a temperature of 0°C to room temperature. The solution or dispersion thus obtained is then added dropwise to an organic solvent or a mixed organic solvent in which the sample is insoluble or hardly soluble, so that treated sample is precipitated. The treated sample is separated by filtration or any other suitable separation method.

In the solvent treatment, a sample is suspended with stirring in an organic solvent, water or a mixture thereof at a temperature from room temperature to 250°C. The organic solvent may be, for example, an aromatic hydrocarbon such as benzene, toluene, dichlorobenzene or nitrobenzene, an alcohol such as methanol, ethanol, n-propanol, n-butanol or n-pentanol, a ketone such as acetone, cyclohexanone or methyl ethyl ketone, an ether such as n-butyl ether, ethylene glycol n-butyl ether or tetrahydrofuran, an amine such as N,N-dimethylformamide, N-methylpyrrlidone or quinoline, an ester such as methyl acetate, ethyl acetate or n-butyl acetate, or a nitrogen-containing solvent such as 2-pyrrolidinone, 1-methyl-2-pyrrolidinone, 5-methyl-2-pyrrolidinone, 1,5-dimethyl-2-pyrrolidinone, 1,3-dimethyl-2-imidazolidinone, 2-imidazolidinone or 1-(2-hydroxyethyl)-2-imidazolidinone.

The amount of the organic solvent is desirably at least 5 times, more preferably 5-100 times, the volume of the solids of the sample for reasons of facilitating stirring. When one or more organic solvents are used by themselves, the solvent treatment is generally performed at a temperature of 250°C or less. When water and an organic solvent are used for the solvent treatment, they are used separately or as a mixture. When water and an organic solvent are used separately, the sample may be treated first with water and then with the organic solvent, or vise versa. When water and an organic solvent are used as a mixture, the sample may be treated with the organic solvent in which water is dissolved in a saturated amount.

The solvent treatment may be preceded by the above-described acid treatment. Thus, the sample treated with an acid may be subsequently treated with a solvent, as such without being dried, at a temperature in the range of from room temperature to 250°C, preferably 50-200°C, more preferably 50-150°C for a period of time sufficient to obtain treated sample having different crystal structures. Of course, the sample treated with the acid may be dried before the solvent treatment. It is easier to convert an amorphous product to a crystalline product than to change a crystal structure to another crystal structure. However, the product obtained by the reaction of the nitrile compound (1) with the phthalonitrile compound (2) or 1,3-diiminoisoindolin (5) and, optionally, with the metal or metal-containing compound in the presence of an organic solvent is generally a crystalline product. Thus, when the crystalline product is to be converted into a product having a different crystal structure, it is preferred that the crystalline product be first subjected to an acid treatment for the conversion of the crystalline product into amorphous product and then to a solvent treatment to form the product having the different crystal structure.

In one embodiment of the mechanical treatment, the sample is treated with a milling device such as sand mill or ball mill using glass beads, steel beads or alumina balls at room temperature or an elevated temperature. The mechanical treatment may be carried out in the presence of the above-described solvent.

As the method of converting crystal structure of a sample to obtain an amorphous product, there may be mentioned, for example, an acid treatment and a mechanical treatment. One preferred acid treatment is an acid paste method in which a sample is added little by little to an acid, such as sulfuric acid, trichloroacetic acid or trifluoroacetic acid, at a temperature of not higher than room temperature, preferably 0°C to room temperature, with stirring, to dissolve or disperse the sample therein. The solution or dispersion thus obtained is then added dropwise to ice, ice water, water or an organic solvent in which the sample is not or scarcely soluble, thereby precipitating the treated sample. After filtration, there is obtained the treated sample which is amorphous in nature. It is preferred that the above solution or dispersion be added to vigorously stirred ice water for 5 quickly diffusing the solution or dispersion into the ice water. The solvent may be selected from those described with reference to the above solvent treatment.

In the mechanical treatment for obtaining amorphous product, the sample is treated with a milling device such as sand mill or ball mill using glass beads, steel beads or alumina balls at room temperature or an elevated temperature. The mechanical treatment may be carried out in the presence of the above-described solvent.

Whether or not the product according to the present invention is amorphous can be confirmed by X-ray analysis. When the product is amorphous, no sharp peaks are present in the X-ray diffraction pattern using CuK_{α} ray. In the present specification, the term "sharp peak" is intended to refer to a peak having a half-width value of not greater than 1°. Thus, a product which does not provide an X-ray diffraction pattern having a diffraction peak of not greater than 1° is an amorphous product.

For the production of a treated product having various different crystal structures, it is effective that a crystalline or amorphous product be subjected to an organic solvent treatment or an acid treatment. In the organic solvent treatment, the crystalline or amorphous product is heated at room temperature or an elevated temperature, preferably below 50°C, in an organic solvent (as described above) with stirring. In the acid treatment, the crystalline or amorphous product is dissolved or dispersed little by little in an acid such as sulfuric acid, trichloroacetic acid, trifluoroacetic acid or a mixture of such an acid with an organic solvent at a temperature of 0°C to room temperature. The solution or dispersion thus obtained is then added dropwise to water, ice water, ice or an organic solvent or a mixed organic solvent in which the product is insoluble or hardly soluble, so that treated product is precipitated. The treated product is separated by filtration or any other suitable separation method.

Crystal structures of the treated product may vary according to the treatment conditions, such as kind of solvent, treatment temperature and treatment time. While any of the above-described product may be used as a charge generating material for electrophotographic photoconductors, it is preferable to use a product providing a CuK_{α} X-ray diffraction pattern having strong diffraction peaks at a Bragg angle 2θ±0.2° of in the range of 20°-30° more preferably to use a product providing a CuK_{α} X-ray diffraction pattern having strong diffraction peaks at a Bragg angle 2θ±0.2° of 27.2°, most preferably to use a product providing a CuK_{α} X-ray diffraction pattern having strong diffraction peaks at a Bragg angle 2θ±0.2° of 27.2° but no diffraction peaks at a Bragg angle 2θ±0.2° of in the range of 4°-10°, for reasons of exhibiting excellent electrophotographic characteristics such as charge generating efficiency.

The term "a diffraction pattern having no diffraction peaks at a Bragg angle 2θ±0.2° of in the range of 4°-10°" used herein is intended to refer to a CuK_{α} X-ray diffraction pattern which does not have, in a Bragg angle 2θ±0.2° of in the range of 4°-10°, any peak attributed to crystals, any low intensity peak with a broad half-width value attributed to fine crystals, and any such a low intensity halo as seen in a glassy state material. When the product having such a peak attributed to crystals or fine crystals or a halo at a Bragg angle 2θ±0.2° of in the range of 4°-10° is used as a charge generating material, there is a fear that stability of the resulting electrophotoconductive photoconductor is adversely affected. Although not wishing to be bound by the theory, a product giving a diffraction pattern having a peak or halo at a Bragg angle 2θ±0.2° of in the range of 4°-10° probably contains a substance serving as a seed crystal so that the product has a tendency to alter the crystal structures thereof during storage or during fabrication of photoconductors. On the other hand, a product providing a diffraction pattern having no diffraction peaks at a Bragg angle 2θ±0.2° of in the range of 4°~10° has excellent stability and can retain its crystal structures even when stored in the form of a solution in an organic solvent for a long period of time. Thus, a coating liquid containing such a product has good storage stability, ensuring reduction in manufacturing costs for photoconductors.

For the production of a treated product having no diffraction peaks at a Bragg angle 2θ±0.2° of in the range of 4°-10°, it is preferred that a product obtained by the reaction of the nitrile compound (1) with the phthalonitrile compound (2) and, optionally, with the metal or metal-containing compound be treated with a mixture of trifluoroacetic acid with an organic solvent The mixing ratio (by weight) of the organic solvent to the trifluoroacetic acid is preferably 0.1:99.9 to 99.9:0.1, more preferably 1:9 to 9:1 for reasons of expediting formation of a solution or dispersion. Thus, the product is dissolved or dispersed little by little in the mixture of trifluoroacetic acid with an organic solvent. The solution or dispersion thus obtained is then added dropwise to an organic solvent in which the above product is insoluble or hardly soluble, so that a treated product is precipitated. While any organic solvent may be used for mixing with trifluoroacetic acid, the use of a mixed solvent containing a chlorine-containing organic solvent, such as chloroform, dichloromethane or 1,2-dichloroethane, and an alcohol, such as methanol or ethanol, is preferred for reasons of expediting formation of a solution or dispersion.

The product according to the present invention (inclusive of the product treated with an acid, solvent, etc. as described above) has charge generating properties and, hence, is suitably used as a charge generating material for electrophotographic photoconductors. When the product is dispersed in a binder and is formed into a film, the film can absorb light of a near infrared region, i.e. a wavelength in the range of 500-700 nm, which corresponds to the wavelength of light emitted from laser diodes (LD). Thus, with the use of the product according to the present invention as a charge generating material, it is possible to obtain an electrophotographic photoconductor having high sensitivity.

The product according to the present invention can be confirmed by mass spectrometry. For example, when the nitrile compound of the following formula (6), the phthalonitrile compound of the formula (7) and titanium tetrabutoxide Ti(OBu)₄ as a metal-containing compound are reacted, there is obtained a reaction product providing a mass spectrum having a peak at 1383±1. 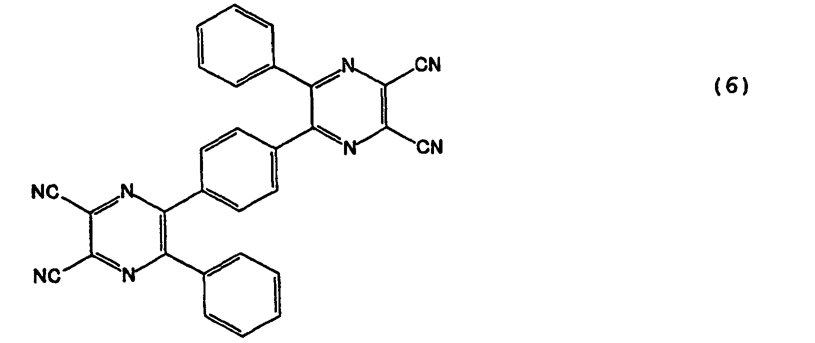 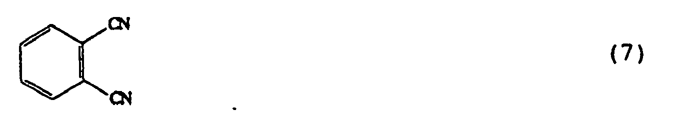
Thus, the above reaction product would contain the compounds of the following formulas (8) and (9).

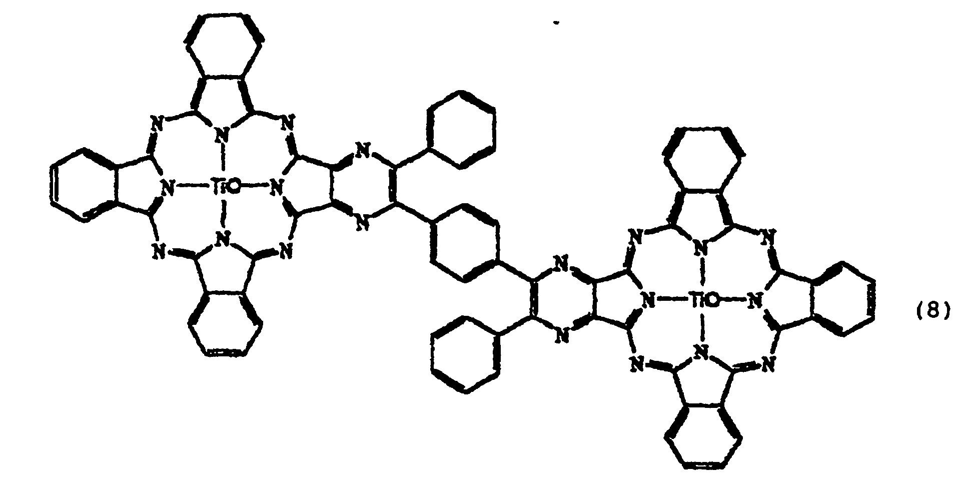

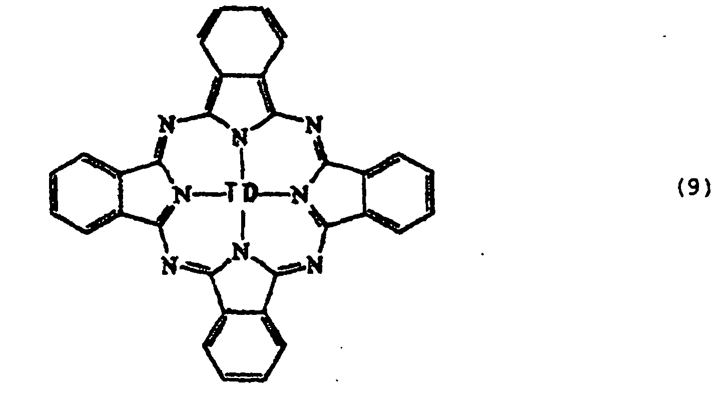

Thus, the above product can be confirmed by the presence of fragment peaks corresponding to the molecular weight of 1383±1 attributed to the compound (8) and the molecular weight of 576±1 attributed to the compound (9). Additionally, X-ray fluorescent elementary analysis can detect Ti in the reaction product.

An electrophotographic photoconductor of a single-layered type or a layered type (function-separating type) can be fabricated, using the product according to the present invention alone or in combination with a charge transport material. To fabricate the electrophotogracphic photoconductor of a single-layered type, a photoconductive layer is provided on an electroconductive support in such a manner that the product is dispersed in a binder resin singly or together with a charge transport material, and the thus prepared dispersion is coated on the support. In the case of a layered type (function-separating type), a charge generation layer comprising the product is provided on an electroconductive support, and a charge transport layer comprising a charge transporting material is overlaid on the charge generation layer. The above-mentioned overlaying order of the charge generation layer and the charge transport layer may be reversed.

In addition to a photoconductive support and a photoconductive layer, the electrophotographic photoconductor of the present invention may further comprise an intermediate layer which is provided between the electroconductive support and the photoconductive layer for the purpose of improving the adhesion between the support and the photoconductive layer and for enhancing the charge blocking characteristics. Furthermore, a protective layer may be provided on the photoconductive layer to improve the wear resistance and the mechanical durability of the photoconductor.

The photoconductive layer may be prepared by applying a coating liquid, in the form of a solution or dispersion, containing the product, a solvent or dispersing medium, and, optionally, a binder resin onto the electroconductive support, followed by drying. The coating liquid may be prepared by mixing the above ingredient using a ball mill, ultrasonic wave or a homomixer. The application of the coating liquid on the electroconductive support may be performed by any coating method such as dip coating, blade coating or spray coating.

To upgrade the dispersibility of the product in the photoconductive layer, it is preferable that the average particle size of the product be 2 µm or less more preferably 1 mµ or less. The lower limit of the average particle size is preferably 0.01 mµ, because too small a particle diameter below 0.01 mµ causes aggregation of fine particles, increase of the resistivity of the photoconductive layer and deterioration of sensitivity and durability due to an increase of defective crystallites.

Specific examples of the solvent which is used to prepare a dispersion or solution for the formation of the photoconductive layer include N,N-dimethylformamide, toluene, xylene, monochlorobenzene, 1-methyl-2-pyrrlidinone, 1,2-dichloroethane 1,1,1-trichloroethane, dichloromethane, 1,1,2 trichloroethane, trichloroethylene, tetrahydrofuran, methyl ethyl ketone, methyl isobutyl ketane, cyclohexanone, ethyl acetate, buty acetate and dioxane.

Any binder resin that has good electrically insulating properties and conventionally used in the preparation of the electrophotographic photoconductor can be employed for the formation of the photoconductive layer in the present invention. Specific examples of such a binder resin include addition polymerization-type resins, polyaddition-type resins and polycondensation-type resins such as polyethylene, polyvinyl butyral, polyvinyl formal, polystyrene resin, phenoxy resin, polypropylene, acrylic resin, methacrylic resin, vinyl chloride resin, vinyl acetate resin, epoxy resin, polyurethane resin, phenolic resin, polyester resin, alkyd resin, polycarbonate resin, polyamide resin, silicone resin and melamine resin; copolymer resins comprising as the repeating units two or more monomers for use in the above-mentioned resins, for example, electrically insulating resins such as vinyl chloride-vinyl acetate copolymer resin, styrene-acrylic copolymer resin, and vinyl chloride-vinyl acetate-maleic anhydride copolymer resin; and a polymeric organic semiconductor such as poly-N-vinylcarbazole. These binder resins may be used alone or in combination.

The product according to the present invention may be used as a mixture with another pigment such as a phthalocyanine pigment. Examples of the phthalocyanine pigment for use in the present invention include metal-free phthalocyanine, copper phthalocyanine, aluminum phthalocyanine, magnesium phthalocyanine, chlorogallium phthalocyanine, hydroxygallium phthalocyanine, vanadyl phthalocyanine, titanyl phthalocyanine, chloroindium phthalocyanine, hydroxyindium phthalocyanine, zinc phthalocyanine, iron phthalocyanine, cobalt phthalocyanine and mixtures thereof. The mixing ratio of the product to the phthalocyanine pigment is, in terms of molar ratio, preferably 0.001:99.999 to 99.999:0.001, more preferably 0.1:99.9 to 99.9:0.1, for reasons of prevention of an increase in residual potential and a reduction of chargeability.

The mixing of the product with a phthalocyanine pigment may be carried out by various suitable methods such as (1) mechanically mixing the product and the pigment using a mill, (2) mechanically mixing the product and the pigment using a tumbler, (3) mixing the product and the pigment in an organic solvent such as xylene using a mixer, (4) mixing the product, the pigment and a binder using a ball mill or sand mill, (5) mixing a binder with one of the product and the pigment, followed by mixing with the other, and (6) dissolving the product and the pigment in an inorganic or organic acid such as sulfuric acid, phosphoric acid, acetic acid or trifluoroacetic acid, followed by coprecipitation with water or an alkaline substance.

The product according to the present invention may be also used in combination with other pigments than phthalocyanine pigment as follows: organic pigments, for example, azo pigments such as C.I. Pigment Blue 25 (C.I. 21180), C.I. Pigment Red 41 (C.I. 21200), C.I. Acid Red 52 (C.I. 45100), C.I. Basic Red 3 (C.I. 45210), an azo pigment having a carbazole skeleton (Japanese Laid-Open Patent Application 53-95033), an azo pigment having a distyryl benzene skeleton (Japanese Laid-Open Patent Application 53-133445), an azo pigment having a triphenylamine skeleton (Japanese Laid-Open Patent Application 53-132347), an azo pigment having a dibenzothiophene skeleton (Japanese Laid-Open Patent Application 54-21728), an azo pigment having an oxadiazole skeleton (Japanese Laid Open Patent Application 54-12742), an azo pigment having a fluorenone skeleton (Japanese Laid-Open Patent Application 54-22834), an azo pigment having a bisstilbene skeleton (Japanese Laid-Open Patent Application 54-17733), an azo pigment having a distyryl oxadiazole skeleton (Japanese Laid-Open Patent Application 54-2129), and an azo pigment having a distyryl carbazole skeleton (Japanese Laid-Open Patent Application 54-14967); indigo pigments such as C.I. Pigment Blue 16 (C.I. 74100) C.I. Vat Brown 5 (C.I. 73410) and C.I. Vat Dye (C.I. 73030); and perylene pigments such as Algol Scarlet B and Indanthrene Scarlet R (made by Bayer Co. Ltd.). Two or more organic pigments mentioned above may be used in combination with the product according to the present invention.

In the layered photoconductor in which the charge generation layer and the charge transport layer are successively overlaid on the electroconductive support in this order, it is preferable that the amount of the product in the charge generation layer be at least 20 % by weight based on the binder resin for use in the charge generation layer. The thickness of the charge generation layer is preferably in the range of 0.01 to 5 µm. In this case, it is preferable that the amount of the charge transport material in the charge transport layer be in the range of 20 to 200 wt % based on the binder resin for use in the charge transport layer. The thickness of the charge transport layer is preferably in the range of 5 to 100 µm. The charge transport layer may be formed using a high-molecular weight charge transport material alone.

It is preferred that the charge generation layer contain a charge transport material for reasons of reducing the residual potential and improving the photosensitivity. The charge transport material is added to the charge generation layer in an amount of 20 to 200 % by weight based on the binder resin for use in the charge generation layer.

In the single-layered photoconductive layer, it is preferable that the amount of the product be in the range of 5 to 95 % by weight based on the binder resin for use in the photoconductive layer. In this case, the thickness of the single-layered photoconductive layer is preferably in the range of 10 to 100 µm. When a charge transport material is added to the single-layered photoconductive layer, it is preferable that the amount of the charge transport material be in the range of 30 to 200 % by weight based on the binder resin for use in the single-layered photoconductive layer. There can be employed a photoconductive layer comprising a high-molecular weight charge transport material and the product according to the present invention. In this case, it is preferable that the amount of the product be in the range of 5 to 95 % by weight based on the high-molecular weight charge transport material. In this case, it is preferable that the thickness of the photoconductive layer be in the range of 10 to 100 µm.

To improve the chargeability, the photoconductive layer may further comprise a phenol compound, a hydroquinone compound, a hindered phenol compound, a hindered amine compound, and a compound having a hindered amine and a hindered phenol in a molecule thereof.

As the electroconductive support, there can be employed a metallic plate, drum or foil made of aluminum, nickel, copper, titanium, gold or stainless steel; a plastic film on which an electroconductive material such as aluminum, nickel, copper, titanium, gold, tin oxide or indium oxide is deposited; and a sheet of paper or a plastic film, which may be formed in a drum, coated with an electroconductive material.

The electrophotographic photoconductor of the present invention may further comprise an intermediate layer which is provided between the electroconductive support and the photoconductive layer, as previously mentioned. The intermediate layer comprises a resin as the main component. Since the photoconductive layer is provided on the intermediate layer by a coating method using a solvent, it is desirable that the resin for use in the intermediate layer have high resistance against general-purpose organic solvents. Preferable examples of the resin for use in the intermediate layer include watersoluble resins such as polyvinyl alcohol, casein and sodium polyacrylate; alcohol-soluble resins such as copolymer nylon and methoxymethylated nylon; and hardenable resins with three-dimensional network such as polyurethane, melamine resin, phenolic resin, alkyd-melamine resin and epoxy resin. The intermediate layer may further comprise finely-divided particles of metallic oxides such as titanium oxide, silica, alumina, zirconium oxide, tin oxide and indium oxide in order to prevent the occurrence of moire and reduce the residual potential. Similar to the previously mentioned photoconductive layer, the intermediate layer can be provided on the electroconductive support by coating method, using an appropriate solvent. Further, the intermediate layer for use in the present invention may be prepared using a coupling agent such as a silane coupling agent, titanium coupling agent or chromium coupling agent. Furthermore, to prepare the intermediate layer, Al₂O₃ may be deposited on the electroconductive support by anodizing process, or an organic material such as poly-para-xylylene (parylene), or an inorganic material such as SiO₂, SnO₂, TiO₂, ITO or Ce0₂ may be deposited on the electroconductive support by vacuum thin film forming method. It is proper that the thickness of the intermediate layer be 5 µm or less.

The electrophotographic photoconductor according to the present invention may further comprise a protective layer which is provided on the photoconductive layer, as previously mentioned. The protective layer for use in the present invention comprises a resin. Examples of such a resin for use in the protective layer include ABS resin, ACS resin, copolymer of olefin and vinyl monomer, chlorinated polyether, allyl resin, phenolic resin, polyacetal polyamide, polyamideimide, polyacrylate, polyallyl sulfone, polybutylene, polybutylene terephthalate, polycarbonate, polyether sulfone, polyethylene, polyethylene terephthalate, polyimide, acrylic resin, polymethylpentene, polypropylene, polyphenylene oxide, polysulfone, polystyrene, AS resin, butadiene-styrene copolymer, polyurethane, polyvinyl chloride, polyvinylidene chloride and epoxy resin. The protective layer may further comprise a fluorine-containing resin such as polytetrafluoroethylene, and a sllicone resin to improve the abrasion resistance. In addition, inorganic materials such as titanium oxide, tin oxide and potassium titanate may be dispersed in the above mentioned fluorine-containing resin and silicone resin. The protective layer may be provided on the photoconductive layer by the conventional coating method. The thickness of the protective layer is preferably in the range of about 0.1 to 10 µm. Furthermore, a vacuum-deposited thin film of a-C or a-SiC may be used as the protective layer in the present invention.

The charge transport material for use in the photoconductive layer include a positive hole transport material and an electron transport material.

There can be employed any conventional positive hole transport materials, for example, poly-N-carbazole and derivatives thereof, poly-γ-carbazolyl ethylglutamate and derivatives thereof, a condensation product of pyrene and formaldehyde and derivatives thereof, polyvinyl pyrene, polyvinyl phenanthrene, oxazole derivatives, imidazole derivatives and triphenylamine derivatives. Stilbene compounds of the following formula (3) may also be suitably used for reasons of excellent charge transporting properties: 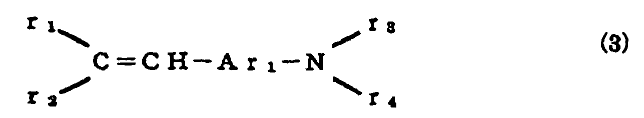
wherein r₁ and r₂ stand, independently from each other, for a substituted or unsubstituted alkyl group or a substituted or unsubstituted aryl group, r₃ and r₄ stand, independently from each other, for a hydrogen atom, a substituted or unsubstituted alkyl group, a substituted or unsubstituted aryl group, or a heterocyclic group, with the proviso that r₁ and r₂ may together form a ring, and Ar₁ stands for a substituted or unsubstituted aryl group or a heterocyclic group.

Illustrative of suitable stilbene compounds are shown in the following tables.

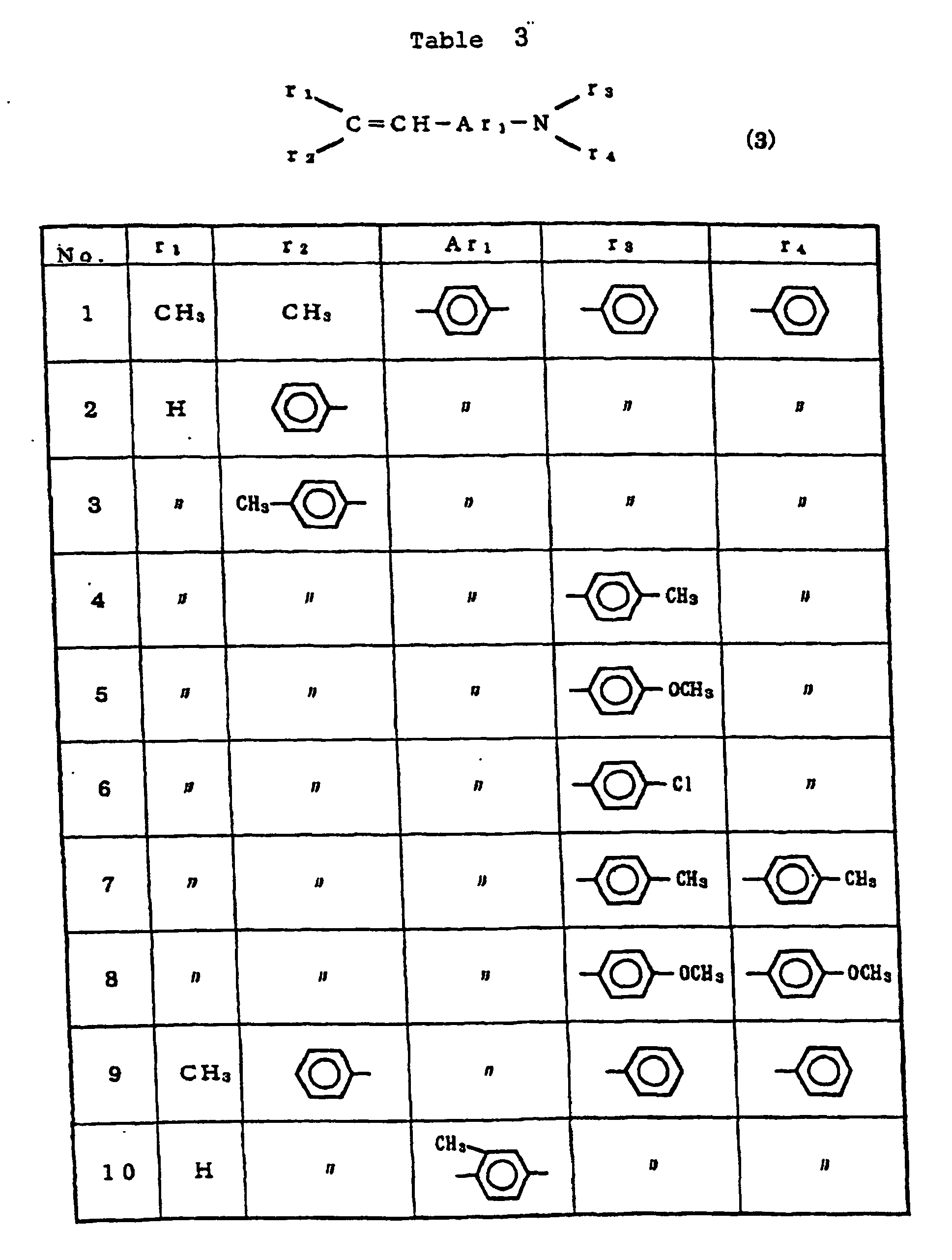

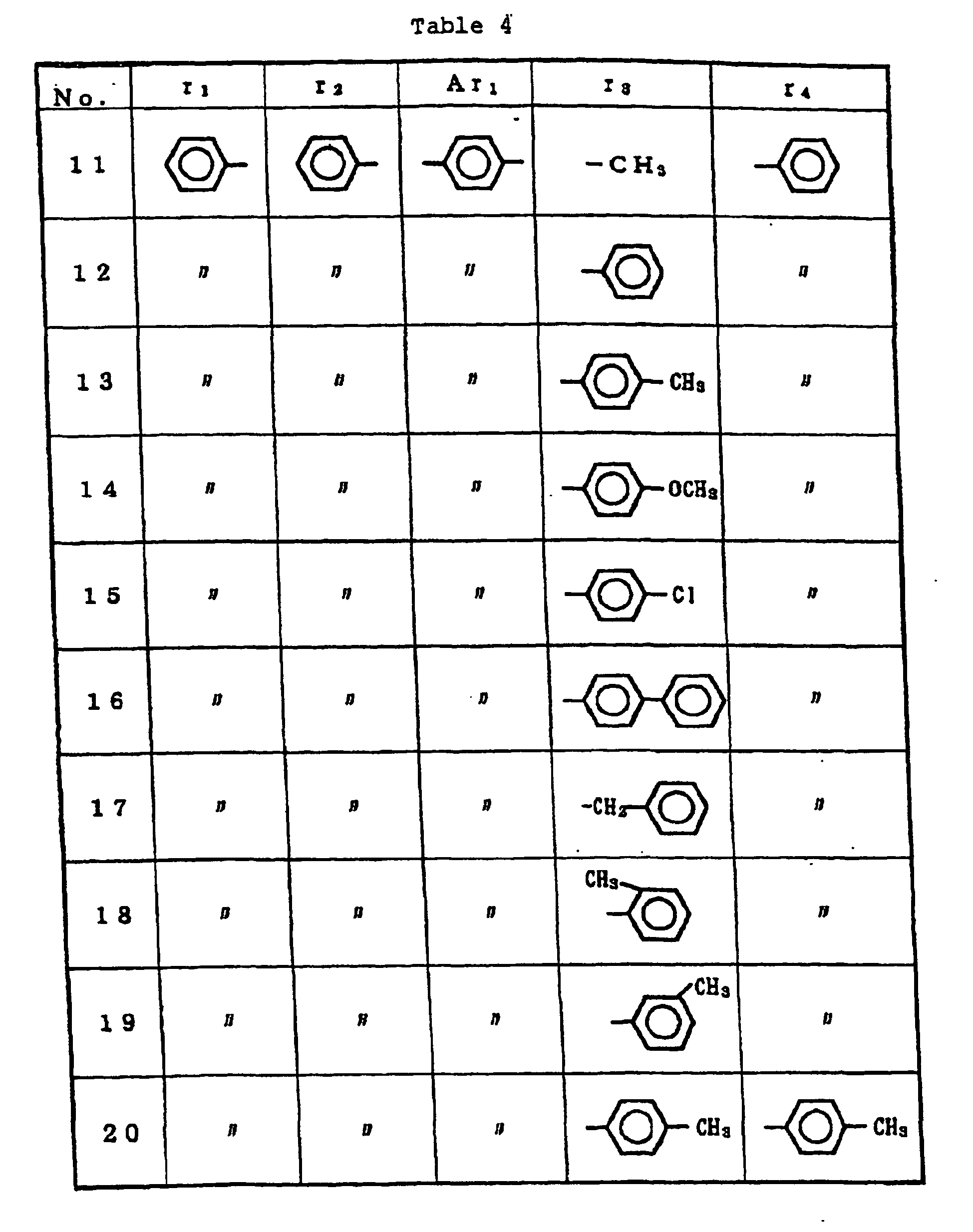

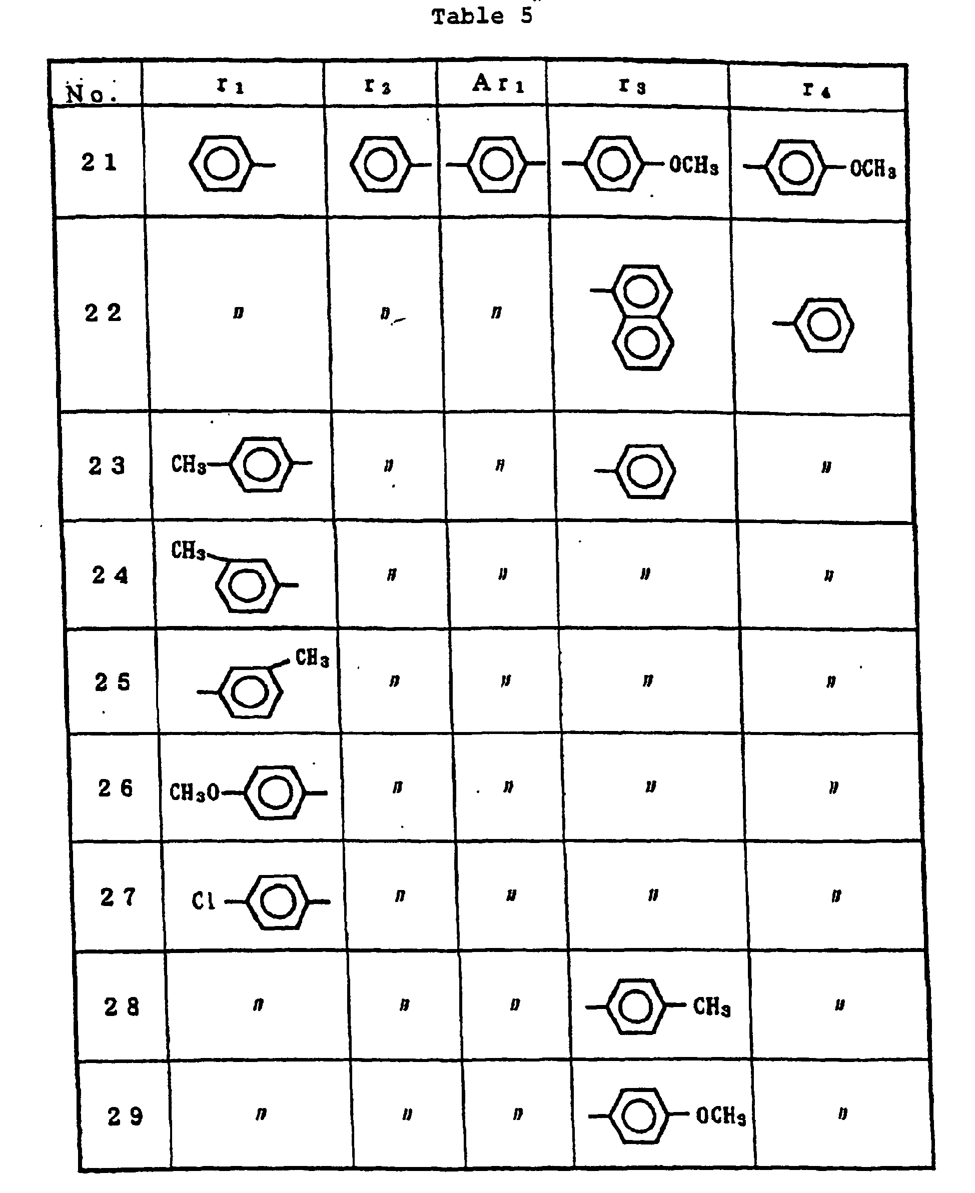

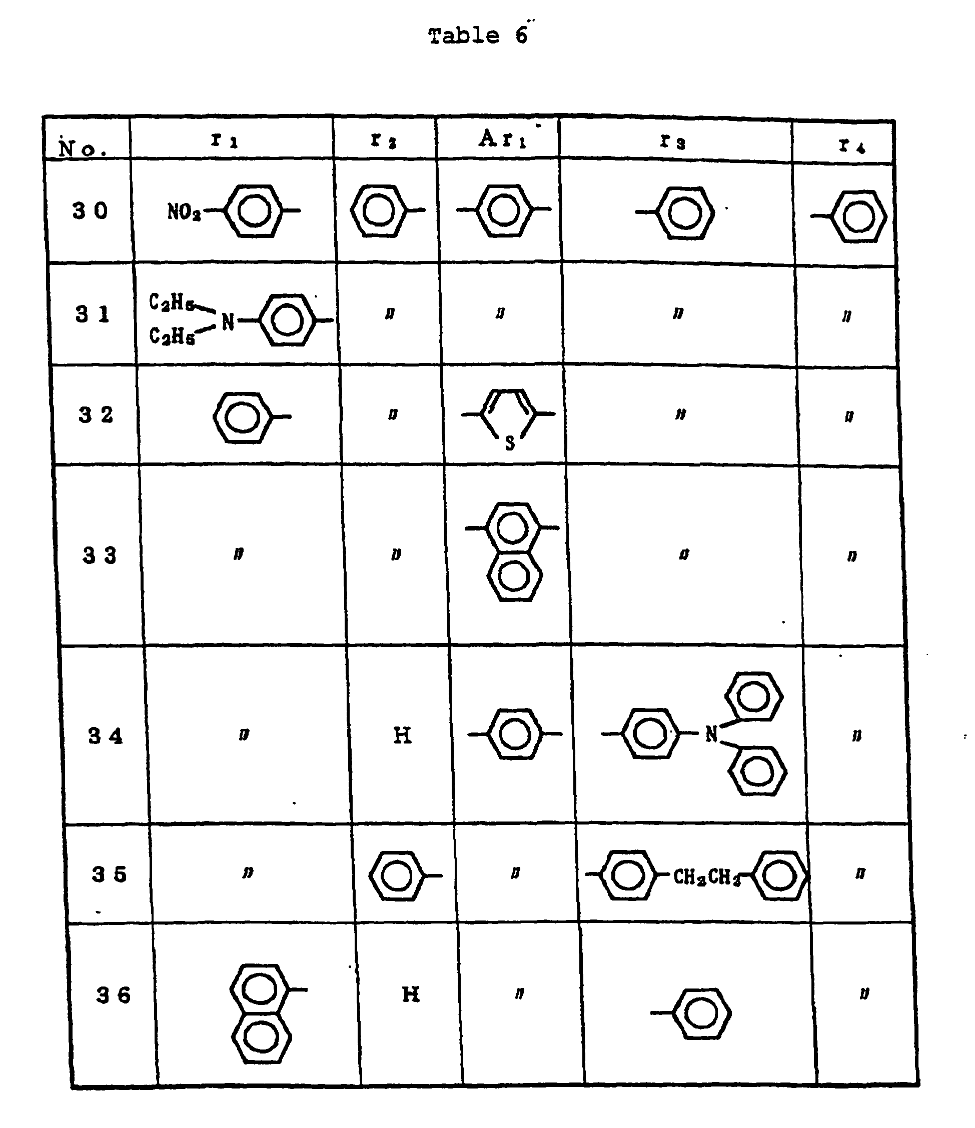

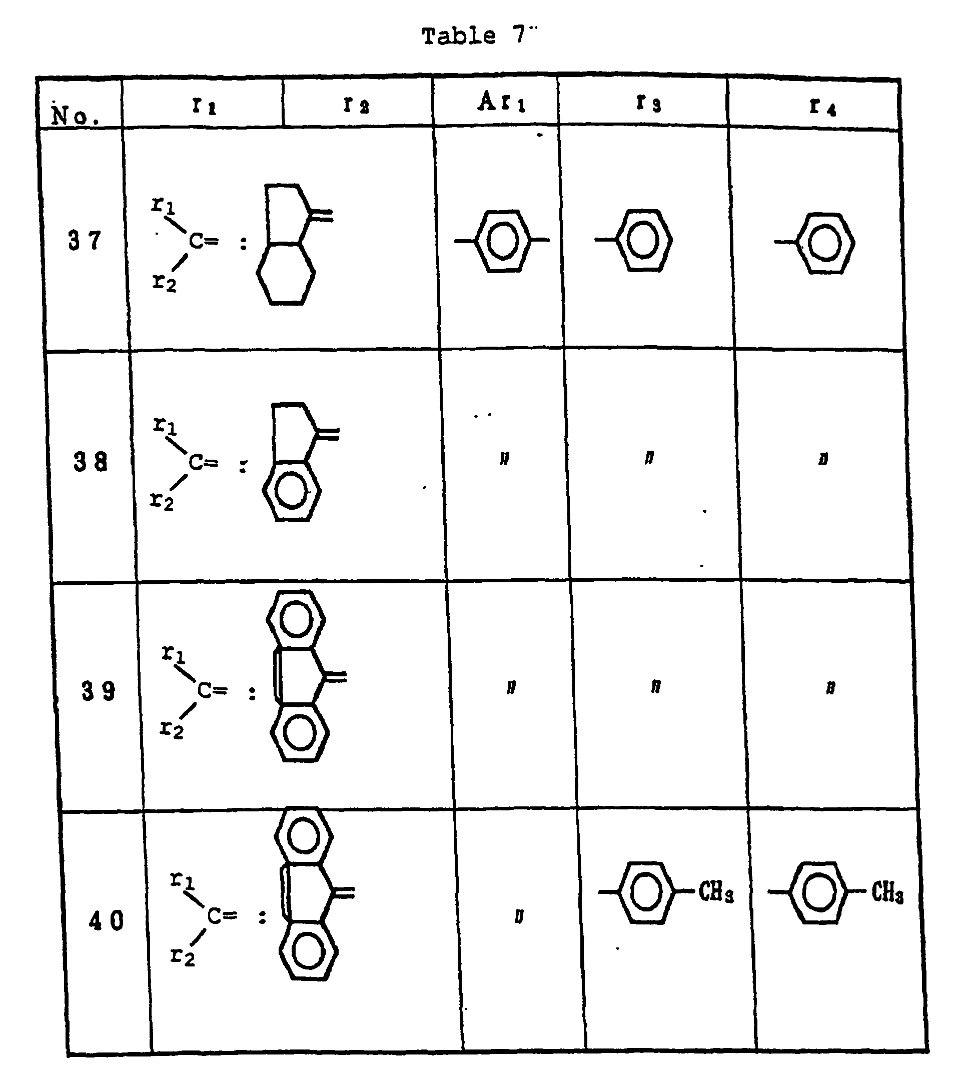

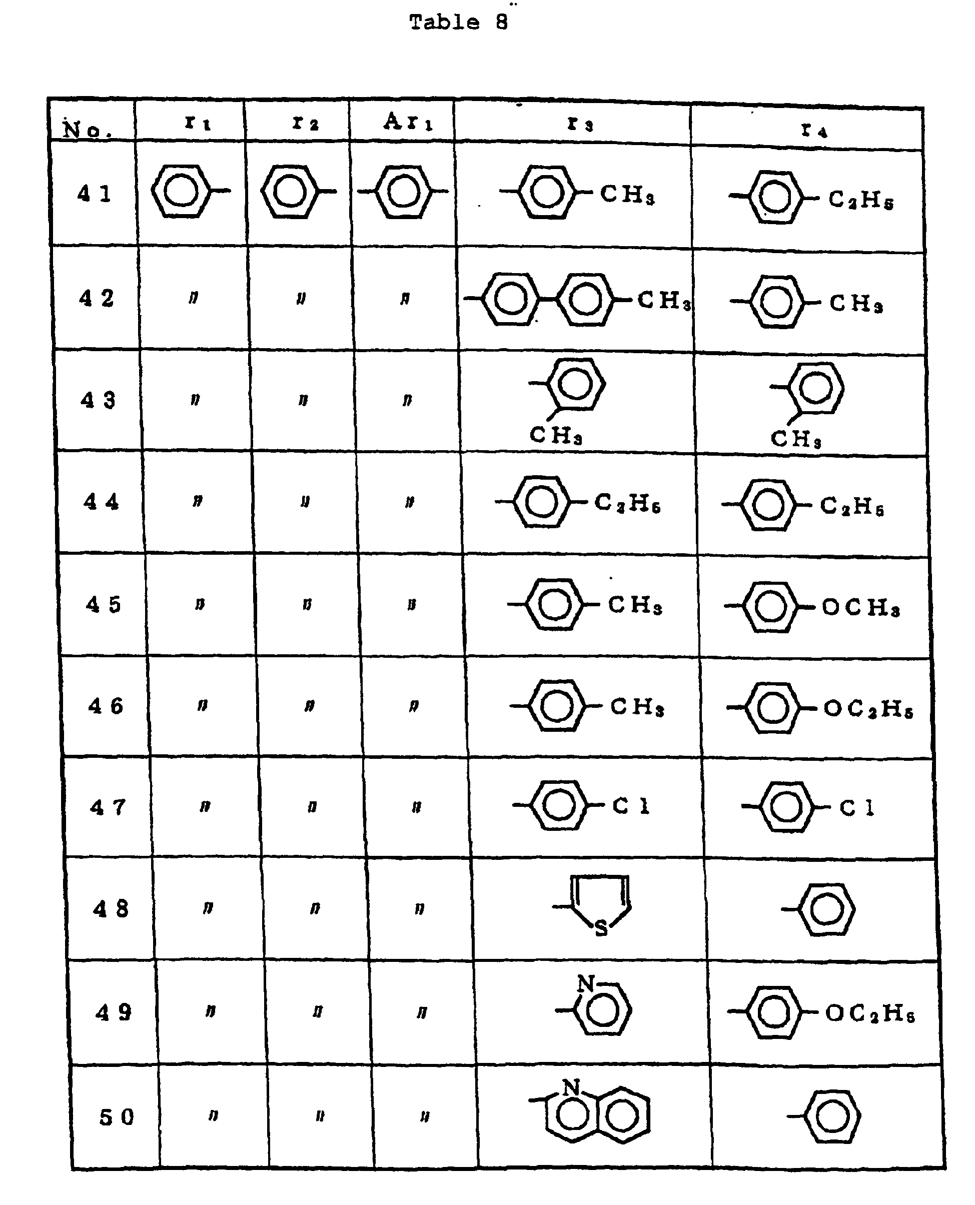

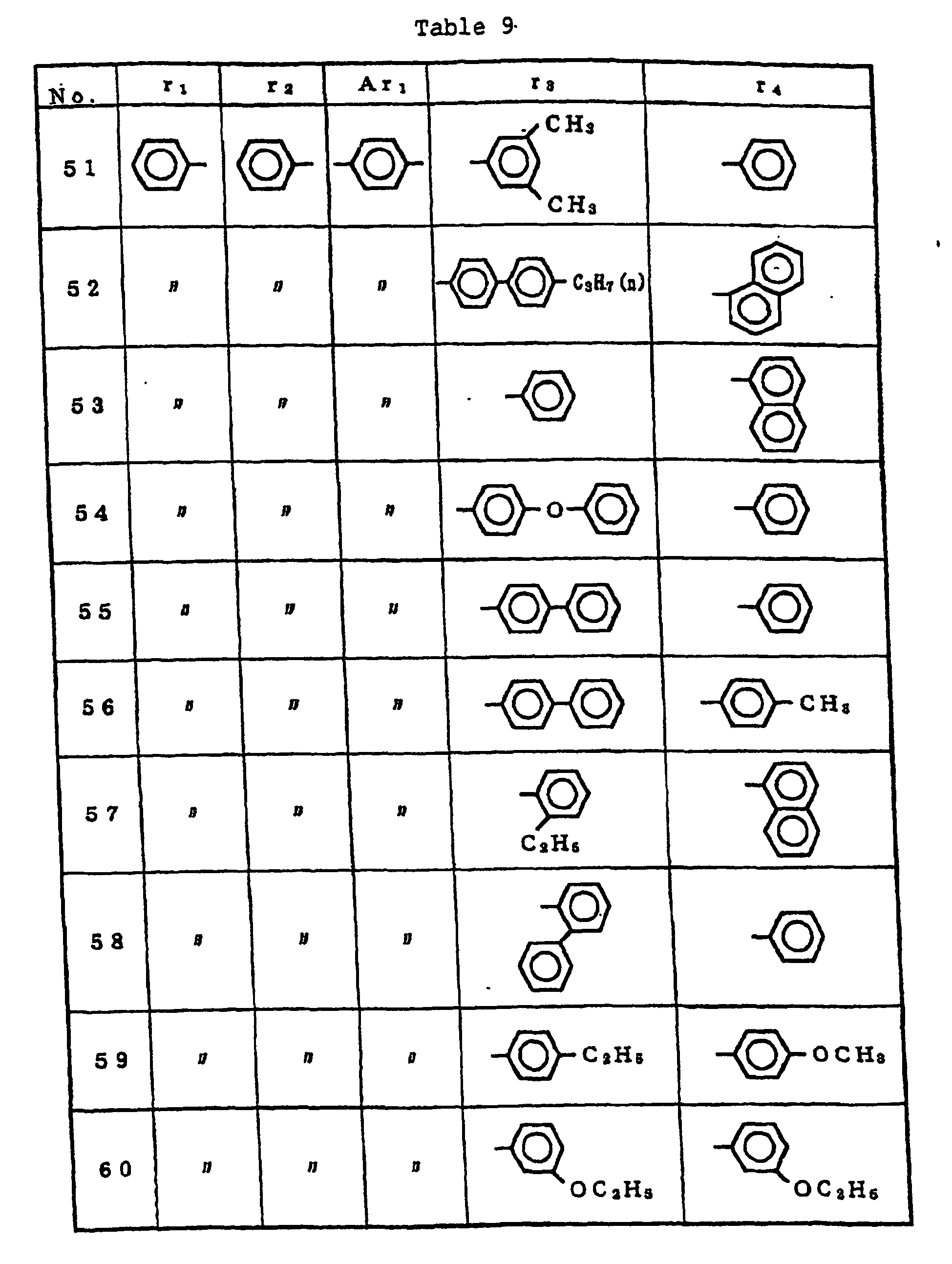

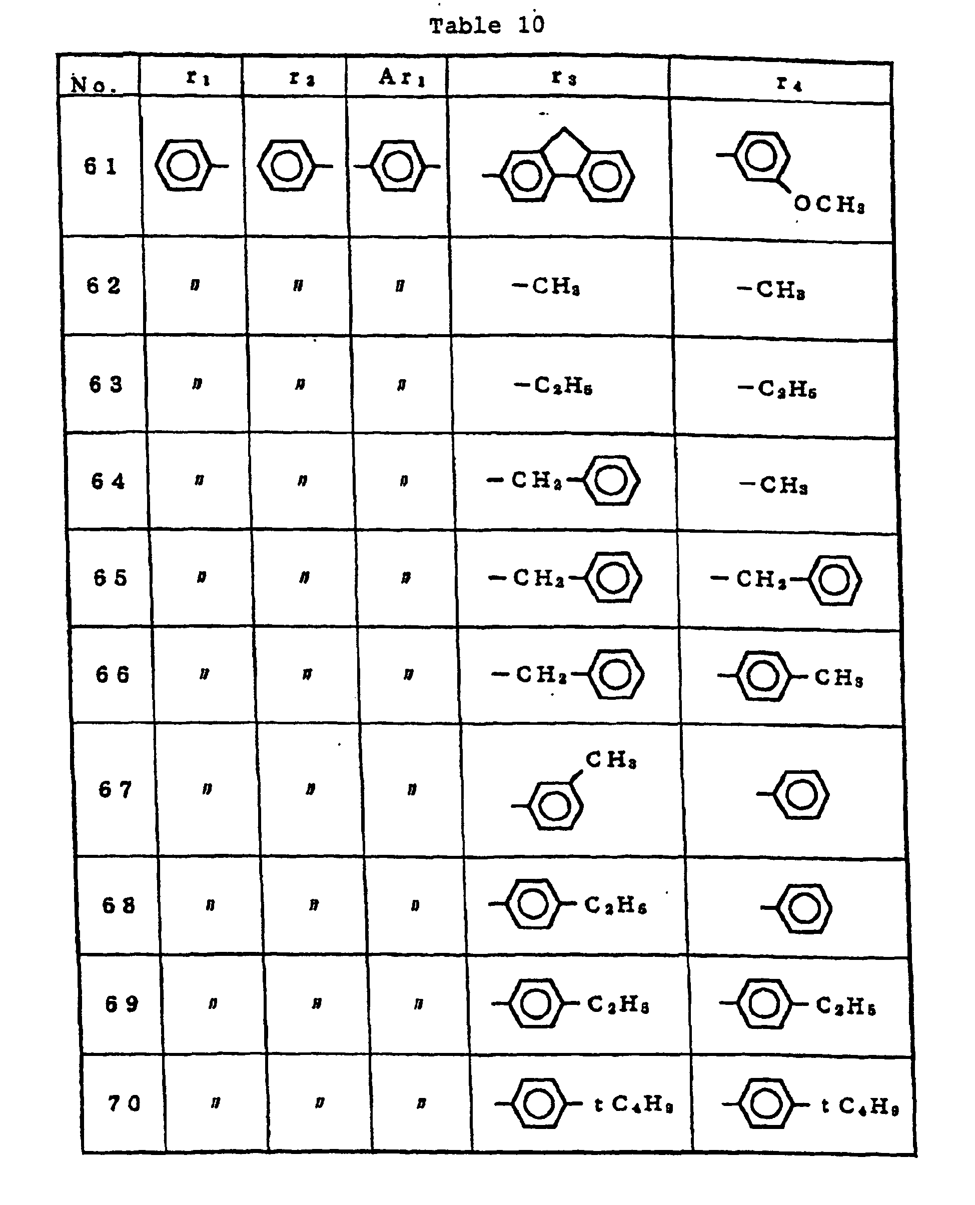

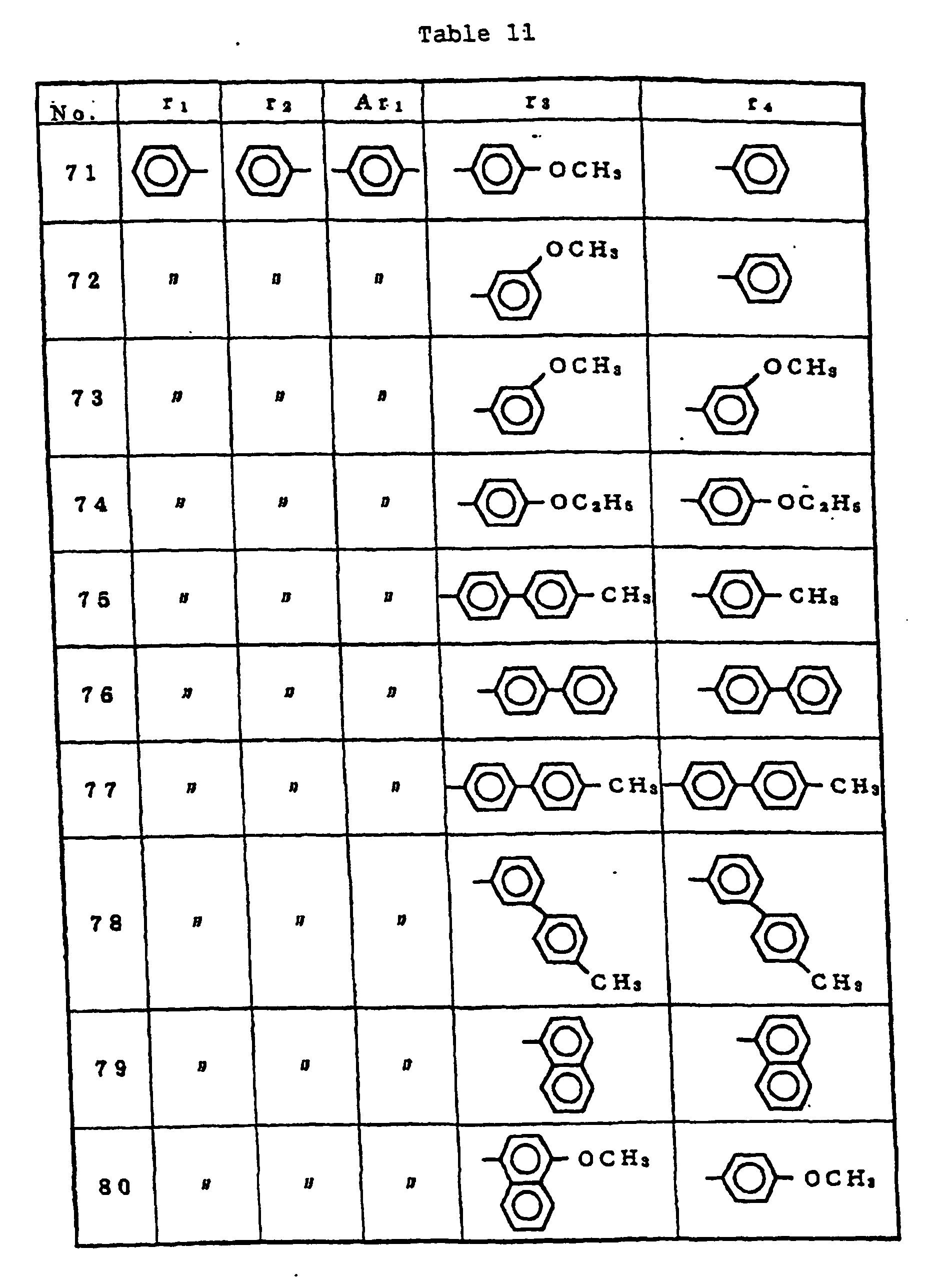

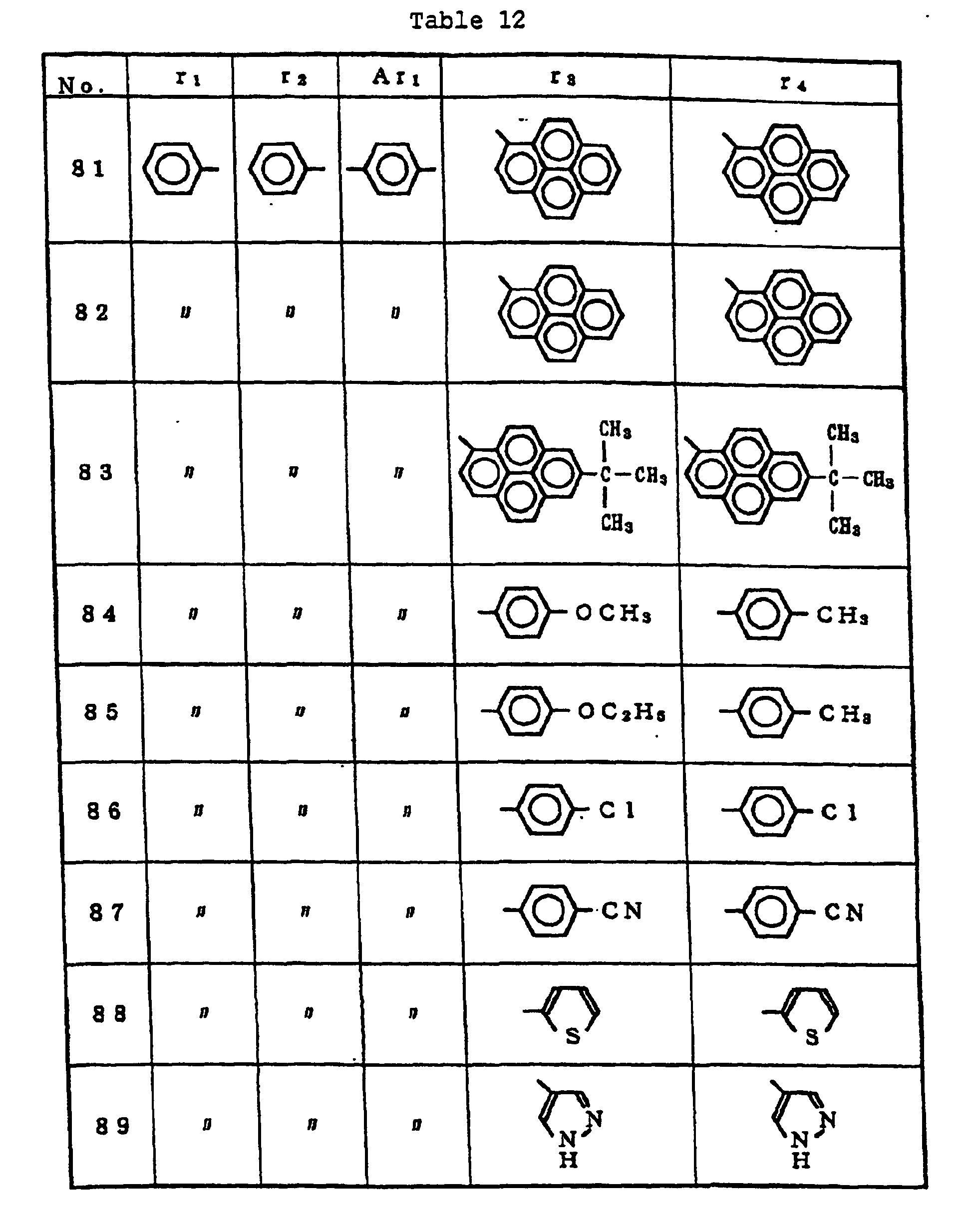

The following compounds (10) to (27) may be also suitably used as the positive hole transport material. Compound (10) [described in Japanese Laid-Open Patent Applications Nos. 55-154955 and 55-156954]: 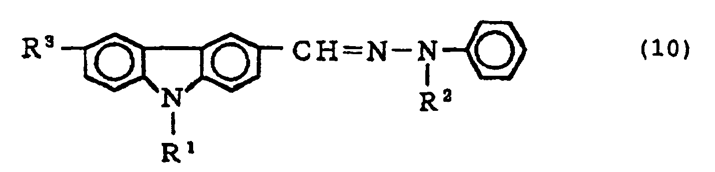
wherein R¹ is methyl group, ethyl group, 2-hydroxyethyl group or 2-chloroethyl group; R² is methyl group, ethyl group, benzyl group or phenyl group; R³ is a hydrogen atom, a chlorine atom, a bromine atom, an alkyl group having 1 to 4 carbon atoms, an alkoxyl group having 1 to 4 carbon atoms, a dialkylamino group or nitro group.

Examples of the above compound (10) are 9-ethylcarbazole-3-aldehyde-1-methyl-1-phenylhydrazone, 9-ethylcarbazole-3-aldehyde-1-benzyl-1-phenylhydrazone, and 9-ethylcarbazole-3 -aldehyde-1,1-diphenylhydrazone. Compound (11) [described in Japanese Laid-Open Patent Application No. 55-52063] 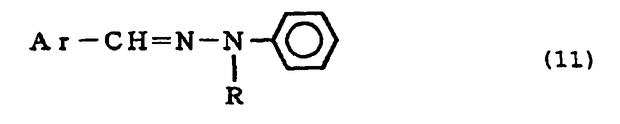
wherein Ar is a naphthalene ring, anthracene ring, styryl ring, each of which may have a substituent, a pyridine ring, furan ring or thiophene ring; and R is an alkyl group or benzyl group. Examples of the above compound of formula (11) are 4-diethylaminostyryl-3-aldehyde-1 - methyl-1-phenylhydrazone, and 4-methoxynaphthalene-1-aldehyde-1-benzyl-1-phenyihydrazone.

### Compound (12) [described in Japanese Laid-Open Patent Application No. 56-81850]:

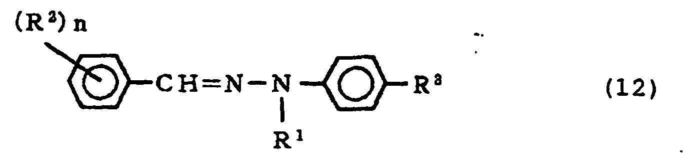

wherein R¹ is an alkyl group, benzyl group, phenyl group or naphthyl group, R² is a hydrogen atom, an alkyl group having 1 to 3 carbon atoms, an alkoxyl group having 1 to 3 carbon atoms, a dialkylamino group, a diaralkylamino group or a diarylamino group, n is an integer of 1 to 4, with the proviso that when n is 2 or more, R² may be the same or different; and R³ is a hydrogen atom or methoxy group.

Examples of the above compound of formula (12) are 4-methoxybenzaldehyde-1-methyl-1-phenylhydrazone, 2,4-dimethoxybenzaldehyde-1-benzyl-1-phenylhydrazone, 4-diethylaminobenzaldehyde-1,1-diphenylhydrazone, 4-methoxybenzaldehyde-1-benzyl-1-(4-methoxy)phenylhydrazone, 4-diphenylaminobenzaldehyde-1-benzyl-1-phenylhydrazone, and 4-dibenzylaminobenzaldehyde-1,1-diphenylhydrazone. Compound (13) [described in Japanese Laid-Open Patent Application No. 51-10983]: 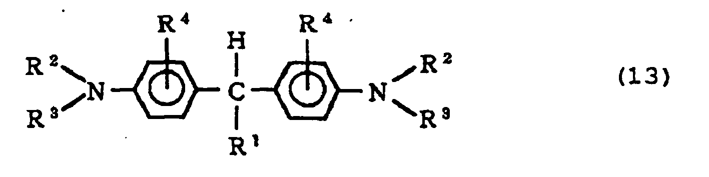
wherein R¹ is an alkyl group having 1 to 11 carbon atoms, a substituted or unsubstituted phenyl group, or a heterocyclic group; R² and R³ are each independently a hydrogen atom, an alkyl group having 1 to 4 carbon atoms, a hydroxyalkyl group, chloroalkyl group, or a substituted or unsubstituted aralkyl group, with the proviso that R² and R³ may form a nitrogen-containing heterocyclic ring in combination; and R⁴, which may be the same or different, each is a hydrogen atom, an alkyl group having 1 to 4 carbon atoms, an alkoxyl group or a halogen atom.

Examples of the above compound of formula (13) are 1,1-bis(4-dibenzylaminophenyl)propane, tris(4-diethylaminophenyl)methane, 1,1-bis(4-dibenzylaminophenyl)propane, and 2,2'-dimethyl-4,4'bis(diethylamino)triphenyimethane. Compound (14) [described in Japanese Laid-Open Patent Application No. 51-94829]: 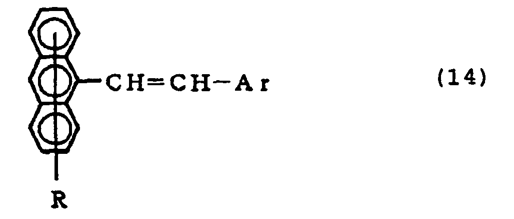
wherein R is a hydrogen atom or a halogen atom and Ar is a substituted or unsubstituted phenyl group, naphthyl group, anthryl group or carbazolyl group.

Examples of the above compound of formula (14) are 9-(4-diethylaminostyryl)anthracene and 9-bromo-10-(4-diethylaminostyryl)anthracene.

### Compound (15) [described in Japanese Laid-Open Patent Application No. 52-128373]:

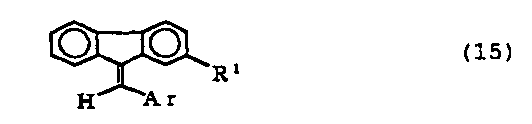

wherein R¹ is a hydrogen atom, a halogen atom, a cyano group, an alkoxyl group having 1 to 4 carbon atoms, or an alkyl group having 1 to 4 carbon atoms; and Ar is 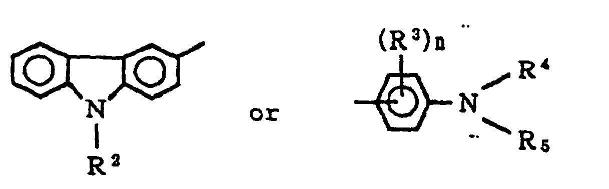
where R¹ is an alkyl group having 1 to 4 carbon atoms; R³ is a hydrogen atom, a halogen atom, an alkyl group having 1 to 4 carbon atoms, an alkoxyl group having 1 to 4 carbon atoms, or a dialkylamino group; n is an integer of 1 or 2 with the proviso that when n is 2, R³ may be the same or different; and R⁴ and R⁵ are each a hydrogen atom, a substituted or unsubstituted alkyl group having 1 to 4 carbon atoms, or a substituted or unsubstituted benzyl group.

Examples of the above compound of formula (15) are 9-(4-dimethylaminobenzylidene)fluorene, and 3-(9-fluorenylidene)-9-ethylcarbazole.

### Compound (16) [described in Japanese Laid-Open Patent Application No. 56-29245]:

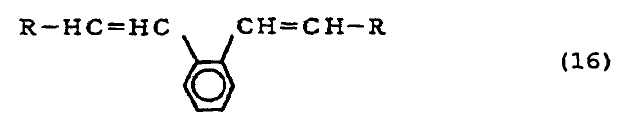

wherein R is a carbazolyl group, pyridine group, thienyl group, indolyl group, furyl group, a substituted or unsubstituted phenyl group, a substituted or unsubstituted styryl group, a substituted or unsubstituted naphthyl group, or a substituted or unsubstituted anthryl group, with the substituent being selected from the group consisting of a dialkylamino group, an alkyl group, an alkoxyl group, a carboxyl group and an ester group thereof, a halogen atom, a cyano group, an aralkylamino group, an N-alkyl-N-aralkylamino group, an amino group, a nitro group and an acetylamino group.

Examples of the above compound of formula (16) are 1,2-bis(4-diethylaminostyryl)benzene, and 1,2-bis (2,4-dimethoxystyryl)benzene.

### Compound (17) [described in Japanese Laid-Open Patent AppllLcatlon No. 58-58552]:

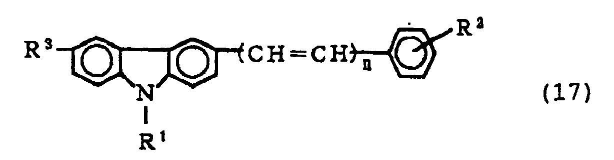

wherein R¹ is a lower alkyl group, a substituted or unsubstituted phenyl group, or benzyl group; R² and R³ are each a hydrogen atom, a lower alkyl group, a lower alkoxyl group, a halogen atom, a nitro group, or an amino group which may have as a substituent a lower alkyl group or benzyl group; and n is an integer of 1 or 2.

Examples of the above compound of formula (17) are 3-styryl-9-ethylcarbazole, and 3-(4-methoxystyryl)-9-ethyl carbazole.

### Compound (18) described in Japanese Laid-Open Patent Application No. 57 73075]:

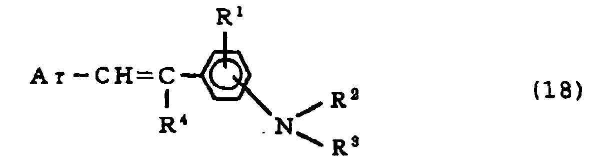

wherein R¹ is a hydrogen atom, an alkyl group, an alkoxyl group or a halogen atom; R² and R³ are each an alkyl group, a substituted or unsubstituted aralkyl group, or a substituted or unsubstituted aryl group; R⁴ is a hydrogen atom or a substituted or unsubstituted phenyl group; and Ar is a substituted or unsubstituted phenyl group or a substituted or unsubstituted naphthyl group.

Examples of the above compound of formula (18) are 4-diphenylaminostilbene, 4-dibenzylaminostilbene, 4-ditolylaminostilbene, 1-(4-diphenylaminostyryl)-naphthalene, and 1-(4-diethylaminostyryl)naphthalene. Compound (19) [described in Japanese Laid-Open Patent Application No. 58-198043]: 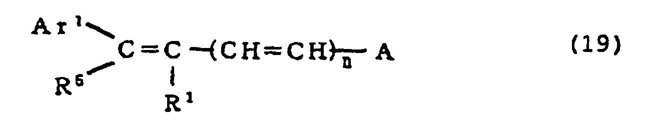
wherein n is an integer of 0 or 1; R¹ is a hydrogen atom, an alkyl group or a substituted or unsubstituted phenyl group; Ar¹ is a substituted or unsubstituted aryl group; R⁵ is a substituted or unsubstituted alkyl group or a substituted or unsubstituted aryl group; and A is 9-anthryl group, a substituted or unsubstituted carbazolyl group or a group of the formula: 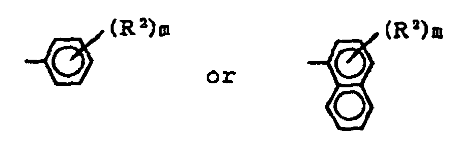
where R² is a hydrogen atom, an alkyl group, an alkoxyl group, a halogen atom or -NR³R⁴ where R³ and R⁴ are each an alkyl group, a substituted or unsubstituted aralkyl group or a substituted or unsubstituted aryl group, with the proviso that R³ and R⁴ may be the same or different, or may form a ring in combination, and m is an integer of 0 to 3, provided that when m is 2 or more R may be the same or different and when n is 0, A and R may form a ring in combination.

Examples of the above compound of formula (19) are 4'-diphenylamino-α-phenylstilbene and 4'-bis(methylphenyl)amino-α-phenylstilbene.

### Compound (20) [described in Japanese Laid-Open Patent Application No. 49-105537]:

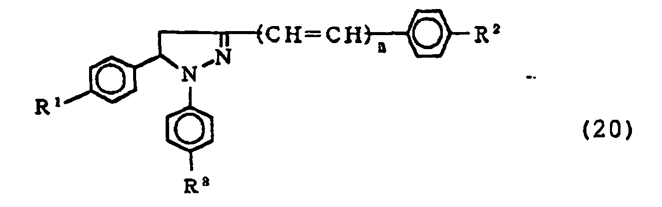

wherein R¹, R² and R³ are each a hydrogen atom, a lower alkyl group, a lower alkoxyl group, a dialkylamino group or a halogen atom; and n is an integer of 0 or 1.

Examples of the above compound of formula (20) include 1-phenyl-3-(4-diethylarninostyryl)-5-(4-diethylaminophenyl)pyrazoline and 1-phenyl-3-(4-dimethylaminostyryl)-5-(4-dimethylaminophenyl)pyrazoline.

### Compound (21) [described in Japanese Laid-Open Patent Application No. 52-139066]:

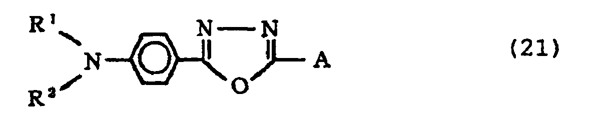

wherein R¹ and R² are each a substituted or unsubstituted alkyl group or a substltuted or unsubstlituted aryl group; and A is a substituted amino group, a substituted or unsubstituted aryl group or an allyl group.

Examples of the above compound of formula (21) are 2,5-bis(4-diethylaminophenyl)-1,3,4-oxadlazole, 2-N,N-diphenylamino-5-(4-diethylaminophenyl)-1,3,4-oxadiazole, and 2-(4-dimethylaminophenyl)-5-(4-diethylaminophenyl)-1,3,4 oxadlazole.

### Compound (22) [described in Japanese T*ald Open Patent Application No. 52-139065]:

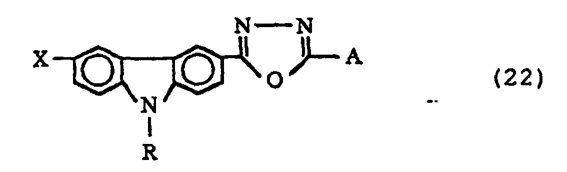

wherein X is a hydrogen atom, a lower alkyl group or a halogen atom; R is a substituted or unsubstituted alkyl group or a substituted or unsubstituted aryl group; and A is a substituted amino group or a substituted or unsubstituted aryl group.

Examples of the above compound of formula (22) are 2-N,N-diphenylamino-5-(N-ethylcarbazole-3-yl)-1,3,4-oxadiazole and 2-(4-diethylaminophenyl)-5-(N-ethylcarbazole-3-yl)-1,3,4-oxadiazole.

### Compound (23) [described in Japanese Laid-Open Patent Application No. 58-32372]:

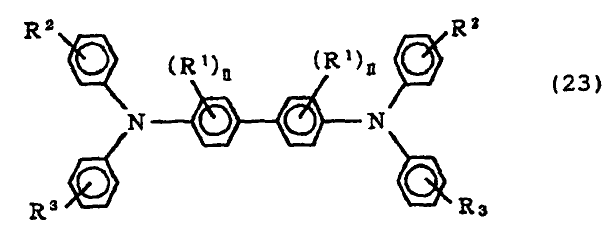

wherein R¹ is a lower alkyl group, a lower alkoxyl group or a halogen atom; n is an integer of 0 to 4; and R² and R³ may be the same or different and are each a hydrogen atom, a lower alkyl group, a lower alkoxyl group or a halogen atom.

Examples of the benzidine compound represented by formula (23) are N,N'-diphenyl-N,N'-bis(3-methylphenyl)-[1, 1'-biphenyl]-4,4'-diamine, and 3,3'-dimethyl-N,N,N',N'-tetrakis(4-methylphenyl)-[1,1'-biphenyl]-4,4'-diamine.

### Compound (24) [described in Japanese Laid-Open Patent Application No. 2-178669]:

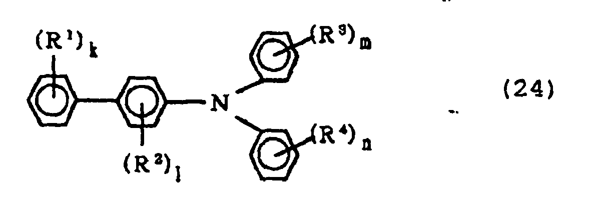

wherein R¹, R³ and R⁴ are each a hydrogen atom, amino group an alkoxyl group, a thioalkoxyl group, an aryloxy group, ma ethylenedioxy group, a substituted or unsubstituted alkyl group, a halogen atom or a substituted or unsubstltuted aryl group; R² is a hydrogen atom, an alkoxyl group, a substituted or unsubstituted alkyl group or a halogen atom with the proviso that R¹, R², R³ and R⁴ are not hydrogen atoms at the same time; and k, 1, m and n are each an integer of 1 to 4 with the proviso that when each is an integer of 2, 3 or 4, R¹, R², R³ and R⁴ may be independently the same or different.

Examples of the biphenylamine compound represented by formula (24) are 4'-methoxy-N,N-diphenyl-[1,1'-biphenyl]-4-amine, 4'-methyl-N, N'-bis(4-methylphenyl)-[1,1'-biphenyl]-4-amine, and 4'-methoxy-N,N'-bis(4-methylphenyl-[1,1'-biphenyl]-4-amine.

### Compound (25) [described in Japanese Laid-Open Patent Application No. 3-285960]:

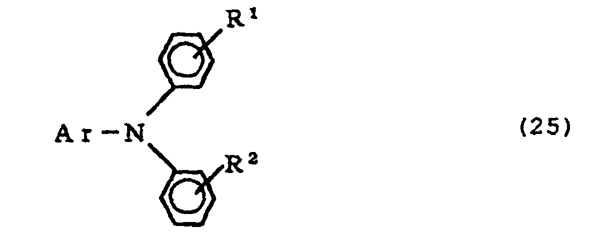

wherein Ar is a condensed polycyclic hydrocarbon group having 18 or less carbon atoms; and R¹ and R² may be the same or different and are each a hydrogen atom, a halogen atom, a substituted or unsubstituted alkyl group, an alkoxyl group or a substituted or unsubstituted phenyl group.

Examples of the triarylamine compound represented by formula (25) are 1-diphenylaminopyrene and 1-di(p-tolylamino)pyrene.

### Compound (26) [described in Japanese Laid-Open Patent Application No. 62 98394]:

A-CH=CH-Ar-CH=CH-A (26)

wherein Ar is a substituted or unsubstituted aromatic hydrocarbon group; and A is 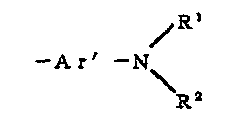
where Ar' is a substituted or unsubstituted aromatic hydrocarbon group; and R¹ and R² are each a substituted or unsubstituted alkyl group or a substituted or unsubstituted aryl group.

Examples of the diolefinic aromatic compound represented by formula (26) are 1,4-bis(4-diphenylaminostyryl) benzene and 1,4-bis[4-di(p-tolyl) aminostyryl]benzene.

### Compound (27) [described in Japanese Laid-Open Patent Application No. 4-230764]:

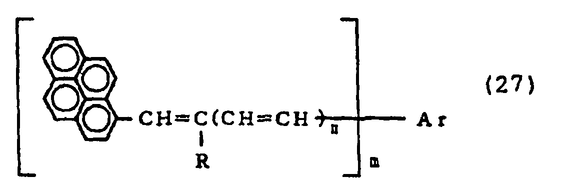

wherein Ar is an aromatic hydrocarbon group; R is a hydrogen atom, a substituted or unsubstituted alkyl group or a substituted or unsubstituted aryl group; and n is an integer of 0 or 1 and m is an integer of 1 or 2, provided that when n=0 and m=1, Ar and R may form a ring in combination.

Examples of the styrylpyrene compound represented by formula (27) are 1-(4-diphenylaminostyryl)pyrene, and 1-[4 -di(p-tolyl)aminostyryl]pyrene.

Electron transport materials for use in the present invention may be, for example, chloroanil, bromoanil, tetracyanoethylene, tetracyanoquinodimethane, 2,4,7-trinitro-9-fluorenone, 2,4,5,7-tetranitro-9-fluorenone, 2,4,5,7-tetranitroxanthone, 2,4,8-trinitrothioxanthone, 2,6,8-trinitroindeno-4H-indeno[1,2-b]thiophen-4-one and 1,3,7-trinitrodibenzothiophene-5,5-dioxide. In particular, 2,3-diphenylindene compound of the following formula (4): 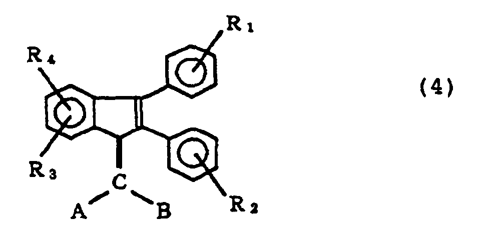
wherein R₁-R₄ stand, independently from each other, for a hydrogen atom, a halogen atom, a substituted or unsubstituted alkyl group, a cyano group or a nitro group, and A and B stand, independently from each other, for a hydrogen atom, a halogen atom, a substituted or unsubstituted aryl group, a cyano group, an alkoxycarbonyl group or an aryloxycarbonyl group,
is preferably employed because of its excellent electron transporting performance. Of the compounds of the formula (4), 2,3-diphenyl-1-indenylidene)marononitrile of the following formula (28) may be particularly suitably used:

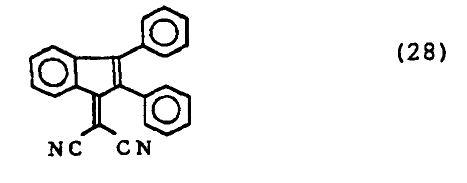

Further, the compounds of the following formulas (29) and (30) may also be preferably used as the electron transport material for reasons of excellent electron transporting performance: 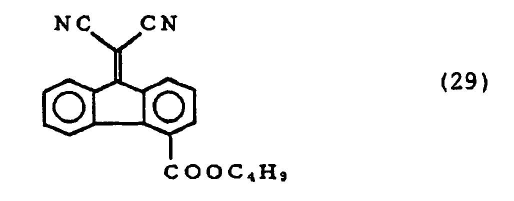 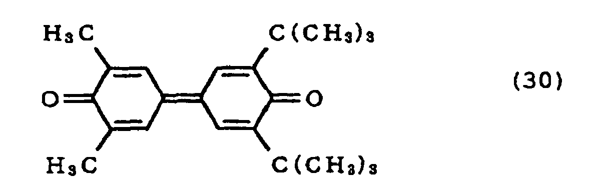 The above-mentioned charge transport materials may be used alone or in combination of two or more.

The product according to the present invention is useful as the photoconductive material for use in the electrophotographic photoconductor, and in addition, as an electronic device for utilization in the field of electronics such as solar batteries and optical disks.

The following examples will further illustrate the present invention

### Example 1

### [Preparation of Product 1-A]

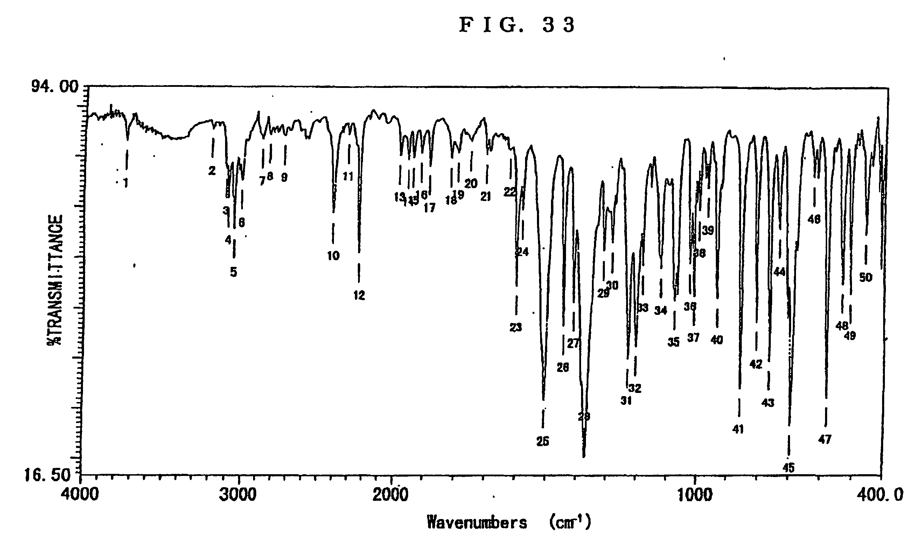

A mixture containing 0.1 mol of 1,4-bisbenzyl, 0.2 mol of diaminomaleonitrile and acetaic acid was refluxed with stirring for 6 hours and then allowed to be cooled to room temperature, thereby precipitating crystals. The crystals were collected by filtration and isolated by column chromatography using chloroform as a developing solvent to obtain a crude product. This was recrystallized from toluene to obtain a nitrile compound represented by the following formula (6) as an intermediate product with a yield of 80 %: 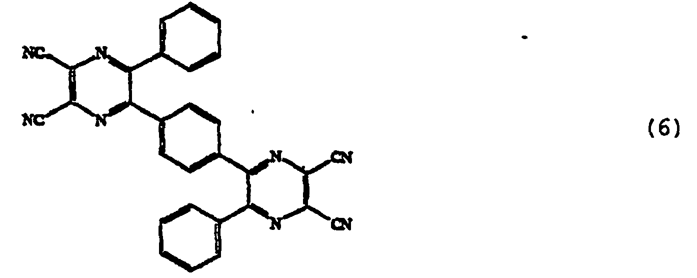
The nitrile compound (6) has a melting point of 270-274°C. An infrared absorption spectrum of the nitrile compound (6) is shown in Fig. 33. The elementary analysis of the nitrile compound (6) gave as follows:

| | C | H | N |
|---|---|---|---|
| Calculated | 74.32 | 2.74 | 23.21 |
| Found | 74.06 | 2.90 | 23.03 |

A mixture containing 0.2 mole of the nitrile compound (6), 0.2 mol of phthalonitrile, 0.1 mol of cuprous chloride and 200 ml of α-chloronaphthalene was gradually heated to 170°C and maintained at 170-180°C with stirring under a nitrogen gas stream for 5 hours. After completion of the reaction, the reaction mixture was allowed to be cooled to 130°C and immediately filtered. The solid phase was then washed well with α - chloronaphthalene and then several times with methanol, toluene and water and dried to obtain Product 1-A with a yield of 76 %.

### Example 2

### [Preparation of Product 1-B]

A mixture containing 0.5 mole of the nitrile compound (6) obtained in Example 1, 0.3 mol of phthalonitrile, 0.11 mol of Ti(OBu)₄ and 200 ml of octanol was gradually heated to 160°C and maintained at 150-160°C with stirring under a nitrogen gas stream for 6 hours. After completion of the reaction, the reaction mixture was allowed to be cooled and filtered. The solid phase was then washed well with octanol and then several times with methanol, toluene and water and dried to obtain Product 1-B with a yield of 75 %.

Using a mass spectrometer (LC/MS Mass Analyzer MS700 manufactured by JEOL), the Product 1-B was analyzed under the following conditions:
Ionization: Electrospray ionization (ESI) +ion mode
Flow rate: 25 µL/min
Injection mode: infusion
Ring voltage: 80V
Skimmer voltage: 0V
Sample: Product 1-B was dissolved in formic acid and the solution was diluted with 50 % aqueous formic acid to a concentration of 50 ppm

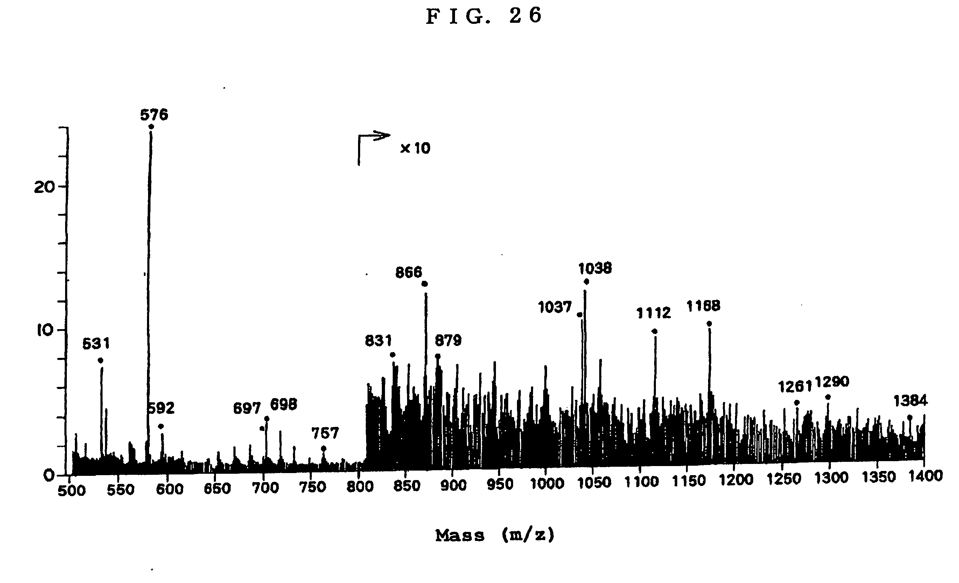

The results of the mass spectrometry are shown in Fig. 26. Two peaks corresponding to molecular weight of 1382 and 576 are observed, suggesting that Product No. 1-B contains the compounds represented by the above formulas (8) and (9)

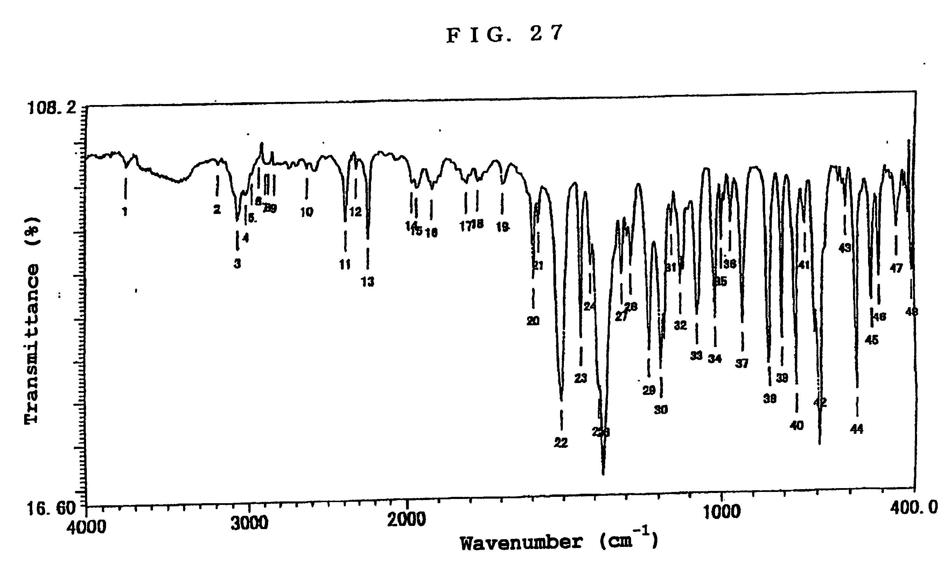

Fig. 27 is an infrared absorption spectrum of Product No. 1-B. The fluorescent X-ray spectroscopy revealed the presence of Ti in Product No. 1-B.

The above procedure was repeated 10 times in total. It was found that the products obtained showed the same MS and IR spectra as shown in Figs. 26 and 27, indicating that Product 1-B was able to be produced with high reproducibility.

### Example 3

A mixture containing 0.0016 mole of the nitrile compound (6) obtained in Example.1, 0.32 mol of phthalonitrile, 0.09 mol of Ti(OBu)₄, 0.16 mole of urea and 200 ml of octanol was gradually heated to 150°C and maintained at 150-160°C with stirring under a nitrogen gas stream for 6 hours. After completion of the reaction, the reaction mixture was allowed to be cooled and filtered. The solid phase was then washed well with octanol and then several times with methanol, toluene and water and dried to obtain Product 1-B with a yield of 75 %. This product (20 g) was then subjected to an acid treatment using an acid pasting method. Thus, the product was dissolved little by little with stirring in 200 g of concentrated sulfuric acid while being cooled in an ice bath. The mixture was then reacted for 1 hour and subsequently poured into 2000 ml of ice water to form crystals. The crystals were separated by filtration and washed with distilled water until no acid was detected in the washed water. After drying, 18 g of an acid-treated product was obtained.

### Example 4

### [Preparation of Product 2-B]

A nitrile compound (31) was prepared according to the following reaction scheme (DAMN means diaminomaleonitrile):

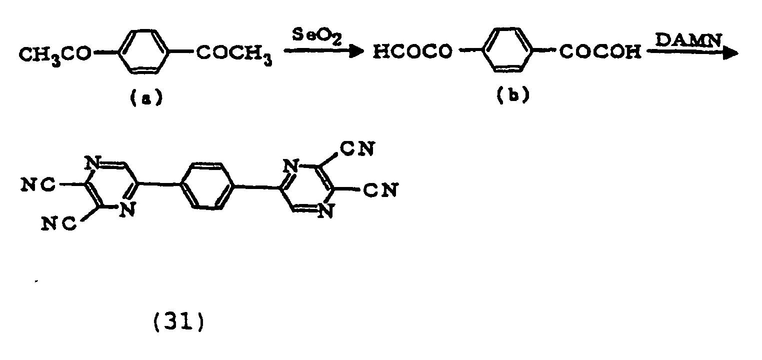

In a reactor, 0.15 mol of SeO₂, 120 ml of dioxane and 5 ml of water were charged and heated at 50°C for 1 hour with stirring, to which a solution of 0.05 ml of (a) dissolved in 20 ml of dioxane was added dropwise. The mixture was then reacted for 8 hours under reflux. Thereafter, the reaction mixture, while hot, was filtered to remove solids. The solvent in the filtrate was removed by distillation to leave (b).

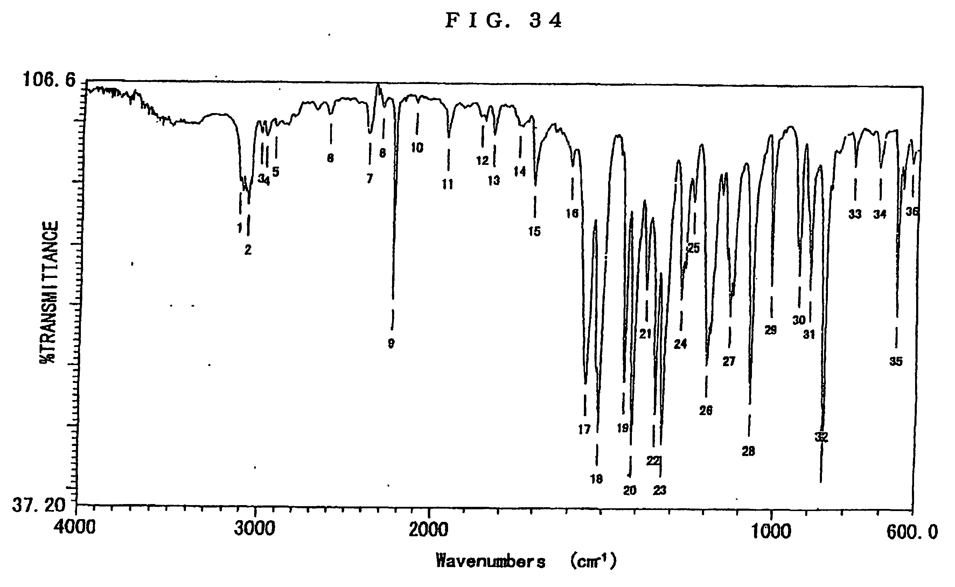

The product (b) was mixed with 0.1 mol of diaminomaleonitrile, 0.01 mol of p-toluenesulfonic acid and 200 ml of dioxane. The mixture was refluxed for 2 hours and then allowed to be cooled to room temperature, followed by removal of the solvent by distillation. The residue was mixed with 200 ml of methanol to precipitate crystals. The crystals were collected by filtration, washed several times with methanol and dried. The crystals were then isolated by column chromatography using dichloromethane as a developing solvent to obtain a crude product. This was recrystallized from dioxane to the obtain nitrile compound (31) with a yield of 60 %. An infrared absorption spectrum of the nitrile compound (31) is shown in Fig. 34. The elementary analysis of the nitrile compound (31) gave as follows:

| | C | H | N |
|---|---|---|---|
| Calculated | 64.67 | 1.81 | 33.52 |
| Found | 64.50 | 1.92 | 33.34 |

A mixture containing 0.0016 mole of the nitrile compound (31), 0.32 mol of phthalonitrile, 0.09 mol of Ti(OBu)₄, 0.16 mole of urea and 200 ml of octanol was gradually heated to 150°C and maintained at 150-160°C with stirring under a nitrogen gas stream for 6 hours. After completion of the reaction, the reaction mixture was allowed to be cooled and filtered. The solid phase was then washed well with octanol and then several times with methanol, toluene and water and dried to obtain Product 2-B with a yield of 70 %.

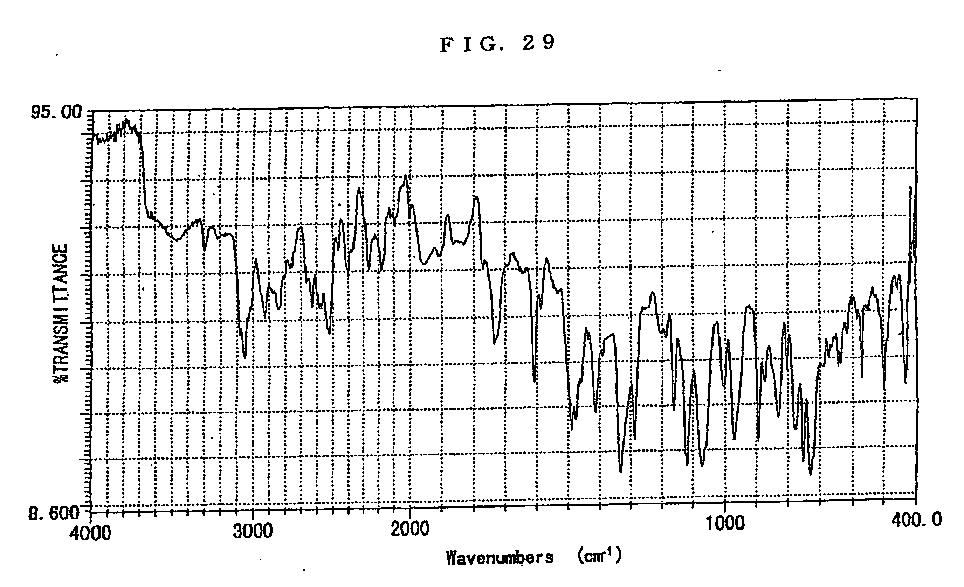

This product (20 g) was then subjected to an acid treatment using an acid pasting method. Thus, the product was dissolved little by little with stirring in 200 g of concentrated sulfuric acid while being cooled in an ice bath. The mixture was then reacted for 1 hour and subsequently poured into 2000 ml of ice water to form crystals. The crystals were separated by filtration and washed with distilled water until no acid was detected in the washed water. After drying, 18 g of an acid-treated product was obtained. An infrared absorption spectrum of acid-treated Product 2-B is shown in Fig. 29.

### Example 5

### [Preparation of Product 2-D]

A nitrile compound (32) was prepared according to the following reaction scheme (DAMN means diaminomaleonitrile):

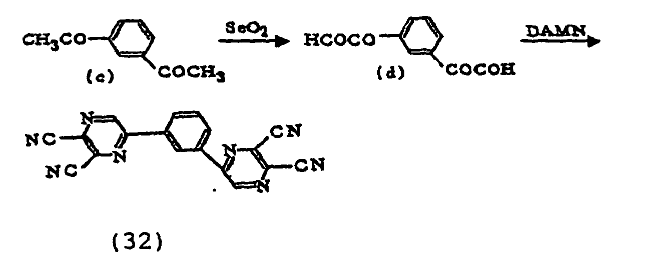

In a reactor, 0.12 mol of SeO₂, 120 ml of dioxane and 4 ml of water were charged and heated at 50°C for 1 hour with stirring, to which a solution of 0.05 ml of (c) dissolved in 20 ml of dioxane was added dropwise. The mixture was then reacted for 8 hours under reflux. Thereafter, the reaction mixture, while hot, was filtered to remove solids. The solvent in the filtrate was removed by distillation to leave (d).

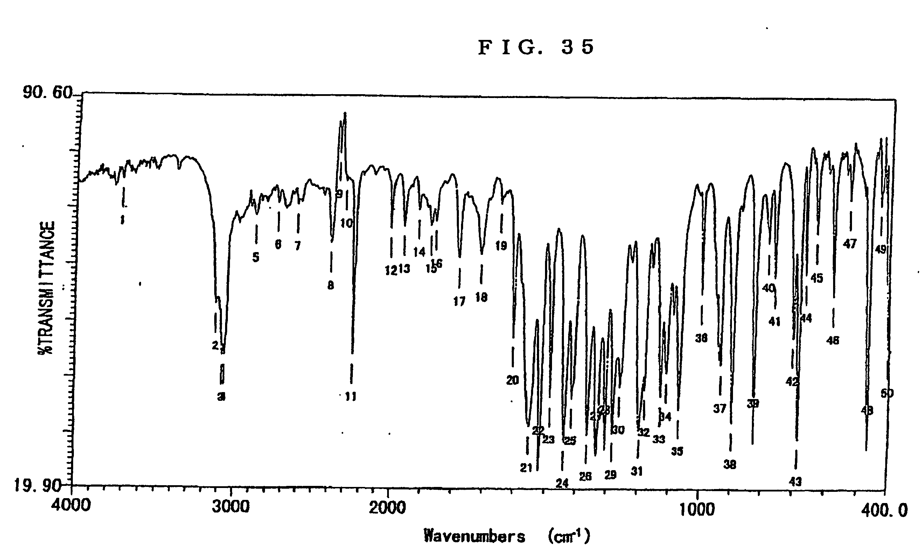

The product (d) was mixed with 0.1 mol of diaminomaleonitrile, 0.01 mol of p-toluenesulfonic acid and 200 ml of dioxane. The mixture was refluxed for 2 hours and then allowed to be cooled to room temperature, followed by removal of the solvent by distillation. The residue was mixed with 200 ml of methanol to precipitate crystals. The crystals were collected by filtration, washed several times with methanol and dried. The crystals were then isolated by column chromatography using dichloromethane as a developing solvent to obtain a crude product. This was recrystallized from dioxane to obtain the nitrile compound (32) as an intermediate product with a yield of 55 %. The nitrile compound (32) was found to have a melting point of 279-284°C. An infrared absorption spectrum of the nitrile compound (32) is shown in Fig. 35. The elementary analysis of the nitrile compound (32) gave as follows:

| | C | H | N |
|---|---|---|---|
| Calculated | 64.67 | 1.81 | 33.52 |
| Found | 64.55 | 1.87 | 33.40 |

A mixture containing 0.0016 mole of the nitrile compound (32), 0.32 mol of phthalonitrile, 0.09 mol of Ti(OBu)₄, 0.16 mole of urea and 200 ml of octanol was gradually heated to 150°C and maintained at 150-160°C with stirring under a nitrogen gas stream for 6 hours. After completion of the reaction, the reaction mixture was allowed to be cooled and filtered. The solid phase was then washed well with octanol and then several times with methanol, toluene and water and dried to obtain Product 2-D with a yield of 71 %.

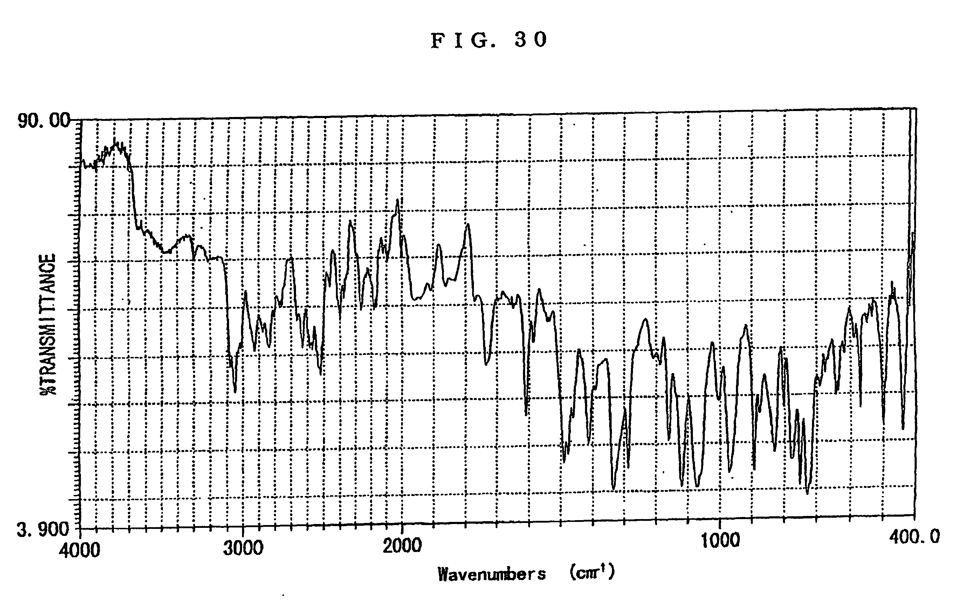

This product (20 g) was then subjected to an acid treatment using an acid pasting method. Thus, the product was dissolved little by little with stirring in 200 g of concentrated sulfuric acid while being cooled in an ice bath. The mixture was then reacted for 1 hour and subsequently poured into 2000 ml of ice water to form crystals. The crystals were separated by filtration and washed with distilled water until no acid was detected in the washed water. After drying, 18 g of an acid-treated product was obtained. An infrared absorption spectrum of acid-treated Product 2-D is shown in Fig. 30.

### Example 6

### [Treatment of Product 1-B]

The acid-treated product (1 g) obtained in Example 3 was placed in an Erlenmeyer flask together with 20 ml of N,N-dimethylformamide and heated under reflux conditions for 4 hours. The mixture was cooled to room temperature and filtered to collect solids. The solids were dried to obtain 0.96 g of a solvent-treated Product 1-B.

### Example 7

### [Treatment of Product 1-B]

The acid-treated product (1 g) obtained in Example 3 was placed in an Erlenmeyer flask together with 20 ml of methyl ethyl ketone and heated under reflux conditions for 4 hours. The mixture was cooled to room temperature and filtered to collect solids. The solids were dried to obtain 0.97 g of a solvent-treated Product 1-B.

### Example 8

### [Treatment of Product 1-B]

The acid-treated product (1 g) obtained in Example 3 was placed in an Erlenmeyer flask together with 20 ml of o-chlorobenzene and heated under reflux conditions for 4 hours. The mixture was cooled to room temperature and filtered to collect solids. The solids were dried to obtain 0.97 g of a solvent-treated Product 1-B.

### Example 9

### [Treatment of Product 1-B]

The acid-treated product (1 g) obtained in Example 3 was placed in an Erlenmeyer flask together with 20 ml of cyclohexanone and heated under reflux conditions for 4 hours. The mixture was cooled to room temperature and filtered to collect solids. The solids were dried to obtain 0.98 g of a solvent-treated Product 1-B.

### Example 10

### [Treatment of Product 1-B]

A mixture containing 0.0016 mole of the nitrile compound (6) obtained in Example 1, 0.32 mol of phthalonitrile, 0.09 mol of Ti(OBu)₄, 0.16 mole of urea and 200 ml of octanol was gradually heated to 150°C and maintained at 150-160°C with stirring under a nitrogen gas stream for 6 hours. After completion of the reaction, the reaction mixture was allowed to be cooled and filtered. The solid phase was then washed well with octanol and then several times with methanol, toluene and water and dried to obtain Product 1-B with a yield of 75 %. This product (5 g) was dissolved little by little with stirring in 50 g of concentrated sulfuric acid while being cooled in an ice bath. The mixture was then reacted for 1 hour and subsequently poured into 500 ml of ice water to form crystals. The crystals were separated by filtration and washed with distilled water until no acid was detected in the washed water, thereby obtaining 20 g of an acid-treated Product 1-B in the form of a wet cake (solid matter content: 20 %).

### Example 11

### [Treatment of Product 1-B]

The wet cake (15 g) obtained in Example 10 was mixed with 12 g of distilled water and 120 g of tetrahydrofuran with stirring for 6 hours. The mixture was then filtered and the solid phase was dried to obtain 3.5 g of treated Product 1-B.

### Example 12

### [Treatment of Product 1-B]

The wet cake (15 g) obtained in Example 10 was mixed with 12 g of distilled water and 120 g of toluene at room temperature with stirring for 6 hours. The mixture was then filtered and the solid phase was dried to obtain 3.6 g of treated Product 1-B.

### Example 13

### [Treatment of Product 1-B]

The wet cake (15 g) obtained in Example 10 was mixed with 12 g of distilled water and 120 g of 1,4-dioxane at room temperature with stirring for 6 hours. The mixture was then filtered and the solid phase was dried to obtain 3.6 g of treated Product 1-B.

### Example 14

### [Treatment of Product 1-B]

The wet cake (15 g) obtained in Example 10 was mixed with 12 g of distilled water and 120 g of N,N-dimethylformamide at 80°C with stirring for 6 hours. The mixture was then filtered and the solid phase was dried to obtain 3.6 g of treated Product 1-B.

### Example 15

### [Preparation of Product 1-B]

A mixture containing 1.95 g (4.0 mols) of the nitrile compound (6) obtained in Example 1, 105.64 g (0.808 mol) of phthalonitrile, 74.87 g (0.22 mols) of Ti(OBu)₄, 24.26 g (0.400 mol) and 100 ml of 1-octanol was gradually heated to 155°C and maintained at 150-155°C with stirring under a nitrogen gas stream for 5 hours. After completion of the reaction, the reaction mixture was allowed to be cooled to 70°C and mixed with 400 ml of methanol. The mixture was again heated at 70°C with stirring for 45 minutes. After being allowed to stand overnight, the solid phase was then filtered, washed thrice with toluene, twice with methanol and once with water and dried to obtain treated Product 1-B.

### Example 16

The product (40.0 g) obtained in Example 15 was added little by little to 640 g of concentrated sulfuric acid through 30 minutes while maintaining the temperature at 4-9°C. The mixture was then heated at 6-9°C for 1 hour with stirring and then added dropwise in 4 liters of ice water through 30 minutes. After stirring for 30 minutes, solids were separated by filtration. The separated solid phase was mixed with 4 liters of ion-exchanged water with stirring and again filtered. Such washing and filtration procedures were repeated 4 times more to obtain 297.7 g of a wet cake (solid matter content: 12.7 %, yield: 94.5 %).

### Example 17

The wet cake (153.86 g) obtained in Example 16 was dried in a vacuum dryer at 70°C for 2 days to obtain 19.52 g of dried product.

### Example 18

The product obtained in Example 15 was subjected to an acid-paste treatment in the same manner as described in Example 16 to obtain a wet cake. The wet cake (8.00 g, solid matter content: 13.1 %) was mixed with 2.5 g of ion-exchanged water and 42.0 g of 1-methyl-2-pyrrolidinone with stirring at room temperature for 6 hours. This was then mixed with methanol and the solid phase was separated by filtration to give 1.03 g of a treated product (yield: 98.1 %).

### Example 19

The wet cake (8.00 g) obtained in Example 16 was mixed with 2.5 g of ion-exchanged water and 42.0 g of 1-methyl-2-pyrrolidinone with stirring at room temperature for 2 hours. This was then mixed with methanol and the solid phase was separated by filtration to give 1.01 g of a treated product (yield: 99.6 %).

### Example 20

Example 19 was repeated in the same manner as described except that the stirring time was increased to 48 hours. 1.01 Grams of a treated product was obtained (yield: 99.6 %).

### Example 21

Example 18 was repeated in the same manner as described except that 1,3-dimethyl-2-imidazoline was substituted for 1-methyl-2-pyrrolidinone. 1.04 Grams of a treated product was obtained (yield: 99.0 %).

### Example 22

Example 18 was repeated in the same manner as described except that the stirring time was increased to 30 hours. 1.01 Grams of a treated product was obtained (yield: 99.6 %).

### Example 23

Example 18 was repeated in the same manner as described except that the treatment temperature was increased to 30 hours. 1.01 Grams of a treated product was raised to 80°C. 1.01 Grams of a treated product was obtained (yield: 99.6 %).

### Example 24

In a mixed solvent containing 2 ml of trifluoroacetic acid and 8 ml of dichloromethane, 1 g of Product 1-B obtained in Example 3 was dissolved. The mixture was then stirred for 1 hour and subsequently added dropwise through 5 minutes to a mixed solvent composed of 100 ml of dichloromethane and 100 ml of methanol in an ice bath with stirring to form crystals. The crystals were separated by filtration and washed with distilled water until no acid was detected in the washed water and dried, thereby obtaining a treated product.

### Example 25

In a mixed solvent containing 2 ml of trifluoroacetic acid and 8 ml of dichloromethane, 1 g of the acid-treated product obtained in Example 3 was dissolved. The mixture was then stirred for 1 hour and subsequently added dropwise through 75 minutes to a mixed solvent composed of 100 ml of dichloromethane and 100 ml of methanol in an ice bath with stirring to form crystals. The crystals were separated by filtration and washed with distilled water until no acid was detected in the washed water and dried, thereby obtaining a treated product.

### Example 26

In a mixed solvent containing 2 ml of trifluoroacetic acid and 8 ml of dichloromethane, 1 g of The acid-treated product obtained in Example 3 was dissolved. The mixture was then stirred for 1 hour and subsequently added dropwise through 5 minutes to 200 ml of ice water to form crystals. The crystals were separated by filtration and washed with distilled water until no acid was detected in the washed water and dried, thereby obtaining a treated product.

### Example 27

In a mixed solvent containing 2 ml of trifluoroacetic acid and 8 ml of dichloromethane, 1 g of The acid-treated product obtained in Example 3 was dissolved. The mixture was then stirred for 1 hour and subsequently added dropwise through 5 minutes to 50 ml of N,N-dimethylformamide in an ice bath with stirring. Thereafter, 50 ml of methanol was added to the mixture and stirred for 1 hour to form crystals. The crystals were separated by filtration and washed with distilled water until no acid was detected in the washed water and dried, thereby obtaining a treated product.

### Example 28

In a mixed solvent containing 2 ml of trifluoroacetic acid and 8 ml of dichloromethane, 1 g of The acid-treated product obtained in Example 3 was dissolved. The mixture was then stirred for 1 hour and subsequently added dropwise through 5 minutes to 50 ml of cylohexane in an ice bath with stirring. Thereafter, 50 ml of methanol was added to the mixture and stirred for 1 hour to form crystals. The crystals were separated by filtration and washed with distilled water until no acid was detected in the washed water and dried, thereby obtaining a treated product.

### Example 29

In a mixed solvent containing 2 ml of trifluoroacetic acid and 8 ml of dichloromethane, 1 g of The acid-treated product obtained in Example 3 was dissolved. The mixture was then stirred for 1 hour and subsequently added dropwise through 5 minutes to 50 ml of isopropyl ether in an ice bath with stirring. Thereafter, 50 ml of methanol was added to the mixture and stirred for 1 hour to form crystals. The crystals were separated by filtration and washed with distilled water until no acid was detected in the washed water and dried, thereby obtaining a treated product.

### Example 30

In a mixed solvent containing 2 ml of trifluoroacetic acid and 8 ml of dichloromethane, 1 g of The acid-treated product obtained in Example 3 was dissolved. The mixture was then stirred for 1 hour and subsequently added dropwise through 5 minutes to 50 ml of methyl ethyl ketone in an ice bath with stirring. Thereafter, 50 ml of methanol was added to the mixture and stirred for 1 hour to form crystals. The crystals were separated by filtration and washed with distilled water until no acid was detected in the washed water and dried, thereby obtaining a treated product.

### Example 31

In a mixed solvent containing 2 ml of trifluoroacetic acid and 8 ml of dichloromethane, 1 g of The acid-treated product obtained in Example 3 was dissolved. The mixture was then stirred for 1 hour and subsequently added dropwise through 5 minutes to 50 ml of methanol with stirring in an ice bath to form crystals. The crystals were separated by filtration and washed with distilled water until no acid was detected in the washed water and dried, thereby obtaining a treated product.

### Example 32

In a mixed solvent containing 2 ml of trifluoroacetic acid and 8 ml of dichloromethane, 1 g of The acid-treated product obtained in Example 3 was dissolved. The mixture was then stirred for 1 hour and subsequently added dropwise through 5 minutes to 50 ml of tetrahydrofuran in an ice bath with stirring. Thereafter, 50 ml of methanol was added to the mixture and stirred for 1 hour to form crystals. The crystals were separated by filtration and washed with distilled water until no acid was detected in the washed water and dried, thereby obtaining a treated product.

### Example 33

In a mixed solvent containing 2 ml of trifluoroacetic acid and 8 ml of dichloromethane, 1 g of The acid-treated product obtained in Example 3 was added through 1 minute. The mixture was then stirred for 45 minutes at room temperature to dissolve the Product and then allowed to sand for 25 minutes. After removal of the supernatant, the mixture was mixed with 50 ml of methanol to form crystals. The crystals were separated by filtration, placed in a beaker, washed with hot water, and then filtered. Such washing with hot water and filtration procedures were repeated two times more. Thereafter, the crystals were dispersed in 50 ml of fluorobenzene, stirred for 15 minutes at room temperature, separated by filtration and dried to obtain a treated product.

### Example 34

In a mixed solvent containing 2 ml of trifluoroacetic acid and 8 ml of dichloromethane, 1 g of The acid-treated product obtained in Example 3 was dissolved. The mixture was then stirred for 1 hour and subsequently added dropwise through 20 seconds to a mixed solvent consisting of 100 ml of dichloromethane and 100 ml methanol with stirring in an ice bath to form crystals. The crystals were separated by filtration and washed with distilled water until no acid was detected in the washed water and dried, thereby obtaining a treated product.

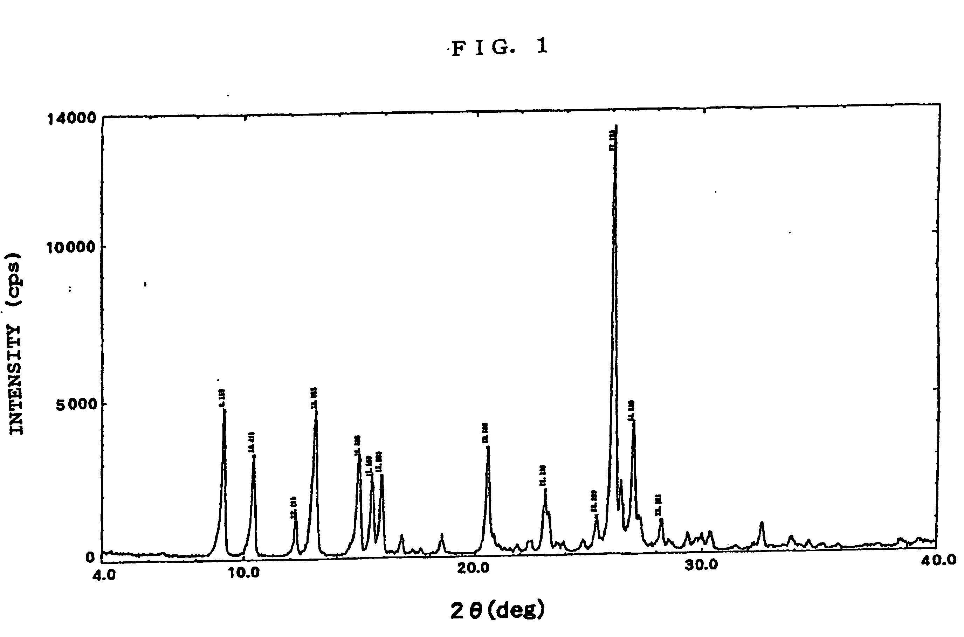

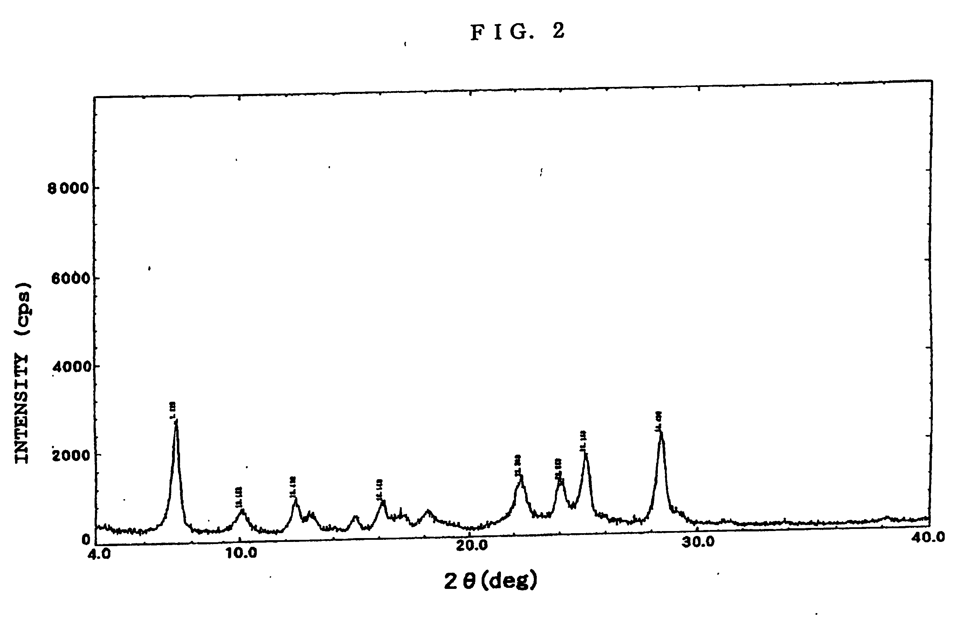

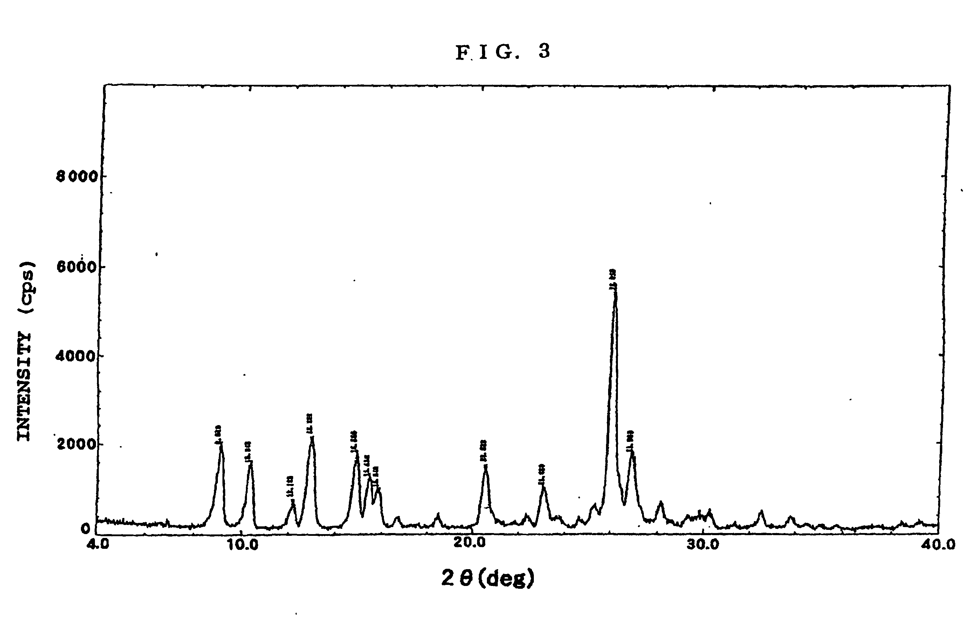

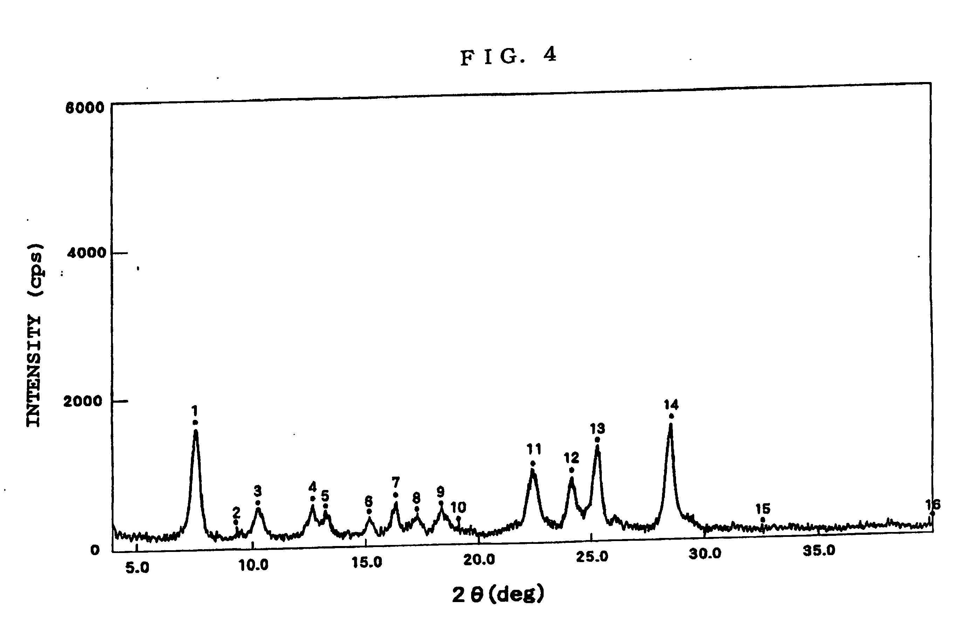

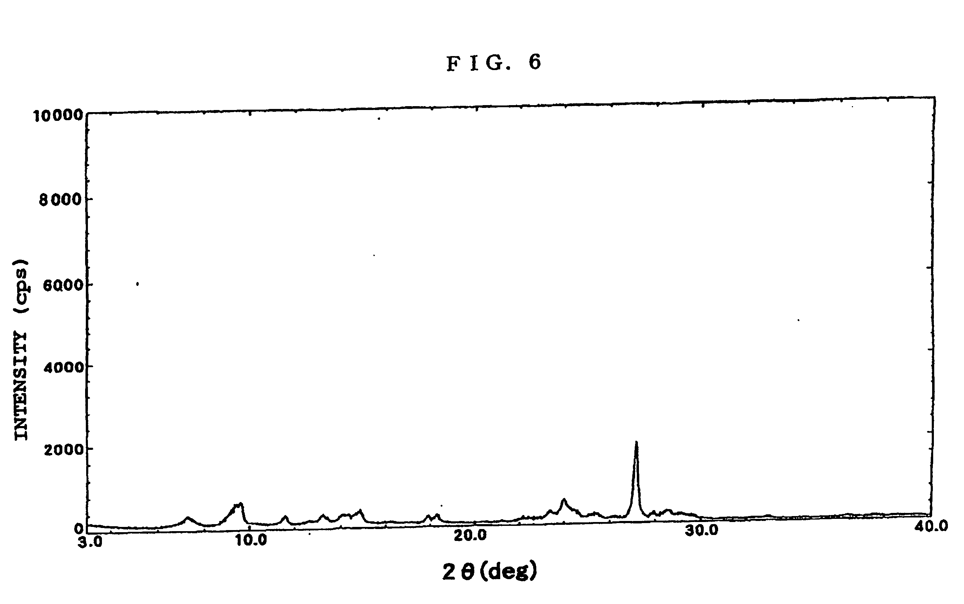

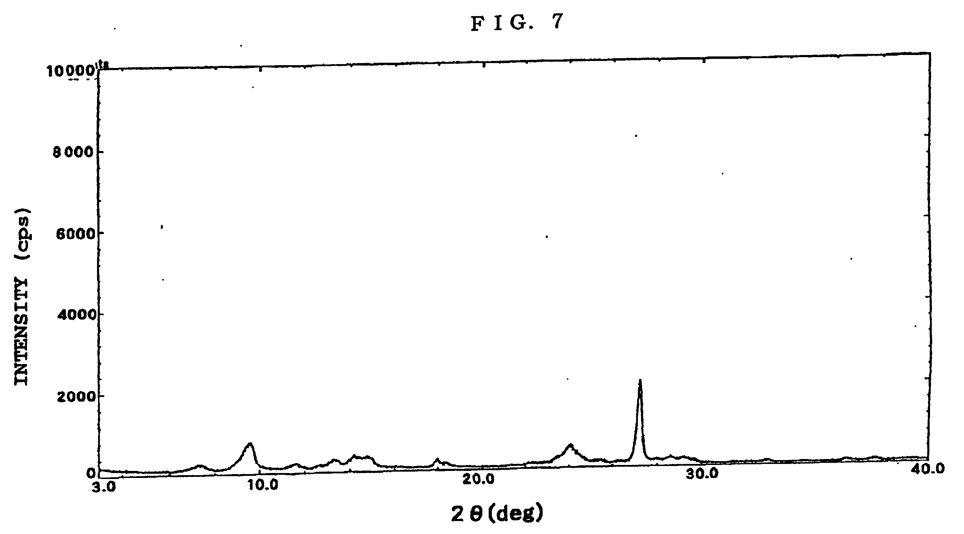

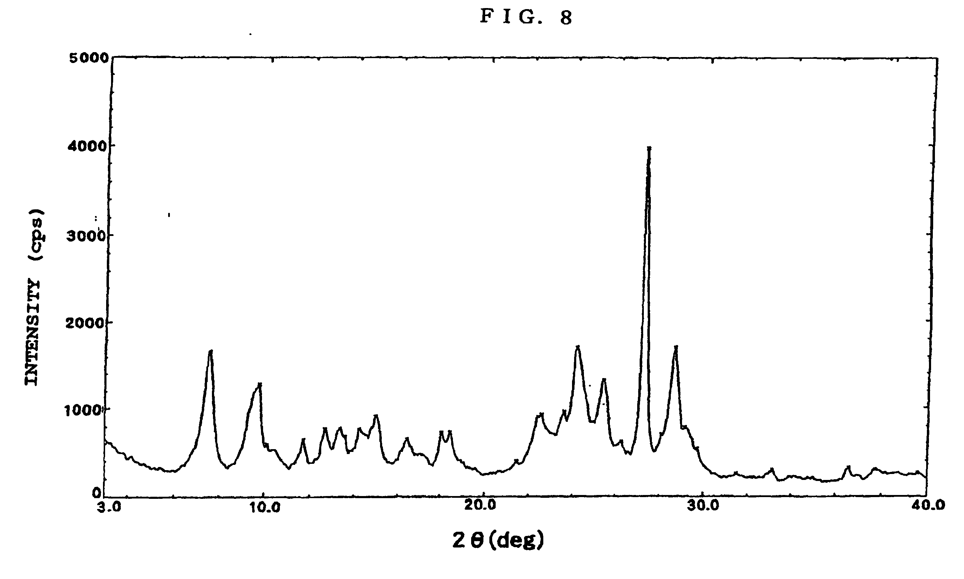

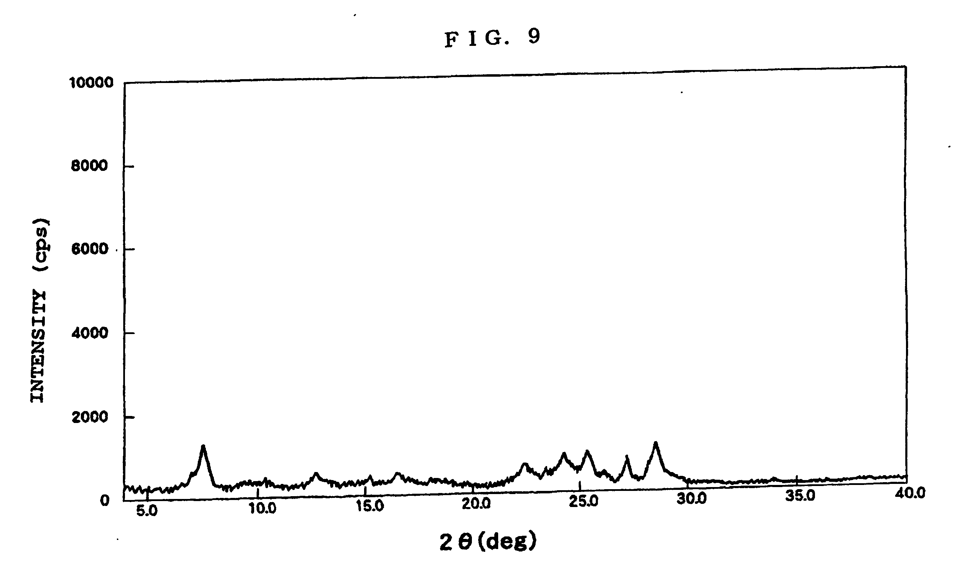

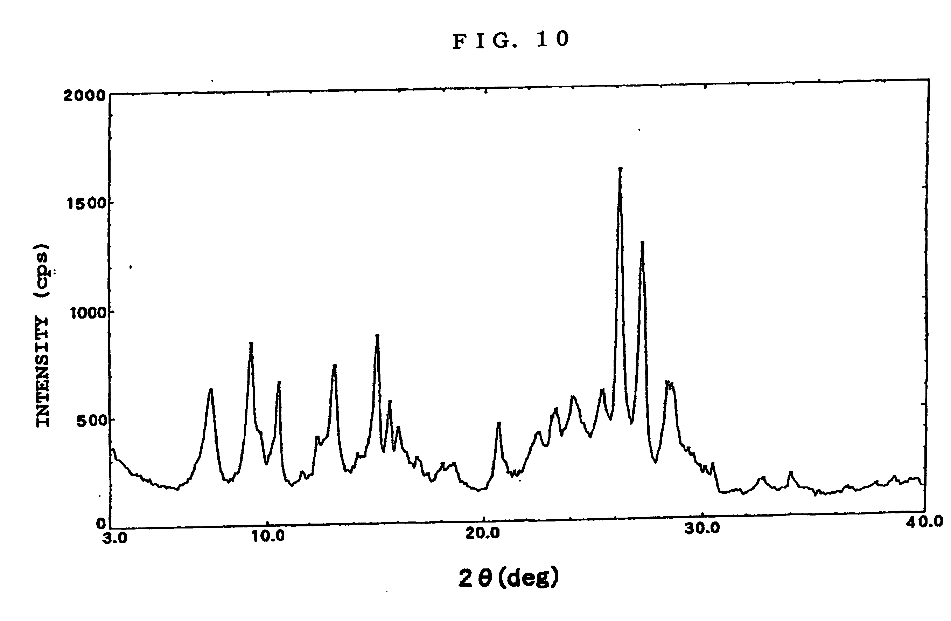

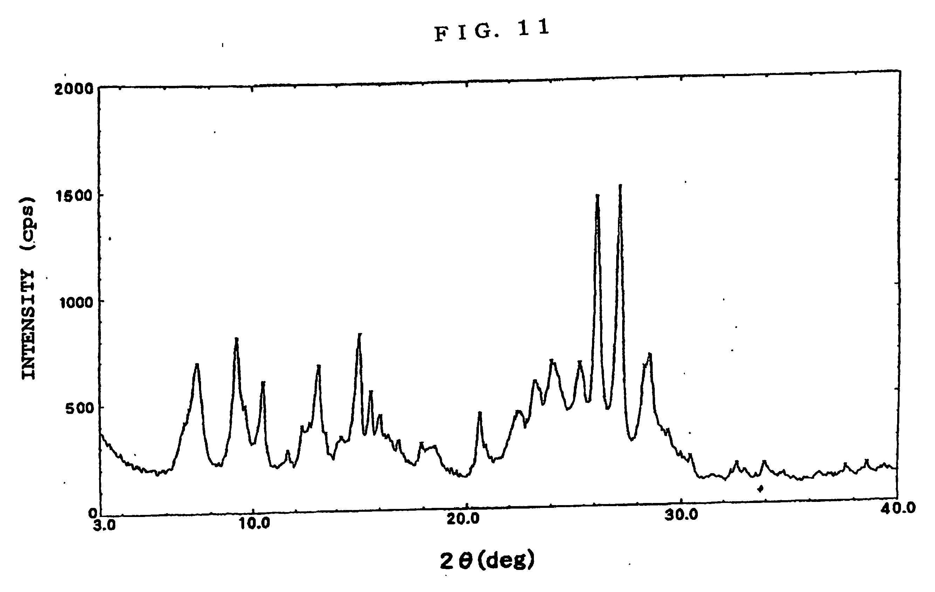

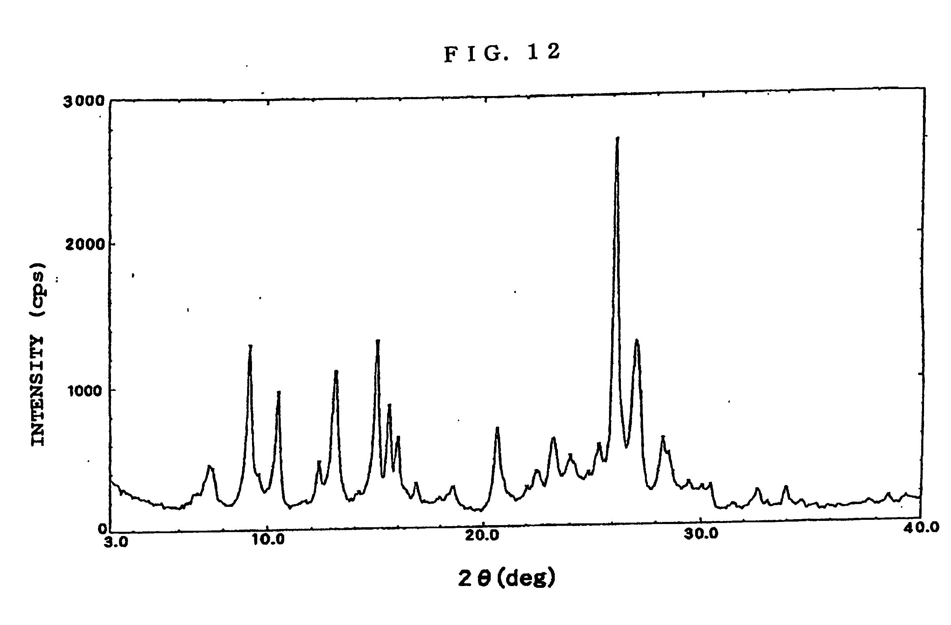

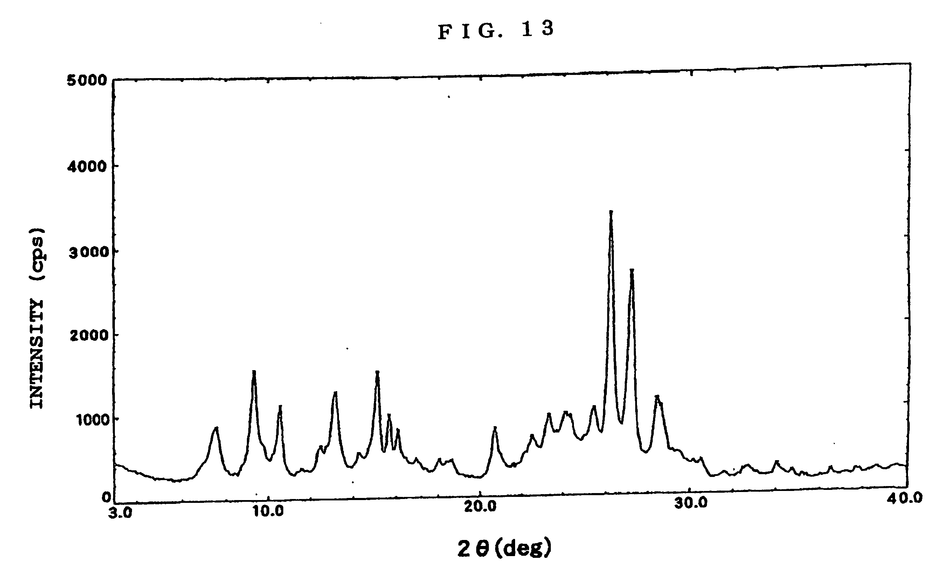

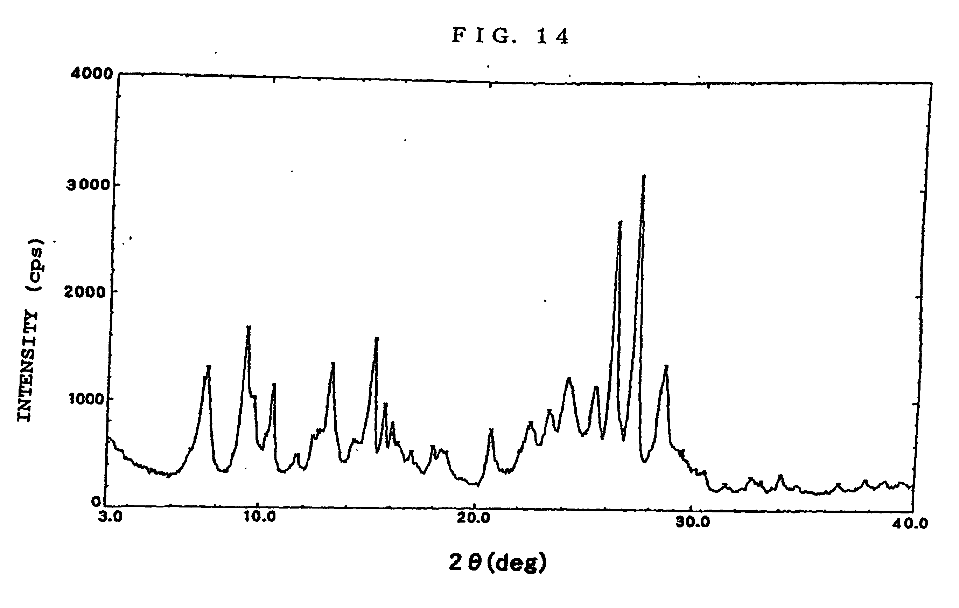

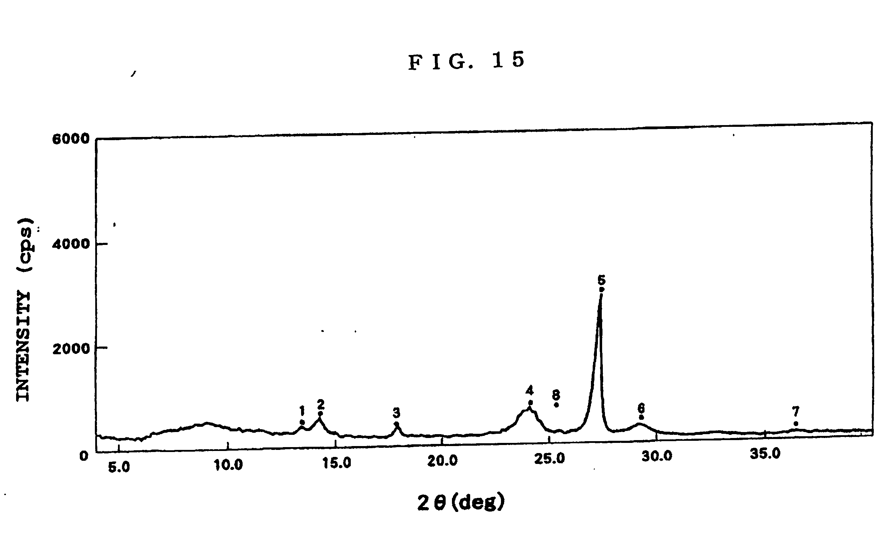

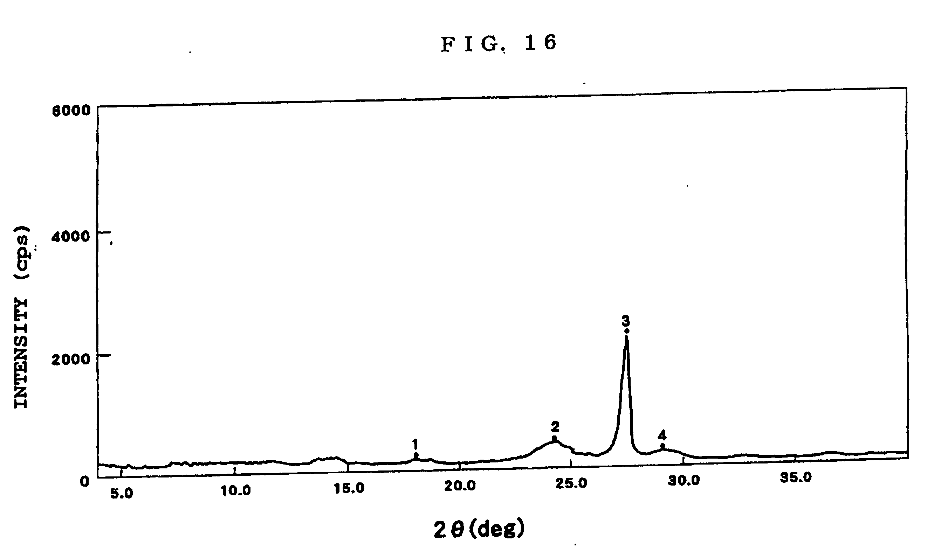

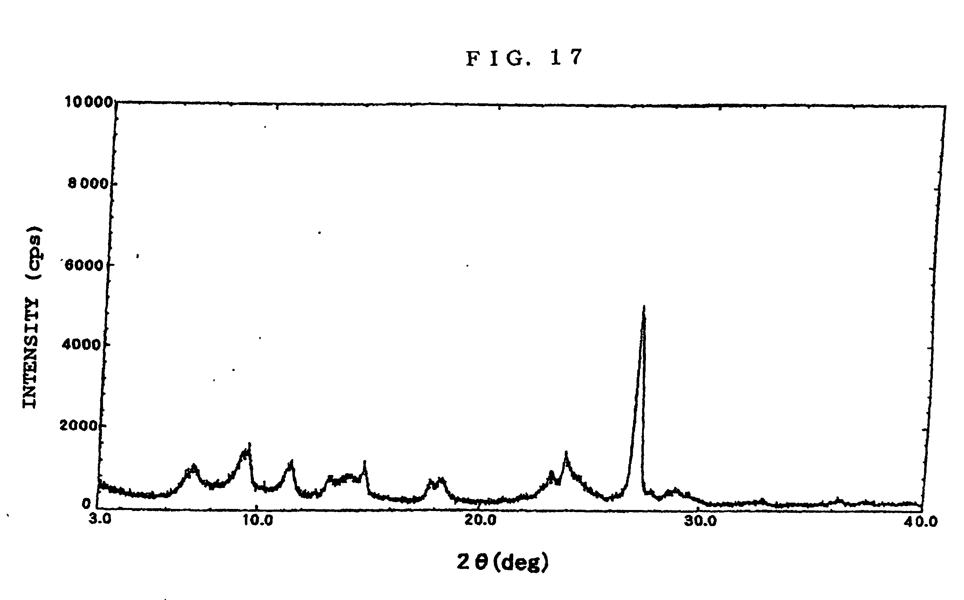

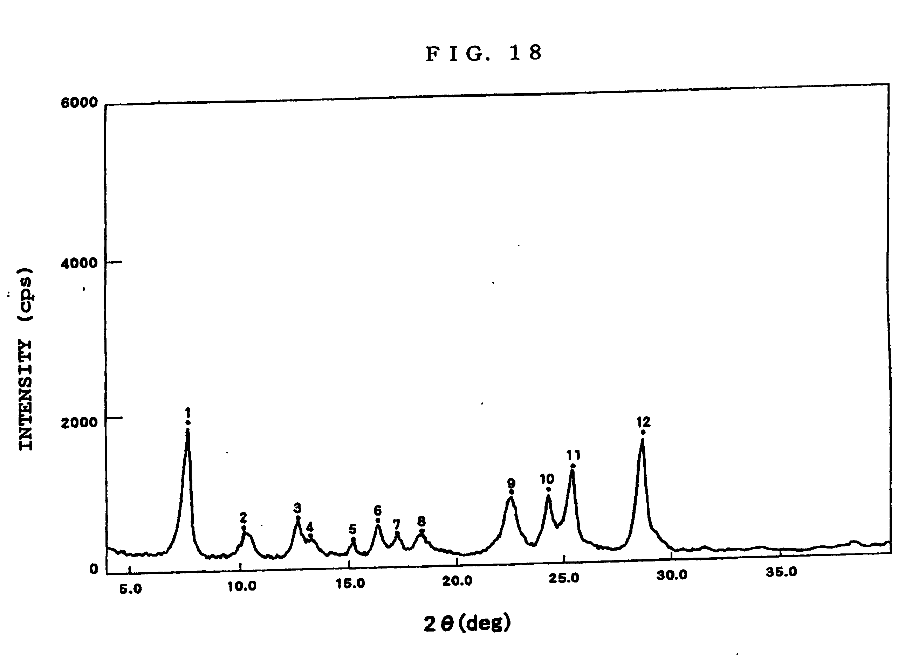

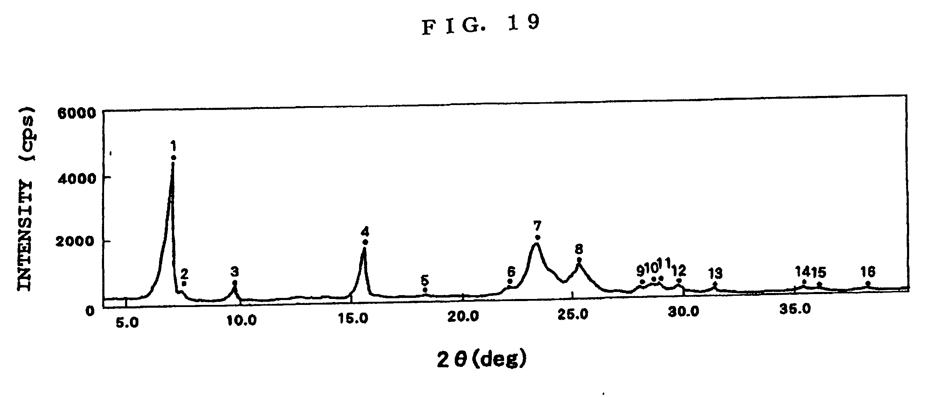

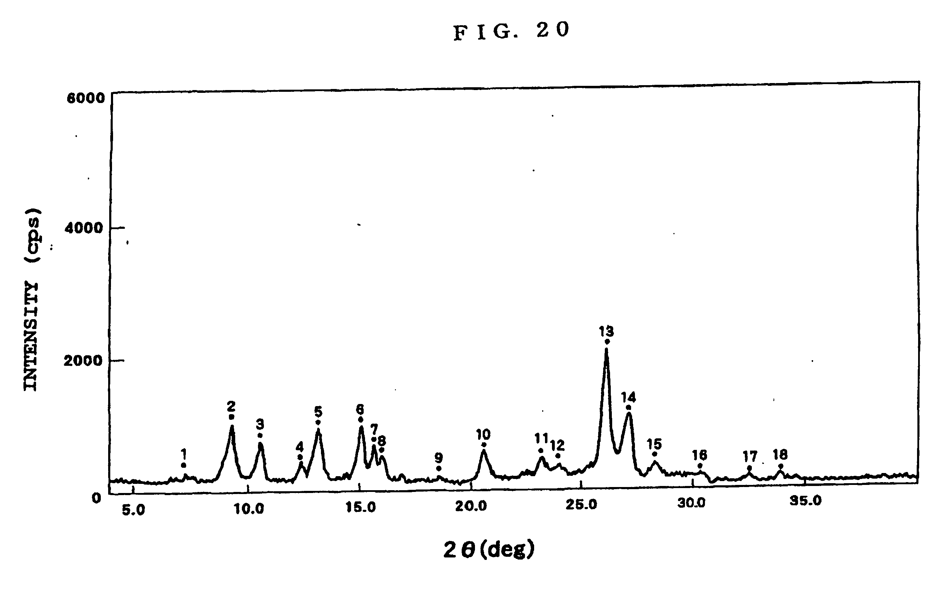

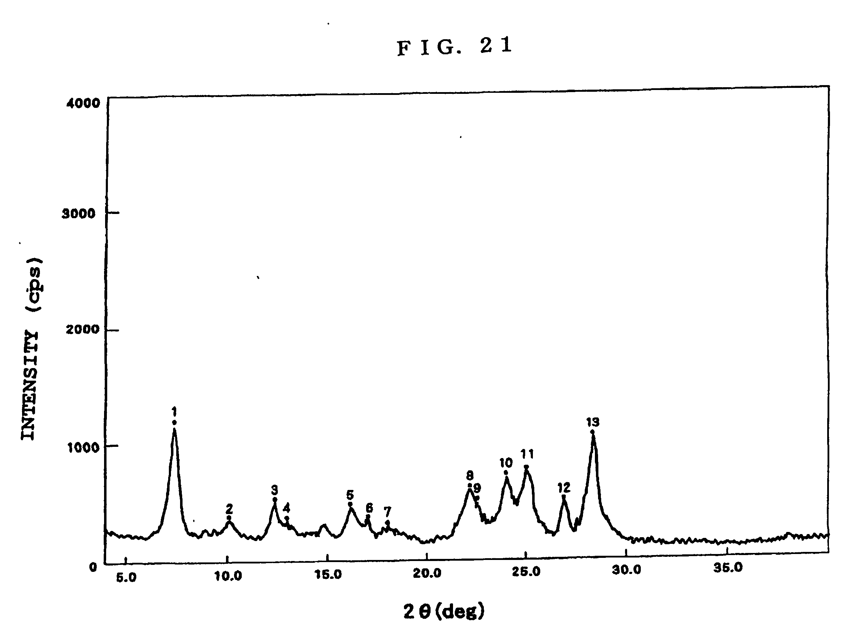

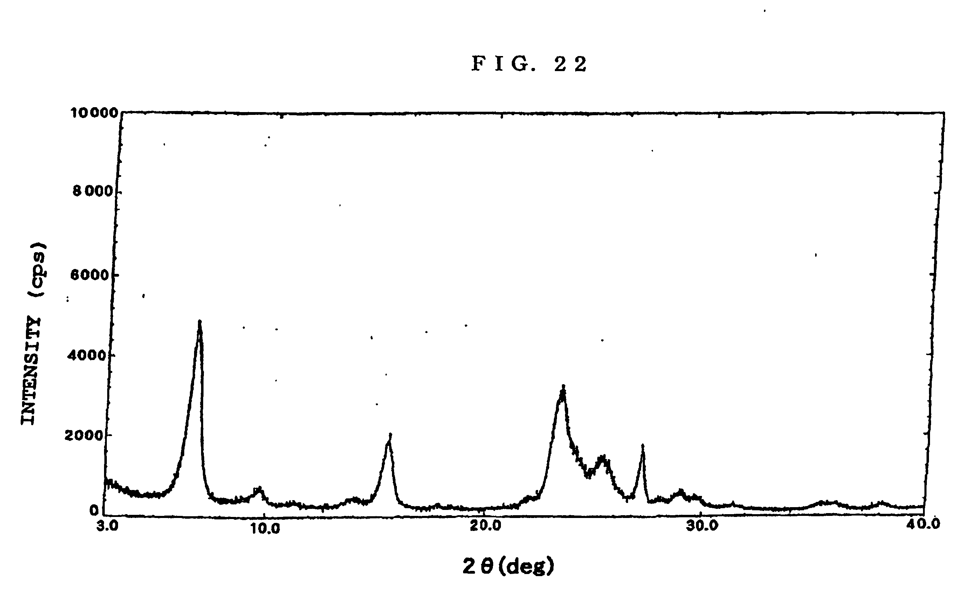

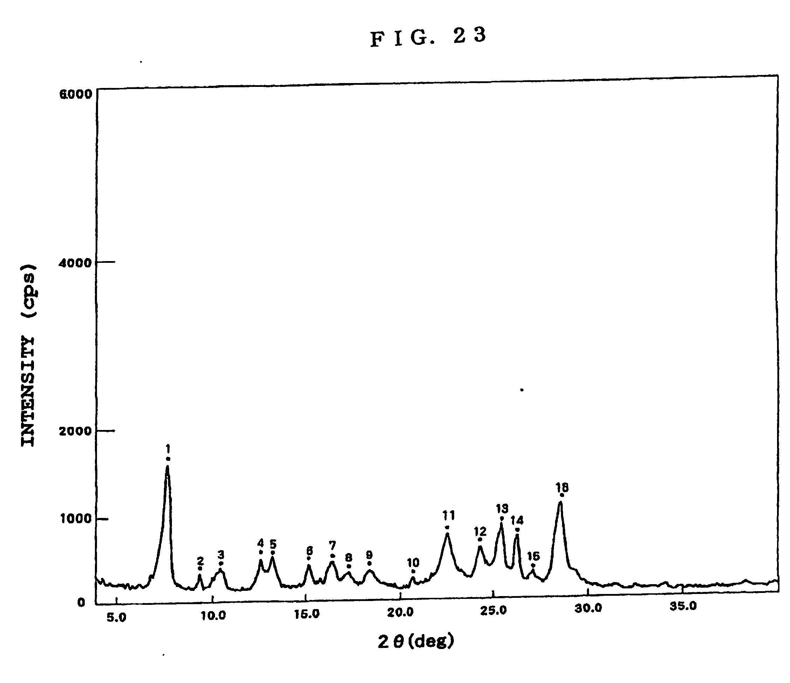

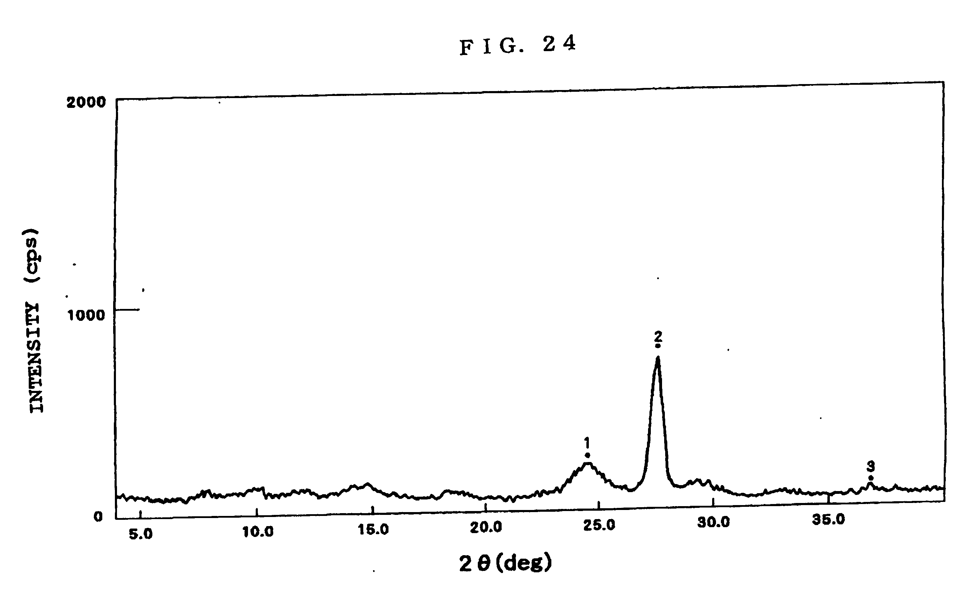

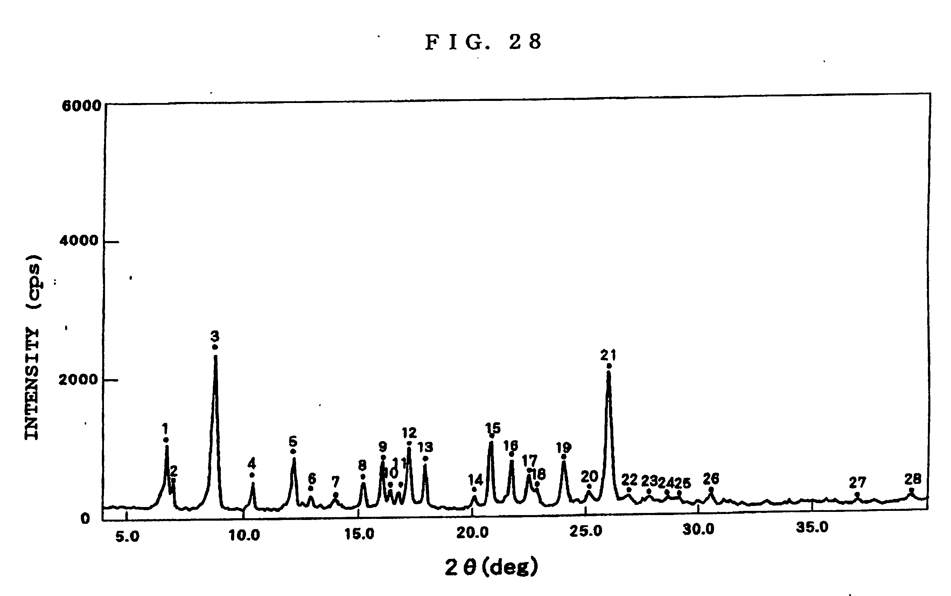

Each of the products or treated products obtained in Examples 6-14, 18-22, 24-34 was measured for X-ray diffraction spectrum thereof to give the results shown in Figs. 1-24 and 28, respectively. The X-ray diffraction spectra were measured under conditions shown below. Wet cake samples were measured after having been dried.

| | |
|---|---|
| X-ray tube: | Cu (wavlength: 1. 54 Å) |
| Voltage: | 50 kV |
| Current: | 30 mA |
| Scanning speed: | 2 deg/min. |
| Scanning scope: | 3-40 deg |

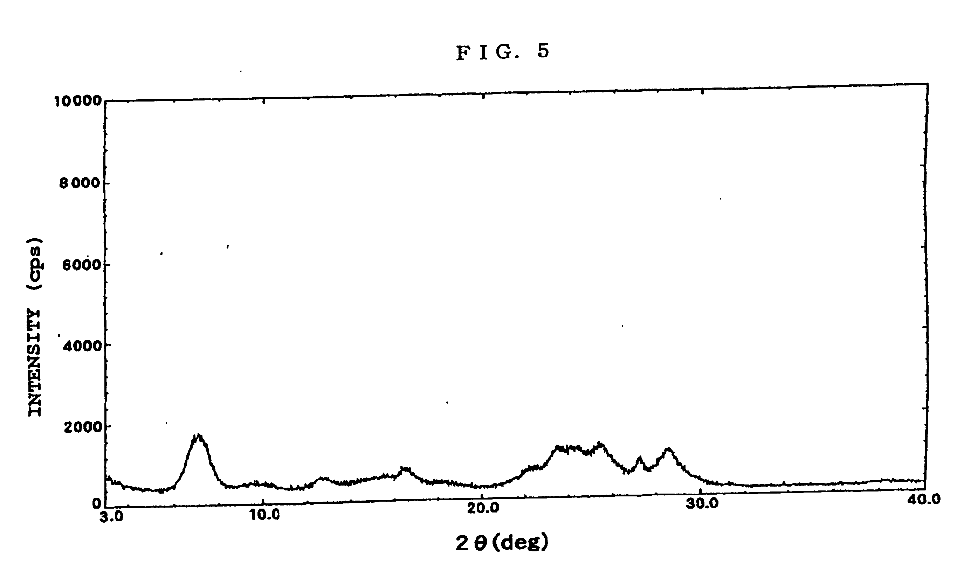

The X-ray diffraction spectrum shown in Fig. 5 has no diffraction peaks having a half-value width of 1° or less, indicating that the product is amorphous. The other X-ray diffraction spectra have peaks with a half-value width of 1° or less, indicating that the products are crystalline in nature. Further, differences in the X-ray patterns suggest that the crystal structures of the products are different from each other.

### Fabrication of Photoconductors

### Example 35

A dispersion containing 3 parts by weight of the product obtained in Example 24, 1 part by weight of a commercially available polyvinyl butyral resin (BM-S manufactured by Sekisui Chemical Co., Ltd.) and 80 parts by weight of methyl ethyl ketone was placed in a ball mill pot and milled for 3 hours using PSZ balls with a diameter of 2 mm to obtain a coating liquid for forming a charge generation layer. This was applied on an aluminum plate and dried at 100°C for 20 minutes to form a charge generation layer having a thickness of about 0.3 µm.

A coating liquid for forming a charge transport layer was prepared by mixing 7 parts by weight of a positive hole charge transporting material of the formula (33) shown below, 10 parts by weight of a commercially available polycarbonate resin (PCX-5 manufactured by Teijin Chemicals Ltd.), 68 parts by weight of dichloromethane and 0.0002 part by weight of a commercially available silicone oil (KF50 manufactured by Shin-Etsu Chemical Co., Ltd.). 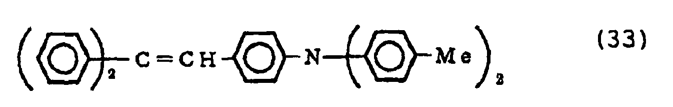
The coating liquid was applied on the above-prepared charge generation layer and dried at 110°C for 20 minutes to obtain a charge transport layer having a thickness of about 28 *µ*m, thereby obtaining an electrophotographic photoconductor of Example 35.

### Example 36

Example 35 was repeated in the same manner as described except that the product obtained in Example 25 was substituted for the product of Example 24, thereby obtaining an electrophotographic photoconductor of Example 36.

### Example 37

Example 35 was repeated in the same manner as described except that the product obtained in Example 26 was substituted for the product of Example 24, thereby obtaining an electrophotographic photoconductor of Example 37.

### Example 38

Example 35 was repeated in the same manner as described except that the product obtained in Example 27 was substituted for the product of Example 24, thereby obtaining an electrophotographic photoconductor of Example 38.

### Example 39

Example 35 was repeated in the same manner as described except that the product obtained in Example 28 was substituted for the product of Example 24, thereby obtaining an electrophotographic photoconductor of Example 39.

### Example 40

Example 35 was repeated in the same manner as described except that the product obtained in Example 29 was substituted for the product of Example 24, thereby obtaining an electrophotographic photoconductor of Example 40.

### Example 41

Example 35 was repeated in the same manner as described except that the product obtained in Example 30 was substituted for the product of Example 24, thereby obtaining an electrophotographic photoconductor of Example 41.

### Example 42

Example 35 was repeated in the same manner as described except that the product obtained in Example 31 was substituted for the product of Example 24, thereby obtaining an electrophotographic photoconductor of Example 42.

### Example 43

Example 35 was repeated in the same manner as described except that the product obtained in Example 32 was substituted for the product of Example 24, thereby obtaining an electrophotographic photoconductor of Example 43.

### Example 44

Example 35 was repeated in the same manner as described except that the product obtained in Example 33 was substituted for the product of Example 24, thereby obtaining an electrophotographic photoconductor of Example 44.

### Example 45

Example 35 was repeated in the same manner as described except that a positive hole charge transporting material represented by the formula (34) below was substituted for the charge transporting material (33), thereby obtaining an electrophotographic photoconductor of Example 45.

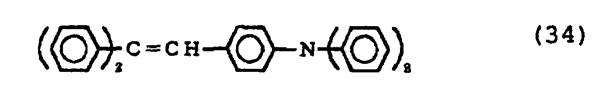

### Example 46

Example 35 was repeated in the same manner as described except that a positive hole charge transporting material represented by the formula (35) below was substituted for the charge transporting material (33), thereby obtaining an electrophotographic photoconductor of Example 46.

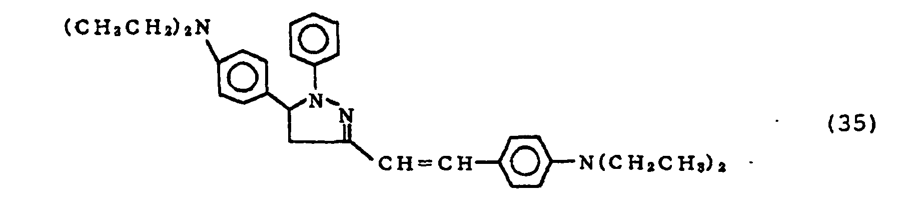

### Example 47

Example 35 was repeated in the same manner as described except that a positive hole charge transporting material represented by the formula (36) below was substituted for the charge transporting material (33), thereby obtaining an electrophotographic photoconductor of Example 47.

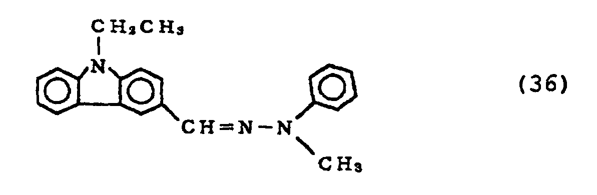

### Example 48

Example 35 was repeated in the same manner as described except that the product obtained in Example 6 was substituted for the product of Example 24, thereby obtaining an electrophotographic photoconductor of Example 48.

### Example 49

Example 35 was repeated in the same manner as described except that the product obtained in Example 7 was substituted for the product of Example 24, thereby obtaining an electrophotographic photoconductor of Example 49.

### Example 50

Example 35 was repeated in the same manner as described except that the product obtained in Example 12 was substituted for the product of Example 24, thereby obtaining an electrophotographic photoconductor of Example 50.

### Comparative Example

A mixture containing 0.1 mole of phthalonitrile, 0.025 mol of Ti(OBu)₄, 0.05 mol of urea and 200 ml of octanol was gradually heated to 150°C and maintained at 150-160°C with stirring under a nitrogen gas stream for 6 hours. After completion of the reaction, the reaction mixture was allowed to be cooled and filtered. The solid phase was then washed well with octanol and then several times with methanol, toluene and water and dried to obtain titanylphthalocyanine with a yield of 70 %.

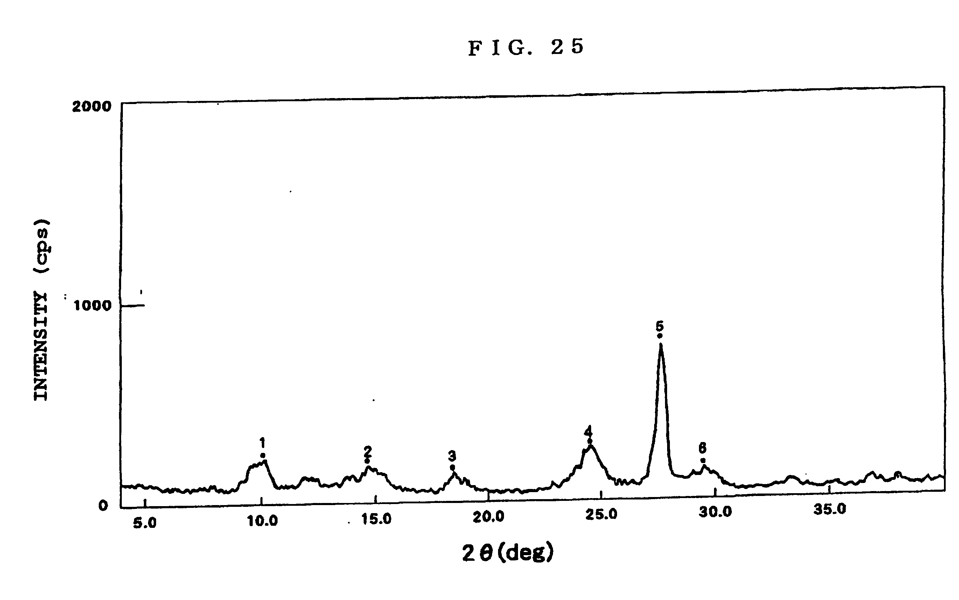

In a mixed solvent containing 2 ml of trifluoroacetic acid and 8 ml of dichloromethane, 1 g of the thus obtained titanylphthalocyanine was added through 1 minute. The mixture was then stirred for 45 minutes at room temperature to dissolve the titanylphthalocyaine and then allowed to sand for 25 minutes. After removal of the supernatant, the mixture was mixed with 50 ml of methanol to form crystals. The crystals were separated by filtration, placed in a beaker, washed with 200 ml of hot water, and then filtered. Such washing with hot water and filtration procedures were repeated two times more. Thereafter, the crystals were dispersed in 50 ml of fluorobenzene, stirred for 15 minutes at room temperature, separated by filtration and dried to obtain treated titanylphthalocyanine whose X-ray diffraction spectrum measured in the same conditions as described above is shown in Fig. 25.

Example 35 was repeated in the same manner as described except that the above treated titanylphthalocyanine was substituted for the product of Example 24, thereby obtaining an electrophotographic photoconductor of Comparative Example.

The photoconductors obtained in Examples 35-50 and Comparative Example were measured for their electrostatic characteristics under conditions'involving a temperature of 25°C and a relative humidity of 55 % using a commercially available electrostatic copying sheet testing apparatus "Paper Analyzer Model EPA-8100" manufactured by Kawaguchi Electro Works Co., Ltd. in accordance with a dynamic mode. Thus, each photoconductor was negatively charged at -6 kV for 20 seconds. Then the photoconductor was allowed to stand in the dark for 20 seconds and the surface potential V₀ [-V] was measured. When the surface potential reached -800V, the photoconductor was irradiated with a monochromatic light with a wavelength of 780 nm such that the illuminance on the surface of the photoconductor was 1 µW/cm². The exposure Em1/2 [µJ/cm²] required to reduce the surface potential from -800V to -400 V was measured for the evaluation of sensitivity of the photoconductor to light emitted from LD (near infrared region). The results are summarized in Table 13 below.

**Table 13**

| Example | V₀ [-V] | Em1/2 [µJ/cm²] |
|---|---|---|
| 35 | 870 | 0.35 |
| 36 | 1050 | 0.2 |
| 37 | 950 | 0.4 |
| 38 | 850 | 0.2 |
| 39 | 700 | 0.95 |
| 40 | 720 | 0.3 |
| 41 | 680 | 0.5 |
| 42 | 900 | 0.7 |
| 43 | 1100 | 0.4 |
| 44 | 1160 | 0.4 |
| 45 | 600 | 0.8 |
| 46 | 650 | 1.6 |
| 47 | 800 | 1.2 |
| 48 | 900 | 0.5 |
| 49 | 950 | 0.6 |
| 50 | 850 | 0.4 |
| Comparative Example | 820 | 0.38 |

The photoconductors obtained in Example 35 and Comparative Example were each measured for the electrostatic fatigue characteristics thereof using a commercially available electrostatic copying sheet testing apparatus "Paper Analyzer Model EPA-8100" manufactured by Kawaguchi Electro Works Co., Ltd. in accordance with a dynamic mode (rotational speed: 1000 rpm). Thus, each photoconductor was repeatedly subjected to negative charging at -6 kV and irradiation with white light for 60 minutes while maintaining the current and potential at 5.6 µA and -800V, respectively. The surface potential V₀ of the photoconductor after the fatigue test was found to be -820 V in the case of Example 35 and -450 V in the case of Comparative Example. Thus, the use of the product according to the present invention is effective in providing photoconductors having a high resistance to electrostatic fatigue.

### Example 51

The same coating liquid for forming a charge generation layer as used in Example 35 was applied onto an aluminum-deposited PET base to form a charge generation layer. A coating liquid, which was a solution obtained by dissolving 8 parts by weight of an electron transporting material of the formula (28) shown below, 11 parts by weight of a polycarbonate resin (Polycarbonate Z manufactured by Teijin Chemicals, Ltd.) and 0.02 part by weight of a silicone oil (KF50 manufactured by Shin-Etsu Chemical Co., Ltd.) in 91 parts by weight of tetrahydrofuran, was then applied to the above charge generation layer with a doctor blade and dried to form thereon a charge transport layer having a thickness of 20 µm, thereby obtaining a photoconductor of Example 51.

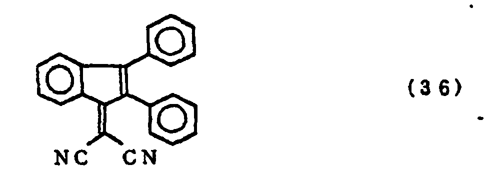

The photoconductor was measured for its electrostatic characteristics in the same manner as that in Example 35 except that the impressed voltage was +6 kv. It was found that V₀ was +1100 V and Em1/2 was 2.50 µJ/cm².

### Example 52

Example 51 was repeated in the same manner as described except that an electron transporting material of the formula (29) shown below was used in lieu of the compound of the formula (28), thereby obtaining a photoconductor of Example 52.

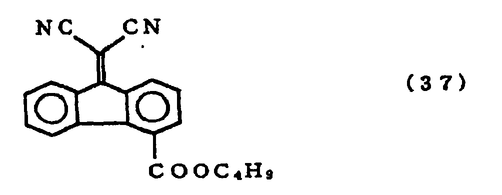

The photoconductor was measured for its electrostatic characteristics in the same manner as that in Example 51. It was found that V₀ was +1200 V and Em1/2 was 2.35 µJ/cm².

### Example 53

The product obtained in Example 24 (0.5 g) was milled with a ball mill together with 9 g of tetrahydrofuran and 10 g of a 10 % by weight solution of a polycarbonate resin (Polycarbonate Z manufactured by Teijin Chemicals, Ltd.) in tetrahydrofuran to obtain a dispersion. Quantities of the dispersion, the charge transporting material of the formula (33) and the 10 % by weight solution of Polycarbonate Z were then blended with sufficient stirring to obtain a coating liquid having the following composition:

| | |
|---|---|
| Product obtained in Example 24: | 2 % by weight |
| Charge transporting material: | 28 % by weight |
| Polycarbonate Z: | 50 % by weight |
| Tetrahydrofuran: | 20 % by weight |

The coating liquid was then applied onto a Al-deposited polyester film with a doctor blade and dried to form a single layer photoconductive layer having a thickness of 15 µm, thereby obtaining a single layer-type photoconductor of Example 53. The photoconductor was measured for its electrostatic characteristics in the same manner as that in Example 35. It was found that V₀ was -1150 V and Em1/2 was 0.6 µJ/cm².

### Example 54

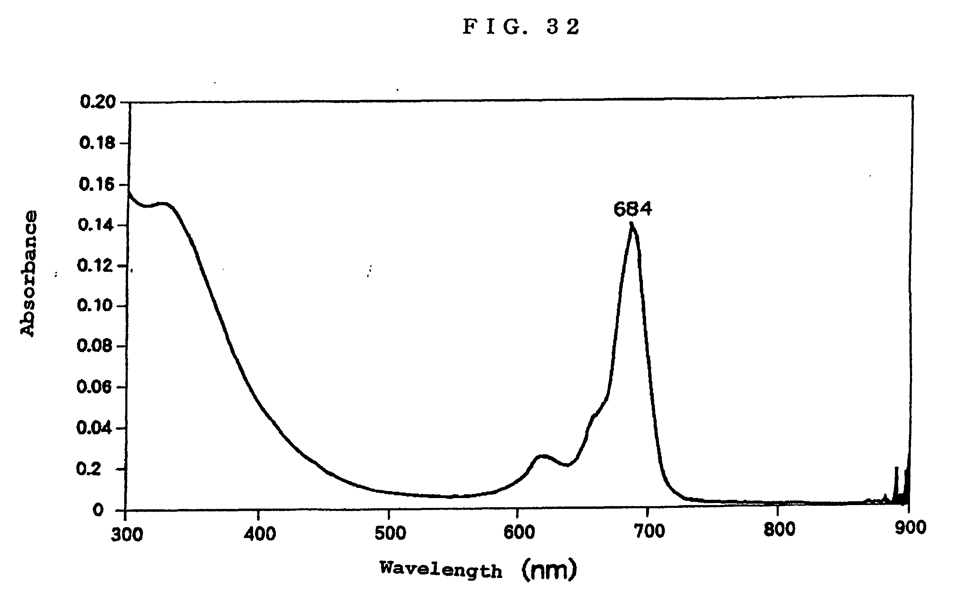

The product obtained in Example 24 was dissolved in N,N-dimethylformamide and the solution was measured for UV-visible light absorption spectrum. The results are shown in Fig. 32. The spectrum shows that λmax is 684 nm.

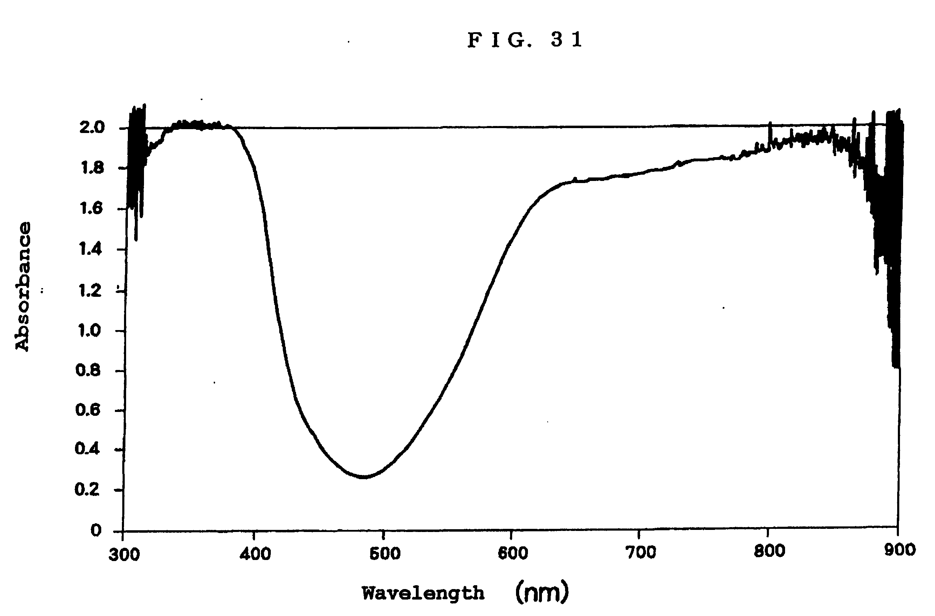

A dispersion containing 3 parts by weight of the product obtained in Example 24, 1 part by weight of a commercially available polyvinyl butyral resin (BM-S manufactured by Sekisui Chemical Co., Ltd.) and 80 parts by weight of methyl ethyl ketone was placed in a ball mill pot and milled for 3 hours using PSZ balls with a diameter of 2 mm to obtain a coating liquid for forming a charge generation layer. This was applied to a polyethylene terephthalate sheet with a thickness of 100 µm and then dried at 100°C for 20 minutes to obtain a sample for measuring a light absorption spectrum. The sample was measured for light absorption spectrum to give the results shown in Fig. 31. The spectrum has an absorption band in the range of 500-700 nm.

### Example 55

The photoconductor fabricated in Example 35 was mounted on an electrophotographic device. It was found that the device produced clear images.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description, and all the changes which come within the meaning and range of equivalency of the claims are therefore intended to be embraced therein.

The teachings of Japanese Patent Application No. 11-175213, filed June 22, 1999, No. 11-175240, filed June 22, 1999, No. 11-260632, filed September 14, 1999, No. 11-260633, filed September 14, 1999, No. 11-260634, filed September 14, 1999, and No. 2000-070353, filed March 14, 2000, inclusive of their specifications, claims and drawings, are hereby incorporated by reference herein.

## Claims

1. A product obtained by reacting a nitrile compound of the formula (1) with a phthalonitrile compound of the formula (2) or a 1,3-diiminoisoindolin compound of the formula (5) and, optionally, with a metal or a metal-containing compound: 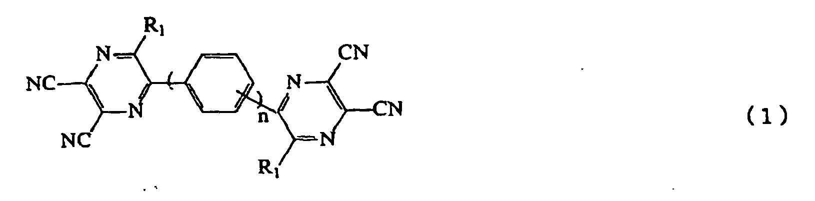 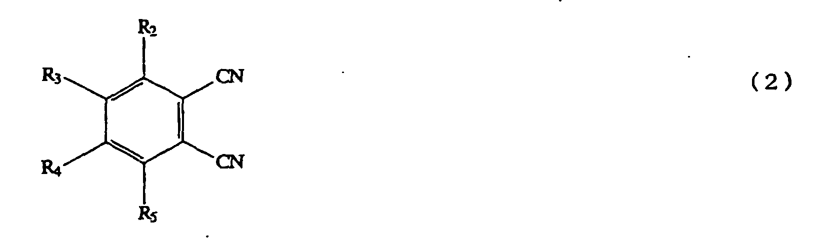 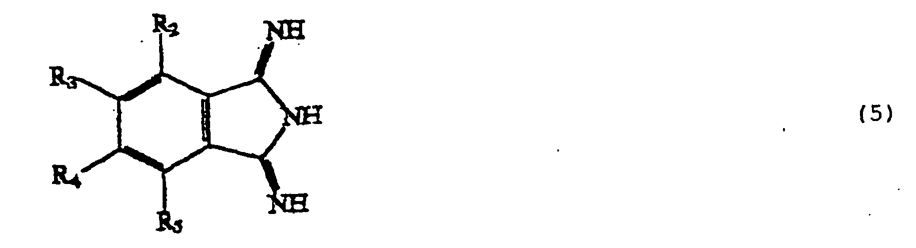
wherein R₁-R₅ stand, independently from each other, for a hydrogen atom, a halogen atom, a substituted or unsubstituted alkyl group, a substituted or unsubstituted aryl group, a heteroaryl group selected from thienyl group, benzothienyl group, furyl group, benzofuranyl group, carbazolyl group, pyridyl group, pyridinyl group, pyrrolidyl group and oxazolyl group, or any of the above heteroaryl groups having one or more substituents, a substituted or unsubstituted cycloalkyl group, a substituted or unsubstituted alkoxyl group, a substituted or unsubstituted aryloxy group, a nitro group or a cyano group and n is an integer of 1 or 2, with the proviso that two adjacent groups R₂-R₅ may together form a ring.

2. A product as claimed in claim 1, having a charge generating property.

3. A product as claimed in claim 1, being a pigment providing a film absorbing light of a wavelength in the range of 500-700 nm in a light absorption spectrum.

4. A product as claimed in claim 1, wherein said metal is titanium.

5. A product as claimed in claim 4, providing a mass spectrum having a peak at 1383±1.

6. A product as claimed in claim 4, permitting detection of Ti by fluorescent X-ray spectroscopy.

7. A product as claimed in claim 1, being amorphous in nature.

8. A product as claimed in claim 7, providing a CuK_{α} X-ray diffraction pattern having no diffraction peaks with a half value width of 1° or less.

9. A product as claimed in claim 1, providing a CuK_{α} X-ray diffraction pattern having strong diffraction peaks at a Bragg angle 2θ±0.2° in the range of 20°-30°.

10. A product as claimed in claim 1, providing a CuK_{α} X-ray diffraction pattern having strong diffraction peaks at a Bragg angle 2θ±0.2° of 27.2°.

11. A product as claimed in claim 10, and providing a CuK_{α} X-ray diffraction pattern having no diffraction peaks at a Bragg angle 2θ±0.2° in the range of 4°-10°.

12. A process for the production of a product according to claim 7, wherein said reaction is followed by a treatment with an organic solvent, an acid, water or a mixture thereof.

13. An electrophotographic photoconductor comprising an electroconductive support and a photoconductive layer provided thereon and containing a product according to anyone of claims 1 to 11.

14. An electrophotographic photoconductor as claimed in claim 13, wherein said photoconductive layer comprises a charge generation layer containing a charge generating substance and a charge transport layer containing a charge transporting substance and wherein said product is contained in said charge generation layer.

15. An electrophotographic photoconductor as claimed in claim 14, wherein said charge transporting substance is a positive hole transport substance and wherein said charge generation layer and said charge transport layer are provided on said support in this order, so that said photoconductor is of a negatively charged type.

16. An electrophotographic photoconductor as claimed in claim 15, wherein said positive hole transport substance is a stilbene compound represented by the following formula (3): 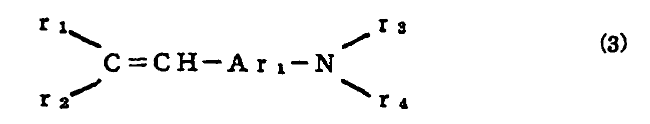 wherein r₁ and r₂ stand, independently from each other, for a substituted or unsubstituted alkyl group or a substituted or unsubstituted aryl group, r₃ and r₄ stand, independently from each other, for a hydrogen atom, a substituted or unsubstituted alkyl group, a substituted or unsubstituted aryl group, or a heterocyclic group, with the proviso that r₁ and r₂ may together form a ring, and Ar₁ stands for a substituted or unsubstituted aryl group or a heterocyclic group.

17. An electrophotographic photoconductor as claimed in claim 14, wherein said charge transporting substance is an electron transport substance and wherein said charge generation layer and said charge transport layer are provided on said support in this order, so that said photoconductor is of a positively charged type.

18. An electrophotographic photoconductor as claimed in claim 17, wherein said electron transport substance is a 2,3-diphenylindene compound represented by the following formula (4): 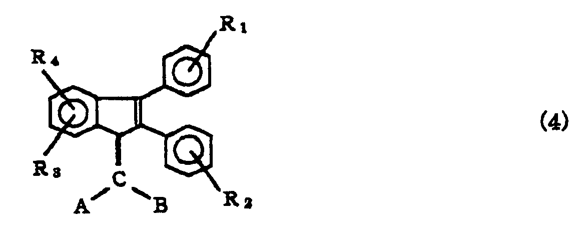
wherein R₁-R₄ stand, independently from each other, for a hydrogen atom, a halogen atom, a substituted or unsubstituted alkyl group, a cyano group or a nitro group, and A and B stand, independently from each other, for a hydrogen atom, a halogen atom, a substituted or unsubstituted aryl group, a cyano group, an alkoxycarbonyl group or an aryloxycarbonyl group.

19. An electrophotographic photoconductor as claimed in claim 13, wherein said photoconductive layer is a single layer.

20. An electrophotographic machine comprising charging means, exposing means, developing means, transfer means, cleaning means, charge removing means and an electrophotographic photoconductor according to claim 13.

21. A process cartridge for an electrophotographic machine comprising charging means and an electrophotographic photoconductor according to claim 13.

## Patentansprüche

1. Produkt, erhalten durch Umsetzen einer Nitrilverbindung der Formel (1) mit einer Phthalonitrilverbindung der Formel (2) oder einer 1,3-Diiminoisoindolinverbindung der Formel (5) und gegebenenfalls mit einem Metall oder einer Metall-haltigen Verbindung: 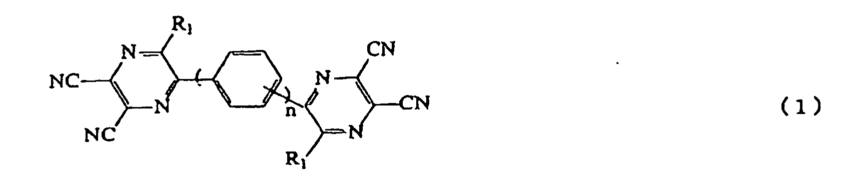 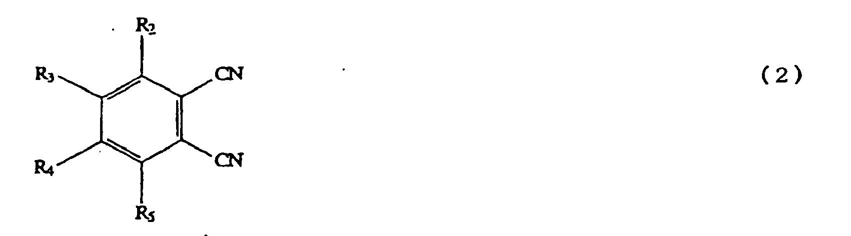 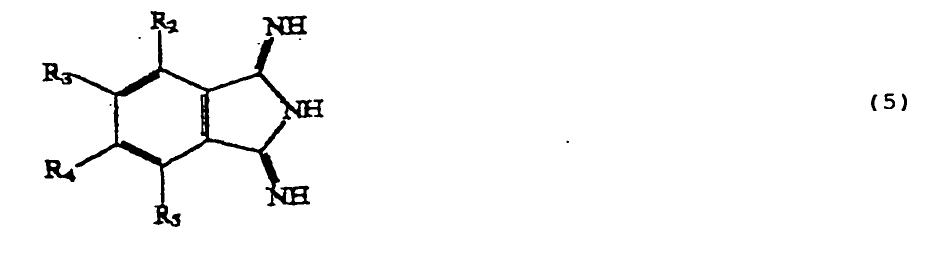
worin R₁ bis R₅ unabhängig voneinander für ein Wasserstoffatom, ein Halogenatom, eine substituierte oder unsubstituierte Alkylgruppe, eine substituierte oder unsubstituierte Arylgruppe, eine Heteroarylgruppe ausgewählt aus Thienylgruppe, Benzothienylgruppe, Furylgruppe, Benzofuranylgruppe, Carbazolylgruppe, Pyridylgruppe, Pyridinylgruppe, Pyrrolidylgruppe und Oxazolylgruppe oder irgendeiner der vorstehenden Heteroarylgruppen mit einem oder mehreren Substituenten, eine substituierte oder unsubstituierte Cycloalkylgruppe, eine substituierte oder unsubstituierte Alkoxylgruppe eine substituierte oder unsubstituierte Aryloxygruppe, eine Nitrogruppe oder eine Cyanogruppe stehen und n eine ganze Zahl von 1 oder 2 ist, mit der Maßgabe, dass zwei benachbarte Gruppen R₂ bis R₅ zusammen einen Ring bilden können.

2. Produkt nach Anspruch 1, mit der Fähigkeit Ladung zu erzeugen.

3. Produkt nach Anspruch 1, das ein Pigment ist, das einen Film bereitstellt, der Licht einer Wellenlänge im Bereich von 500 - 700 nm im Lichtabsorptionsspektrum absorbiert.

4. Produkt nach Anspruch 1, wobei das Metall Titan ist.

5. Produkt nach Anspruch 4, das ein Massenspektrum mit einem Peak bei 1383±1 bereitstellt.

6. Produkt nach Anspruch 4, das es erlaubt, darin Ti mittels Röntgenfluoreszenzspektroskopie nachzuweisen.

7. Produkt nach Anspruch 1, das von amorpher Art ist.

8. Produkt nach Anspruch 7, das ein CuKα - Röntgenbeugungsmuster ergibt, welches keine Beugungspeaks mit einer Halbwertsbreite von 1° oder weniger aufweist.

9. Produkt nach Anspruch 1, das ein CuKα - Röntgenbeugungsmuster ergibt, welches starke Beugungspeaks bei einem Bragg-Winkel 2θ±0,2° in dem Bereich von 20° bis 30° aufweist.

10. Produkt nach Anspruch 1, das ein CuKα - Röntgenbeugungsmuster ergibt, welches starke Beugungspeaks bei einem Bragg-Winkel 2θ±0,2° von 27,2° aufweist.

11. Produkt nach Anspruch 10, das ein CuKα - Röntgenbeugungsmuster ergibt, welches keine Beugungspeaks bei einem Bragg-Winkel 2θ±0,2° in dem Bereich von 4° bis 10° aufweist.

12. Verfahren zur Herstellung eines Produkts nach Anspruch 7, wobei auf die Reaktion eine Behandlung mit einem organischen Lösungsmittel, einer Säure, Wasser oder einer Mischung davon folgt.

13. Elektrophotographischer Photoleiter, umfassend einen elektrisch leitfähigen Träger und eine darauf bereitgestellte photoleitende Schicht und enthaltend ein Produkt nach irgendeinem der Ansprüche 1 bis 11.

14. Elektrophotographischer Photoleiter nach Anspruch 13, wobei die photoleitende Schicht eine Ladungserzeugungsschicht, enthaltend eine Ladungserzeugungssubstanz, und eine Ladungstransportschicht, enthaltend eine Ladungstransportsubstanz, umfasst und das Produkt in der Ladungserzeugungsschicht enthalten ist.

15. Elektrophotographischer Photoleiter nach Anspruch 14, wobei die Ladungstransportsubstanz eine Transportsubstanz für positive Löcher ist und die Ladungserzeugungsschicht und die Ladungstransportschicht in dieser Reihenfolge auf dem Träger bereitgestellt sind, so dass der Photoleiter einer vom negativ geladenen Typ ist.

16. Elektrophotographischer Photoleiter nach Anspruch 15, wobei die Transportsubstanz für positive Löcher eine Stilbenverbindung ist, die durch die folgende Formel (3) dargestellt wird: 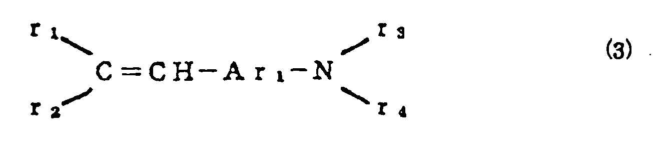
wobei r₁ und r₂ unabhängig voneinander für eine substituierte oder unsubstituierte Alkylgruppe oder eine substituierte oder unsubstituierte Arylgruppe stehen, r₃ und r₄ unabhängig voneinander für ein Wasserstoffatom, eine substituierte oder unsubstituierte Alkylgruppe, eine substituierte oder unsubstituierte Arylgruppe oder eine heterocyclische Gruppe stehen, mit der Maßgabe, dass r₁ und r₂ zusammen einen Ring bilden können und Ar₁ für eine substituierte oder unsubstituierte Arylgruppe oder eine heterocyclische Gruppe steht.

17. Elektrophotographischer Photoleiter nach Anspruch 14, wobei die Ladungstransportsubstanz eine Transportsubstanz für Elektronen ist und die Ladungserzeugungsschicht und die Ladungstransportschicht in dieser Reihenfolge auf dem Träger bereitgestellt sind, so dass der Photoleiter einer vom positiv geladenen Typ ist.

18. Elektrophotographischer Photoleiter nach Anspruch 17, wobei die Transportsubstanz für Elektronen eine 2,3-Diphenylindenverbindung ist, die durch die folgende Formel (4) dargestellt wird: 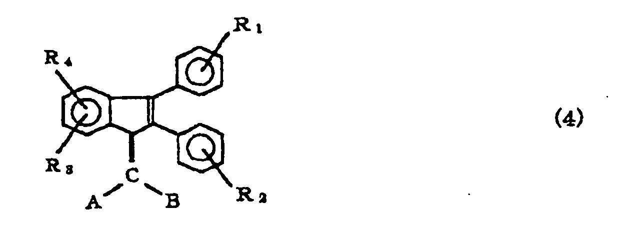
worin R₁ bis R₄ unabhängig voneinander für ein Wasserstoffatom, ein Halogenatom, eine substituierte oder unsubstituierte Alkylgruppe, eine Cyanogruppe oder eine Nitrogruppe stehen, und A und B unabhängig voneinander für ein Wasserstoffatom, ein Halogenatom, eine substituierte oder unsubstituierte Arylgruppe, eine Cyanogruppe, eine Alkoxycarbonylgruppe oder eine Aryloxycarbonylgruppe stehen.

19. Elektrophotographischer Photoleiter nach Anspruch 13, wobei die photoleitende Schicht eine Einzelschicht ist.

20. Elektrophotographisches Gerät, umfassend eine Aufladungseinrichtung, eine Belichtungseinrichtung, eine Entwicklungseinrichtung, eine Übertragungseinrichtung, eine Reinigungseinrichtung, eine Ladungsentfernungseinrichtung und einen elektrophotographischen Photoleiter nach Anspruch 13.

21. Prozesskartusche für ein elektrophotographisches Gerät, umfassend eine Aufladungseinrichtung und einen elektrophotographischen Photoleiter nach Anspruch 13.

## Revendications

1. Produit obtenu par réaction d'un composé nitrile de la formule (1) avec un composé phtalonitrile de la formule (2) ou un composé 1,3-diiminoisoindoline de la formule (5) et facultativement avec un métal ou un composé contenant un métal : 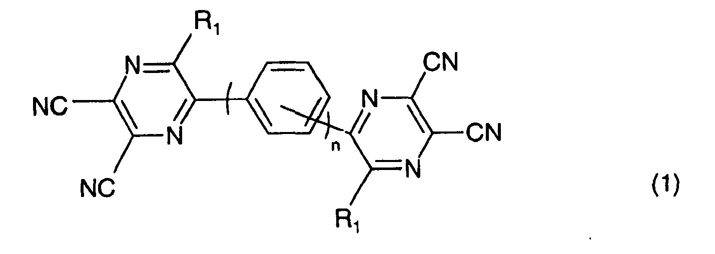 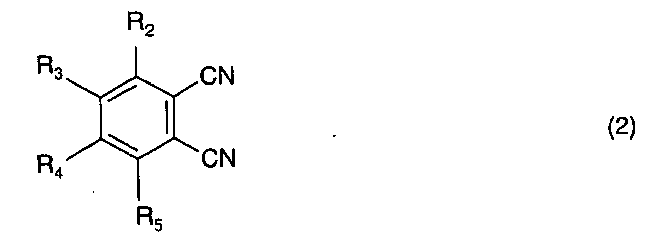 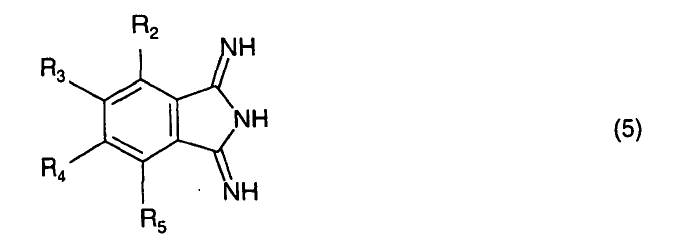
dans lesquelles R₁-R₅ représentent, indépendamment l'un de l'autre, un atome d'hydrogène, un atome d'halogène, un groupe alkyle substitué ou non substitué, un groupe aryle substitué ou non substitué, un groupe hétéroaryle choisi parmi un groupe thiényle, un groupe benzothiényle, un groupe furyle, un groupe benzofuranyle, un groupe carbazolyle, un groupe pyridyle, un groupe pyridinyle, un groupe pyrrolidyle et un groupe oxazolyle, ou l'un quelconque des groupes hétéroaryle ci-dessus ayant un ou plusieurs substituants, un groupe cycloalkyle substitué ou non substitué, un groupe alcoxyle substitué ou non substitué, un groupe aryloxy substitué ou non substitué, un groupe nitro ou un groupe cyano et n est un nombre entier de 1 ou 2, à condition que deux groupes adjacents R₂-R₅ peuvent former ensemble un cycle.

2. Produit selon la revendication 1 ayant une propriété de production de charge.

3. Produit selon la revendication 1 étant un pigment fournissant un film absorbant de la lumière d'une longueur d'onde dans l'intervalle de 500-700 nm dans un spectre d'absorption de lumière.

4. Produit selon la revendication 1, dans lequel ledit métal est le titane.

5. Produit selon la revendication 4 fournissant un spectre de masse ayant un pic à 1383 ± 1.

6. Produit selon la revendication 4 permettant la détection de Ti par spectroscopie fluorescente aux rayons X fluorescents.

7. Produit selon la revendication 1 étant de nature amorphe.

8. Produit selon la revendication 7 fournissant un motif de diffraction aux rayons X CuK_{α} n'ayant pas de pic de diffraction avec une largeur de bande effective de 1° ou inférieure.

9. Produit selon la revendication 1 fournissant un motif de diffraction aux rayons X CuK_{α} ayant de forts pics de diffraction sous un angle Bragg de 2θ ± 0,2° dans l'intervalle de 20°-30°.

10. Produit selon la revendication 1 fournissant un motif de diffraction aux rayons X CuK_{α} ayant de forts pics de diffraction sous un angle Bragg 2θ ± 0,2° de 27,2°.

11. Produit selon la revendication 10 fournissant un motif de diffraction aux rayons X CuK_{α} n'ayant pas de pic de diffraction sous un angle Bragg 2θ ± 0,2° dans l'intervalle de 4°-10°.

12. Procédé pour la production d'un produit selon la revendication 7, dans lequel ladite réaction est suivie par un traitement avec un solvant organique, un acide, de l'eau ou un mélange de ceux-ci.

13. Photoconducteur électrophotographique comprenant un support électroconducteur et une couche photoconductrice fournie sur son dessus et contenant un produit selon l'une quelconque des revendications 1 à 11.

14. Photoconducteur électrophotographique selon la revendication 13, dans lequel ladite couche photoconductrice comprend une couche de production de charge contenant une substance de production de charge et une couche de transport de charge contenant une substance de transport de charge et dans lequel ledit produit est contenu dans ladite couche de production de charge.

15. Photoconducteur électrophotographique selon la revendication 14, dans lequel ladite substance de transport de charge est une substance de transport de trou positif et dans lequel ladite couche de production de charge et ladite couche de transport de charge sont fournies sur ledit support dans cet ordre de telle sorte que ledit photoconducteur est d'un type négativement chargé.

16. Photoconducteur électrophotographique selon la revendication 15, dans lequel ladite substance de transport de trou positif est un composé de stilbène représenté par la formule (3) suivante : 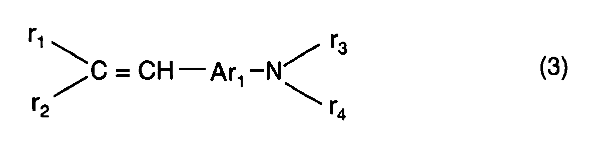
dans laquelle r₁ et r₂ représentent, indépendamment l'un de l'autre un groupe alkyle substitué ou non substitué ou un groupe aryle substitué ou non substitué, r₃ et r₄ représentent, indépendamment l'un de l'autre un atome d'hydrogène, un groupe alkyle substitué ou non substitué, un groupe aryle substitué ou non substitué ou un groupe hétérocyclique à condition que r₁ et r₂ peuvent former ensemble un cycle, et Ar₁ représente un groupe aryle substitué ou non substitué ou un groupe hétérocyclique.

17. Photoconducteur électrophotographique selon la revendication 14, dans lequel ladite substance de transport de charge est une substance de transport d'électron et dans lequel ladite couche de production de charge et ladite couche de transport de charge sont fournis sur ledit support dans cet ordre, de telle sorte que ledit photoconducteur est d'un type positivement chargé.

18. Photoconducteur électrophotographique selon la revendication 17, dans lequel ladite substance de transport d'électrons est un composé de 2,3-diphénylindène représenté par la formule (4) suivante : 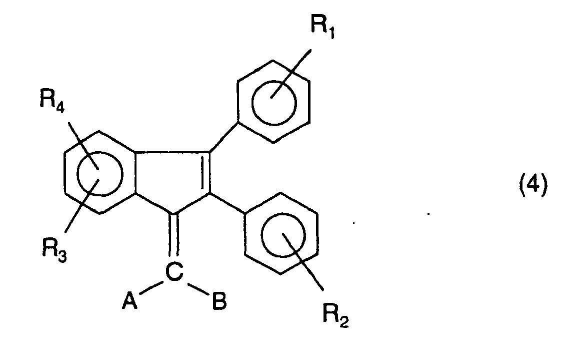
dans laquelle R₁-R₄ représentent, indépendamment l'un de l'autre, un atome d'hydrogène, un atome d'halogène, un groupe alkyle substitué ou non substitué, un groupe cyano ou un groupe nitro, et A et B représentent, indépendamment l'un de l'autre, un atome d'hydrogène, un atome d'halogène, un groupe aryle substitué ou non substitué, un groupe cyano, un groupe alcoxycarbonyle ou un groupe aryloxycarbonyle.

19. Photoconducteur électrophotographique selon la revendication 13, dans lequel ladite couche photoconductrice est une couche unique.

20. Machine électrophotographique comprenant un moyen de charge, un moyen d'exposition, un moyen de développement, un moyen de transfert, un moyen de nettoyage, un moyen d'élimination de charge et un photoconducteur électrophotographique selon la revendication 13.

21. Cartouche de traitement pour une machine électrophotographique comprenant un moyen de charge et un photoconducteur électrophotographique selon la revendication 13.
